(19) Europäisches Patentamt
European Patent Office
Office européen des brevets

(11) **EP 1 789 907 B1**

(12) **EUROPEAN PATENT SPECIFICATION**

(45) Date of publication and mention of the grant of the patent:
**14.04.2010 Bulletin 2010/15**

(21) Application number: **05792505.9**

(22) Date of filing: **30.08.2005**

(51) Int Cl.:
***G06K 9/00*** (2006.01)

(86) International application number:
**PCT/US2005/030662**

(87) International publication number:
**WO 2006/026548 (09.03.2006 Gazette 2006/10)**

(54) **BIOPOTENTIAL WAVEFORM DATA FUSION ANALYSIS AND CLASSIFICATION METHOD**

BIOPOTENTIAL-SIGNALFORM-DATENFUSIONSANALYSE- UND KLASSIFIKATIONSVERFAHREN

PROCEDE DE CLASSIFICATION ET D'ANALYSE DE FUSION DE DONNEES DE FORMES D'ONDES DE BIOPOTENTIELS

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.08.2004 US 605630 P**

(43) Date of publication of application:
**30.05.2007 Bulletin 2007/22**

(73) Proprietors:
• **Southern Illinois University Carbondale**
  **Carbondale IL 62901-6603 (US)**
• **Neuronetrix Solutions, LLC**
  **Louisville KY 40204 (US)**

(72) Inventors:
• **GUPTA, Lalitmore**
  **Carbondale, IL 62903-7691 (US)**
• **FADEM, Kalford, C.**
  **Louisville**
  **KY 40206 (US)**

(74) Representative: **Samson & Partner**
**Widenmayerstrasse 5**
**80538 München (DE)**

(56) References cited:
**EP-A- 0 764 001        US-A- 4 860 216**
**US-A- 5 661 666**

• **L. GUPTA: "Parametric Classification of Multichannel Averaged Event-Related Potential" IEEE TRANSACTION ON BIOMEDICAL ENGINEERING, vol. 49, no. 8, 8 August 2002 (2002-08-08), pages 905-911, XP009059089**
• **GUPTA L ET AL: "Robust classification of multichannel match and mismatch evoked potentials" ENGINEERING IN MEDICINE AND BIOLOGY SOCIETY, 2000. PROCEEDINGS OF THE 22ND ANNUAL INTERNATIONAL CONFERENCE OF THE IEEE 23-28 JULY 2000, PISCATAWAY, NJ, USA,IEEE, vol. 2, 23 July 2000 (2000-07-23), pages 1302-1308, XP010529168 ISBN: 0-7803-6465-1**

**Description**

**Cross Reference to Related Application**

The present application hereby claims the benefit of the U.S. provisional patent application entitled "EVOKED RESPONSE POTENTIAL DATA FUSION ANALYSIS METHOD", Serial No. 60/605,630, filed on 30 August 2004.
The present application is also related to co-pending PCT Int'l Pat. Appln. No. WO2004US19418, "DEVICE AND METHOD FOR AN AUTOMATED E.E.G. SYSTEM FOR AUDITORY EVOKED RESPONSES", to Fadem et al., filed 18 June 2004, published as Pat. No. WO200411260 (A2), which claimed the benefit of the U.S. provisional patent application of the same title, Serial No. 60/479,684, filed on 19 June 2003.
The present application is also related to co-pending PCT Int'l Pat. Appln. No. W02005US010515 and U.S. Pat. Appln. Ser. No. 11/092,395, both entitled "DEVICE AND METHOD FOR AN AUTOMATED E.E.G. SYSTEM FOR AUDITORY EVOKED RESPONSE", filed on 29 March 2005 and claiming priority to U.S. Pat. Appln. Ser. No. 60/557,230, "ACTIVE, MULTIPLEXED DIGITAL NEURO ELECTRODES FOR EEG, ECG, EMG APPLICATIONS", filed on 29 March 2004.
The present application is also related to co-pending PCT Int'l Pat. Appln. No. WO2005US21272, "Evoked Response Testing System for Neurological Disorders", filed 16 June 2005, which claimed the benefit of U.S. Pat. Appln. Ser. No. 60/580,853, "AUDITORY EVOKED RESPONSE MAPPING SYSTEM FOR AUDITORY NEUROME", filed 18 June 2004.

**Field of the Invention**

The present invention relates generally to a method for classifying biopotential waveforms. More particularly, the present invention provides a method of classifying biopotential waveform measurements such as ERPs, EEGs, ECGs, and EMGs, for the purpose of diagnosing abnormal physiologic and/or cognitive conditions such as learning or developmental disabilities, heart conduction disorders, and/or neuromuscular disorders, by measuring voltage potentials from the surface of the skin produced by the electrochemical activity within muscles and nerves and correlating those measurements with the respective disorders.

**Background of the Invention**

Biopotential signals are measured on the surface of the skin as a varying electric field produced by the underlying electrochemical activity within living muscles and nerves. Many physiological or cognitive disorders can be identified by classifying the biopotential waveforms and correlating them with the various disorders.
The brain produces electrical activity that can be measured non-invasively by placing electrodes on the scalp. The electroencephalogram (EEG) is a recording of the ever-present, on-going electrical activity generated by the neurons in the brain. EEGs are used to diagnose medical conditions such as epilepsy, brain tumors, sleep disorders, and brain injury.
Evoked response potentials (ERPs), buried in the on-going EEG, are brain responses that are time-locked to the onset of an external event. The external event may be a sensory stimulus such as a visual flash or an auditory sound, a mental event such as the recognition of a specified target stimulus, or the omission of a stimulus such as an increased time gap between stimuli. ERPs recorded in response (i) to visual stimuli are called visual evoked potentials (VEPs), (ii) to auditory stimuli are called auditory evoked potentials (AEPs), and (iii) to mild electrical impulses applied to the arms or legs are called somatosensory (SEPs). Like EEGs, ERPs are recorded by placing electrodes on different parts of the scalp. The high temporal millisecond resolution of ERPs is ideal for studying timing aspects of both normal and abnormal cognitive processes such as those involved in learning, attention, decision-making, reading, language processing, and memory. Clinical investigations employ ERPs to assist in diagnosing brain dysfunction in patients with dyslexia, schizophrenia, Alzheimer's disease, Parkinson's disease, aphasia, and alcoholism.
Other biopotential signals can be used to diagnose a variety of other disorders. Electrocardiograms (ECGs) use several electrodes attached to the chest to record electrical potentials produced from the beating heart. Variations in the waveform shape, amplitude, latency, and power spectrum can be correlated with many abnormal cardiac conditions such as; myocardial infarction, pericarditis, ventricular hypertrophy, and hiss bundle blocks. Electromyograms (EMGs) use electrodes attached to various parts of the body, particularly the extremities, to detect abnormal electrical activity related to many neuromuscular diseases including muscular dystrophy, pinched nerves, peripheral nerve damage, amyotrophic lateral sclerosis (ALS), myasthenia gravis, carpal tunnel syndrome, and disc herniation.
A major difference between ERPs and the other biopotential signals such as EEG, ECG, or EMG is that ERPs are time-locked to a specific event such as the presentation of a sound stimulus or flash stimulus. EEGs, ECGs, and EMGs are ongoing signals which are generally not measured from a specific point in time. However, these ongoing signals can be subdivided into time intervals of specific durations such that each time interval can be treated as an explicit event and then analyzed in a similar manner to ERP classification. In ECGs for example, the ongoing waveform could be subdivided at the beginning of each "P-wave". In this way, individual heartbeats can be treated much like individual ERPs and evaluated with respect to a range of normal or abnormal variations. For example, tall (or peaked) T waves may be seen in hyperkalemia or very early myocardial infarction. Inverted T waves may be seen in both ischemia and infarction, late in pericarditis, ventricular hypertrophy, bundle branch block, and cerebral disease.

In many electrophysiology applications a multitude of electrodes are used to measure the electrical signals from various locations on the body. In some EEG systems such as the GEODESIC EEG SYSTEM 250 FROM ELECTRICAL GEODESICS INC., data is recorded from as many as 256 electrodes distributed around the head. Standard 12-lead ECG recording uses electrodes attached to 10 sites on the chest arms and legs. The NC-STAT SYSTEM from NEUROMETRIX, INC. uses preconfigured electrode sensor strips with between 3 and 6 electrodes for nerve conduction testing. In ERP tests, it is common to use more than one stimulus while recording the ERPs from a multitude of electrodes.

In evaluating the results of biopotential tests where data is recorded from multiple sources or "channels" such as multiple electrodes or tests performed with multiple stimuli, certain channels may be more useful in discriminating the separate classes at certain times and at other times, other channels may be more useful. This creates significant problems for the user to select which points from which channels should be used to discriminate the classes. It would also be helpful if linear combinations of the multi-channel data could be used in the discrimination process.

PCT Pat. Appln. Ser. No. WO 2004US194018 entitled "DEVICE AND METHOD FOR AN AUTOMATED E.E.G. SYSTEM FOR AUDITORY EVOKED RESPONSES", to Kalford C. Fadem, filed 18 June 2004, and PCT Int'l Pat. Appln. No. WO2005US21272, "Evoked Response Testing System for Neurological Disorders", filed 16 June 2005, describe an advantageous clinical auditory screening system that performs an auditory evoked response (AEP) test by positioning electrodes about the ears of the subject. An integral control module automatically performs the test, providing simplified controls and indications to the clinician. A number of screening tests that are stored in the headset are periodically uploaded for, remote analysis and result reporting. The resulting system addressed the needs for performing a range of ERP tests more appropriate for routine screening for abnormalities at an earlier stage where intervention has increased efficacy.

While sensitive electrodes and processing criteria disclosed therein advantageously detected and recorded these weak brain wave signals, it would be desirable to enhance the classification accuracy by incorporating methods that would use data from all electrodes, epochs, and stimuli (collectively referred to as channels) in the classification process.

The major limitations of existing methodologies for ERP classification (or classification of other biopotential waveforms related to specific events) can be summarized as follows:

First, most ERP or other biopotential waveform measurements require recording data from multiple electrodes, epochs, stimuli, and/or tests (collectively referred to as channels). Each channel of data contains points which represent a measured value, usually in $\mu V$, versus time. The ability to discriminate between classes varies from channel to channel as a function of time. Most classifiers focus on classifying the ERPs of each channel independently and do not fully exploit the different but complementary information buried in the multi-channel recordings of biopotential activity.

Second, because of the poor signal-to-noise-ratio (SNR), analysis and classification is typically conducted on ERPs averaged over a large number of trials. In practice, collecting a large number of single-trial ERPs to form averaged ERPs is quite prohibitive because individuals experience fatigue and lose concentration over long data collection sessions. Currently, having to repeat experiments many times is the most limiting feature of EPs in brain waveform studies and clinical applications.

Third, most classifiers for brain waveforms are multivariate classifiers and require the estimation of second order parameters such as the covariance matrix. For the covariance matrix to be non-singular, the number of ERPs in the design set has to exceed the dimension of the ERP. Even if the number of single-trial ERPs exceeds the dimension, the number of averaged ERPs available for design and testing decreases when larger ERP averages are formed. As a result, it is not possible to estimate the multivariate parameters.

Fourth, classifier formulations are typically limited to dichotomous classification (2-categories) and are not generalized for polychotomous classification.

In addition, U.S. Pat. Nos. 5,467,777; 5,406,956; 5,363,858 and 4,941,477 describe various methods for electroencephalographic information detection, also referred to as a brain fingerprinting method. The system basically works by flashing words or pictures relevant to an incident in question (e.g., a crime) on a computer screen, along with irrelevant words and pictures. Electrical brain responses are measured through a headband equipped with sensors. Memory and encoding related multifaceted electroencephalographic responses (MERMER) were elicited when the brain processed noteworthy information that it recognized.

US 5,661,666 discloses a system realizing a data fusion from a plurality of sensors. Each sensor samples input data and attempts to classify it independently. Each single decision is then weighted by a factor indicating the relative reliability of the corresponding sensor.

While this approach has certain desirable features, it would be desirable to enhance so as to make such testing more efficient and applicable to a larger population of subjects and uses.

## Brief Summary of the Invention

This invention overcomes the major limitations listed above by developing a new mathematical system which dynamically exploits the most useful discriminatory information from multi-channel biopotential waveform recordings to accurately classify differential biopotential activity, even for small averages, into the respective physiologic/cognitive categories.

In one aspect of the invention, classification accuracy of the biopotential activity is improved by introducing a new parametric multi-channel decision fusion strategy which dynamically exploits multi-channel biopotential waveform information at different time instants. A classifier is designed for each time-instant for each channel and the classifiers are ranked and selected according to their classification accuracies. The decisions of the selected classifiers are combined into a decision fusion vector and the decision fusion vector is classified using a discrete Bayes classifier.

Specifically, the invention overcomes the limitations listed above in the following ways:

First, information is exploited from multiple channels by fusing the classification information from different channels at different time instants.
Second, biopotential waveforms do not have to be averaged over a large number of single-trials to be classified accurately.
Third, due to the univariate formulation, multivariate parameter estimation is not required.
Fourth, the formulation is polychotomous and is therefore not restricted to dichotomous brain activity classification.

The improvement in performance using the new multi-channel decision fusion classification strategy has already been demonstrated by comparing the performance with the single best channel on real 2-category match/mismatch ERP cognitive study and a 3-category control/dyslexic/poor reader clinical study.

Particularly, the present invention provides an apparatus according to claim 1. Preferred embodiments are defined in the dependent claims.

In another aspect of the invention, the method could be used for truth detection by providing a sequence of auditory word prompts, some unrelated to an incident in question and other words closely related. The AEPs are then compared in order to assess whether the subject has some close association with the incident.

These and other objects and advantages of the present invention shall be made apparent from the accompanying drawings and the description thereof.

## Brief Description of the Figures

The accompanying drawings, which are incorporated in and constitute a part of this specification, illustrate embodiments of the invention, and, together with the general description of the invention given above, and the detailed description of the embodiments given below, serve to explain the principles of the present invention.

FIG. 1 shows a system level diagram for an ERP testing and analysis system.

FIG. 2 shows an integrated headset for use in ERP testing.

FIG. 3 shows a diagram of a standard electrode positioning coordinate system for EEG or ERP recording.

FIG. 4 shows an example of ERP waveforms recorded from multiple electrodes, epochs, and stimuli.

FIG. 5 shows the workflow logic for an ERP testing and analysis system.

FIG. 6 is an example of the differences in the 6-channel ensemble-averaged ERPs collected from an individual engaged in a 2-category match versus mismatch decision-making task.

FIG. 7 shows a diagram which summarizes the steps involved in classifying an unknown ERP signal using the fusion classifier method.

FIG. 8 shows a diagram which summarizes the training phase and dynamic ranking of the channel-time samples.

FIG. 9 shows a standard electrode positioning coordinate system for ECG recording.

FIG. 10 shows an example of a normal ECG waveform recording.

FIG. 11 shows a diagram of the components of a single heartbeat ECG waveform.

FIG. 12 is a diagram of classification accuracies of the fusion rule classifier for the real ERPs of Subject A and for the simulated ERPs.

FIG. 13 is a diagram of classification accuracy of the fusion rule classifiers for the four subjects in the second match/mismatch experiment.

FIG. 14 is a diagram of classification of each MSMC ERP type.

FIG. 15 is a diagram of the MSMC vector data fusion strategy.

FIG. 16 is a diagram of the MSMC vector decision fusion strategy.

FIG. 17 is a diagram of the MSMC element data fusion strategy.

FIG. 18 is a diagram of the MSMC element decision fusion strategy.

FIG. 19 is a diagram of Multi-channel decision fusion.

FIG. 20 is a diagram of the Multi-channel EP-sum fusion.

FIG. 21 is a diagram of the Multi-channel EP-concatenation fusion.

FIG. 22 is a diagram of Match and mismatch classification rates averaged across the subjects.

FIG. 23 is a diagram of Classification accuracies, averaged across the subjects, as a function of the number of channels.

## Detailed Description of the Invention

The general system description is provided in the cross-referenced application.

A general approach to data fusion is described in (1) L. Gupta, J. Phegley, & D.L. Molfese, "Parameter estimation and multichannel fusion for classifying averaged ERPs," The Second Joint Meeting of the IEEE Engineering in Medicine and Biology Society and the Biomedical Engineering Society, October 23-26, Houston, Texas, 2002; (2) L. Gupta, B. Chung, J. Phegley, & D.L. Molfese, "A multi-channel EP fusion classification strategy for brain-computer interface development," The 7 World Multiconference on Systemics, Cybernetics and Informatics, July 27-30, Orlando, Florida, 2003; and (3) L. Gupta, B. Chung, J. Phegley, & D.L. Molfese, "Multi-channel fusion models for the parametric classification of multi-category differential brain activity," 26th Annual International Conference of the IEEE Engineering in Medicine and Biology Society, September 1-5, San Francisco, California, 2004, each of which is hereby incorporated by reference in their entirety.

In an illustrative example of applying this data fusion method to auditory evoked response (AER) testing to accurately classify Control/Dyslexia data is described in H. Kook, L. Gupta, D. Molfese, & K. Fadem, "Multi-stimuli multi-channel data and decision fusion strategies for dyslexia prediction using neonatal ERPs", Pattern Recognition, (in press). The results show that by selecting certain combinations of time instants across the 6 electrodes and across the 4 stimuli, we can consistently get 100% classification accuracies. The selection is done automatically using the inter-class separation measures. The classifier is a nearest mean classifier. The results were obtained by randomly selecting 12 out of the 24 control and 12 out of the 24 dyslexics randomly and classifying the remaining 12 and 12. This was repeated 50 times to estimate the classification accuracy. The total number of tests, therefore, were, (12+12)(50)=1200. No classification errors occurred.

Using 0.1 and 0.9, the prior probabilities for dyslexic and not dyslexic, respectively, the sensitivity and specificity values are exact. Using Bayes rule, 0.3285 value for PV+ and 0.988 value for PV- were obtained, validating our assumption that improvement to PV+ may be achieved by decreasing the false positives in the denominator term in the PV+ computation.

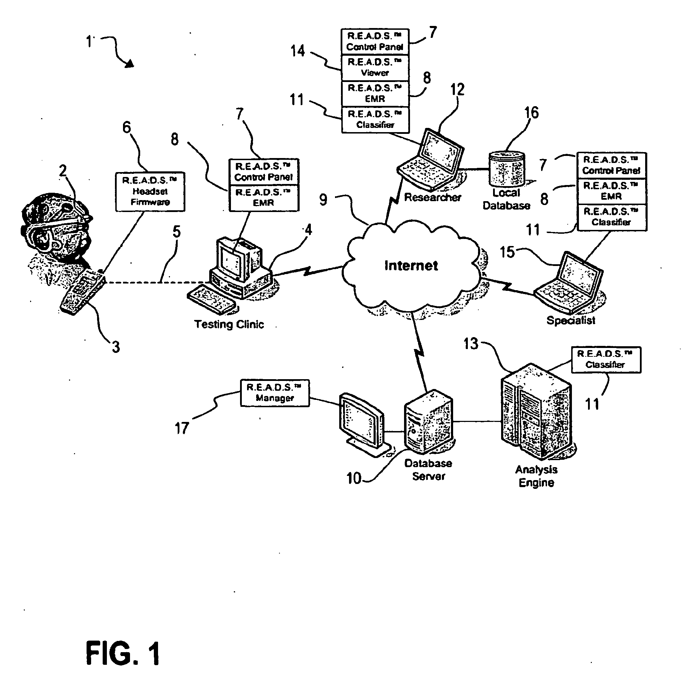

In the drawings where like members are given the same reference numeral, FIG. 1 depicts an exemplary Evoked Response Potential (ERP) (e.g., dyslexia) screening system 1 that advantageously provides for economical testing, data storage, analysis, and other features. To this end, the headset 2 and control box 3 may be in electrical communication with a hospital system 4 via a cable or wireless link 5 so that accomplishment of the dyslexia screening test, performed under the control of the headset firmware 6. Administration of the test is controlled through the control panel software application 7. Additional information is noted for patient health records and for billing records through the electronic medical records (EMR) software application 8. Also, the hospital system 4 may facilitate communication across a network, such as the Internet 9, to a remote processing facility, depicted as a data repository computer 10. Analysis using the fusion classifier software application 11 may be performed remotely on the researcher computer 12 or an analysis computer 13. Users of the ERP screening system 1 may access the system 2 through research computer 12 for the purpose of creating testing protocols with the control panel software application 7 or visualizing testing results using viewer software application 14. Users of the ERP screening system 1 may access the system 2 for the purpose of evaluating patient tests through physician (specialist) computer 15. Users may also store data on a database 16 connected to their own computers 12 and 15. Administrators of the system 1 may have direct access to the system database on data repository computer 10 through management console software application 17.

Positive, inconclusive, and/or negative screening test results may be forwarded to an appropriate recipient, such as a referral physician 15 for further diagnostic testing and/or therapeutic measures.

An exemplary ERP testing system 1 is described in greater detail in the afore-mentioned PCT Int'l Pat. Appln. No. WO2005US21272, "Evoked Response Testing System for Neurological Disorders", filed 16 June 2005.

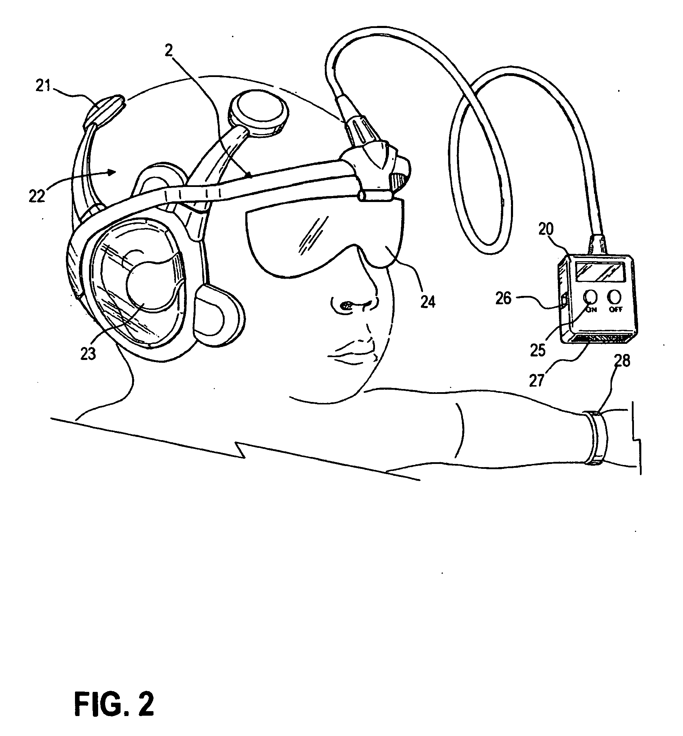

FIG. 2 depicts an exemplary integrated ERP headset 2 includes embedded features that enable clinicians to readily perform an ERP test without the necessity of extensive training. Portability of diagnostic data taking allows use whenever and wherever desired. Economy of use is achieved by centralized processing of the diagnostic data so that a great number of headsets 2 may be used without the necessity of expensive waveform processing equipment at each location. Collecting data from many screened individuals enables enhanced and improved diagnostic algorithms to be created and implemented. Furthermore, the headset 2 includes features that speed its use while avoiding human error and the need for extensive training.

To these ends, the ERP headset 2 incorporates a control module 20 that advantageously allows the headset 2 to be portable and to be used in a clinical setting by including pre-loaded or downloadable testing protocols managed by the control module 20, enhancing ease of use. The headset 10 automatically positions a plurality of conductive electrode plugs ("electrodes") 21, which pick up the voltage signal of the ERP, to specific positions relative to the ears of the testing subject 22 correlating to portions of the brain responsible for auditory or visual processing.

An exemplary electrode 21 may employ an active digital electrode approach for incorporation into the headset 2 to address the need for sensitivity, enhanced signal to noise performance, and economy, described in greater detail in the afore-mentioned PCT Int'l Pat. Appln. No. WO2005US010515 and U.S. Pat. Appln. Ser. No. 11/092,395, both entitled "DEVICE AND METHOD FOR AN AUTOMATED E.E.G. SYSTEM FOR AUDITORY EVOKED RESPONSE", filed on 29 March 2005.

The headset 2 also sound projectors 23 in front of the respective subject's ear that generates an auditory signal in response to an electrical signal from the control module 12.

A visual display device 24 may also be advantageously incorporated into the headset 2 for the purpose of presenting a visual stimulus to the subject. The visual display device 24 may incorporate discrete illumination devices or video display devices.

Although the headset 2 may include all of the functionality required to perform a (e.g., dyslexia) ERP testing protocol, the headset 2 advantageously accepts an external electrical connector at an interface 26 so that additional functionality may be selectively used. The interface 26 may accept subject identification information to be linked with the diagnostic data taken. An illustrative input device is depicted such as an identity scanning device 27, which is integrated into a control box 20 to read a patient identification band 28.

The keypad 25 may also be used as an input device used by the testing subject 22 when specific testing protocols require an active response from the testing subject 22. Certain paradigms require the subject to actively respond to a particular stimuli, audio or visual. ie; "press the button each time you see an animal". Stimuli: cat - dog - rabbit - cow - flower. This is called a "stop-signal" paradigm and evaluates the inhibition response (among others).

An exemplary ERP headset 2 is described in greater detail in the afore-mentioned PCT patent application No. WO2004US19418, "DEVICE AND METHOD FOR AN AUTOMATED E.E.G. SYSTEM FOR AUDITORY EVOKED RESPONSES", to Fadem et al., filed 18 June 2004

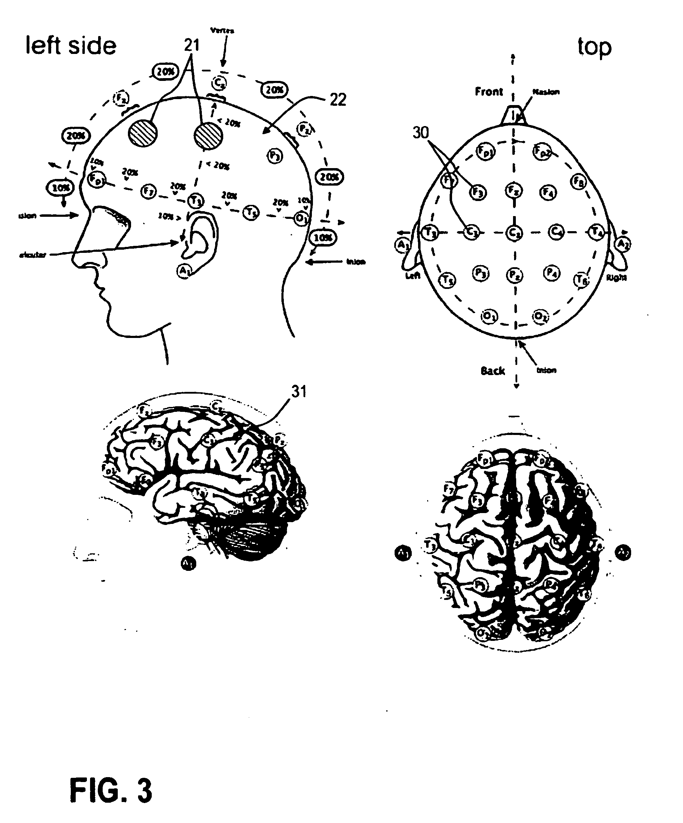

FIG. 3 depicts an exemplary coordinate system used to locate EEG/ERP electrodes 21. A plurality of EEG electrodes 21 could be advantageously positioned on the scalp of a subject 22 at predetermined locations 30 which conform to the "10-20 EEG Coordinate System" know to those skilled in the art. These locations 30 have been advantageously selected to provide multiple channels of biopotential waveform data with wide spatial distributions proximate to the subject's 22 brain 31.

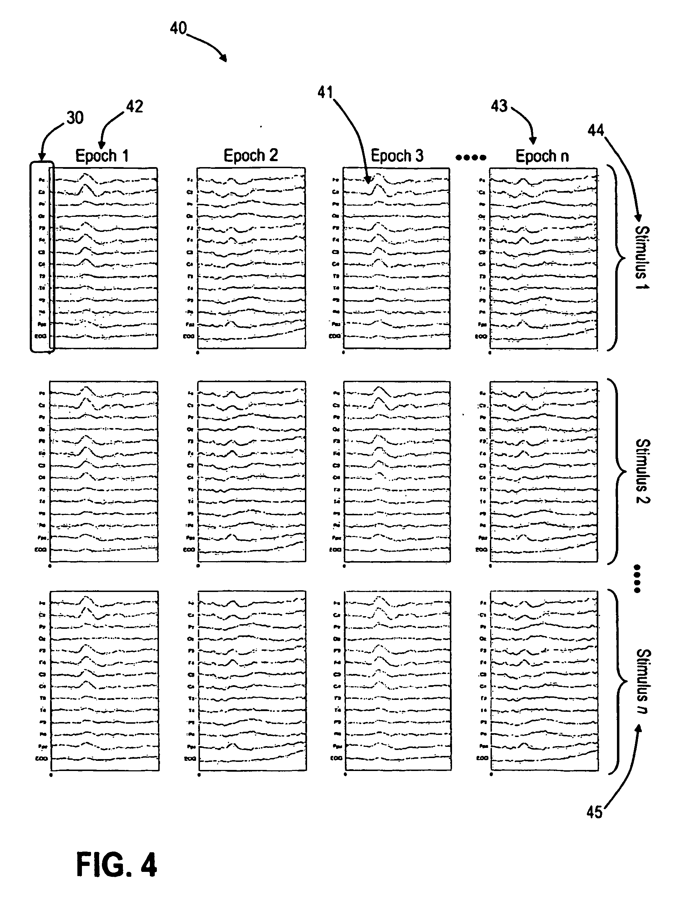

FIG. 4 depicts the results from an ERP test 40 wherein a plurality of biopotential waveforms 41 recorded from electrodes 21 placed at each of fourteen locations 30 on a subject's scalp 22. The fourteen waveforms 41 are recorded in response to a single stimulus and are grouped into epochs 42. The diagram depicts an unlimited number or "$n$" epochs 43. Each group of $n$ epochs 43 are recorded for each of a plurality of stimuli 44. There may be an unlimited of number or "$n$" stimuli 45. A channel of data may be described by an individual waveform 41, a group of waveforms for a single electrode and a single stimulus, or a group of waveforms for each stimulus. A channel of data may also be described by an algebraic combination of waveforms or groups of waveforms such as a group average from one stimulus minus a group average from another stimulus.

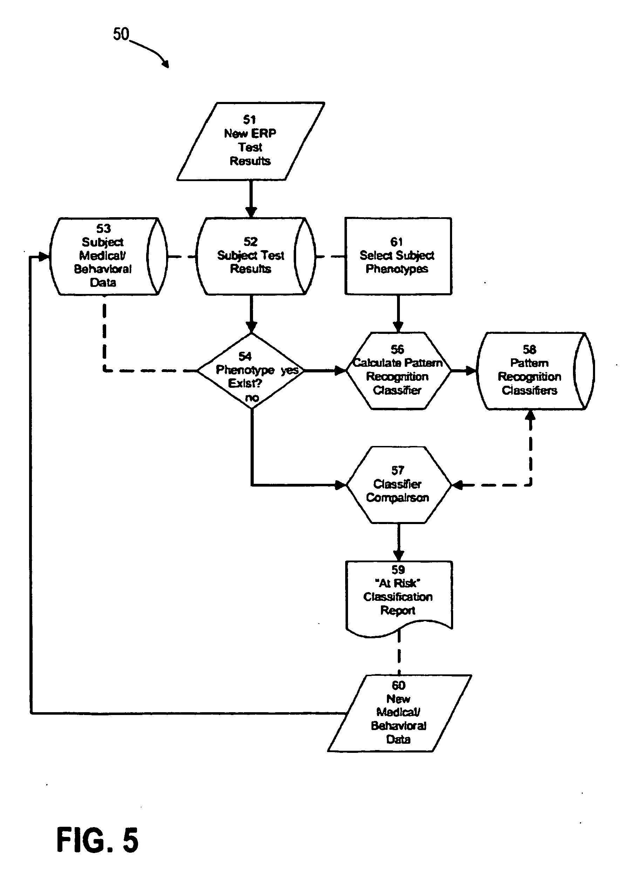

FIG. 5 is an exemplary diagram which shows the logic for creating pattern recognition classifiers and for comparing new biopotential waveforms (ERPs) 40 to the classifiers for the purpose of performing a predictive diagnosis using a biopotential waveform classification system 50.

New ERPs 40 are captured (block 51) using an ERP system FIG. 1. The ERPs 40 are uploaded to a subject test results database (block 52). If medical or behavioral data (block 53) about the test subject which can describe a particular phenotype of interest (block 54) exists, then the ERPs (block 52) are passed to the pattern recognition classifier (block 56). The ERPs 40, along with a phenotype identifier (block 53), are then used to train (block 56) the pattern recognition classification comparator (block 57) to detect the phenotypes of interest. The classifiers are then stored in the pattern recognition classifier database (block 58) for later use.

If the new ERP subject test results (block 51) do not include medical or behavioral data and are to be used to perform a classification (block 57) then the ERPs (block 51) are then compared (block 57) to the stored classifiers (block 58) and a classification report (block 59) is created.

If, at some later time, new medical or behavioral data (block 60) which could correlate with a particular subject phenotype (block 61) is generated, the subjects original ERPs (block S1) can then be used to begin a new classifier training loop (blocks 52, 54, 56, 57, and 59). This feedback loop has the advantage of constantly improving the classification accuracy and performance of the ERP classification system 50.

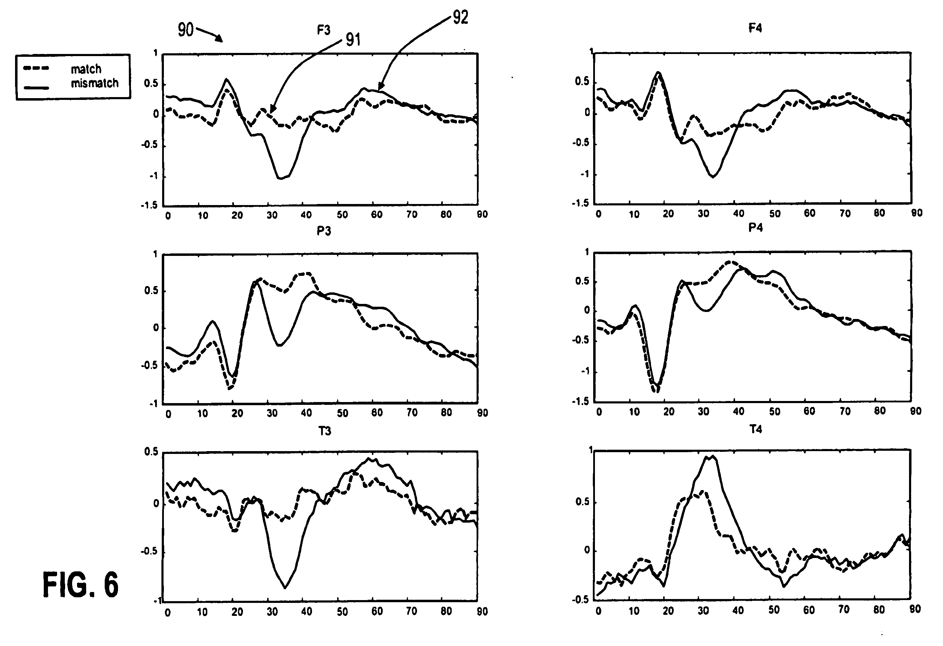

Analyses of multi-channel ERPs show that different channels reflect different aspects of the brain activity from the presentation of an external stimulus [2]. For example, consider the ensemble averaged ERPs of 6 different channels 90 shown in FIG. 6. Each graph shows ERPs belonging to 2 different categories (match 91 and mismatch 92). The ERPs within the same category (responses to the same external input) are clearly different across the 6 channels and the differences between the 2 categories vary across the 6 channels. Furthermore, it is important to note that the major differences between the categories occur at different times across the 6 channels.

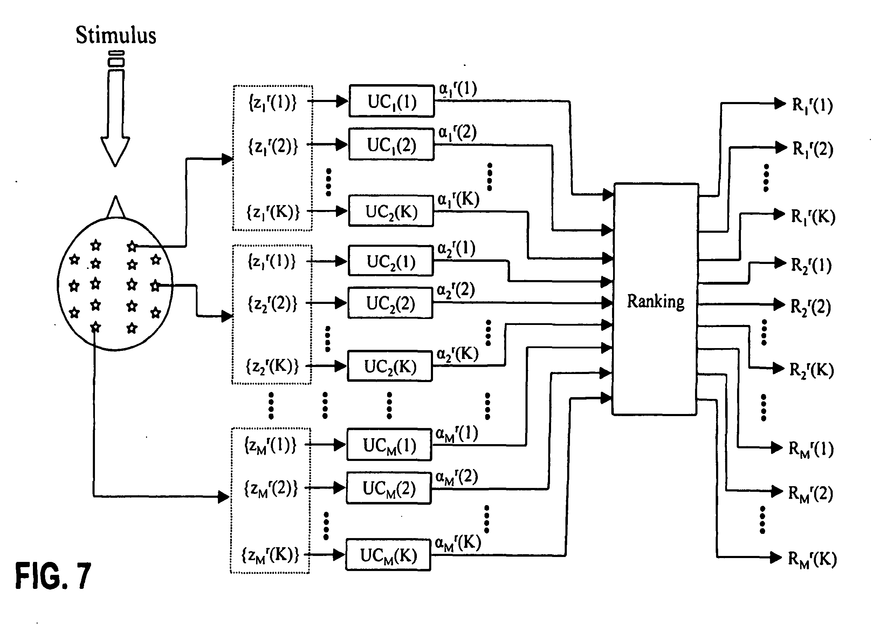

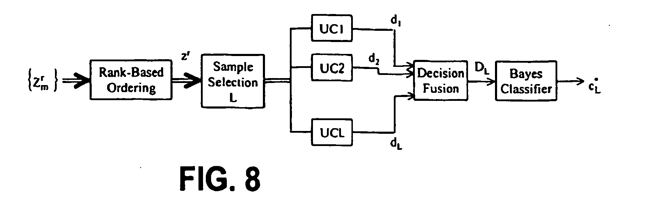

The new multi-channel decision fusion classification strategy is introduced to dynamically exploit the information from the multiple channels. The strategy is dynamic in the sense that different channels are selected at different time instants. The channels are ranked, at different time instants, according to their classification accuracies. During testing, the ERP sample of the selected channel and time-instants with the highest ranks are classified, independently, and the decisions are fused into a single decision fusion vector. The resulting decision fusion vector is classified using a discrete Bayes classifier. The design and testing phases of the decision fusion classification strategy are summarized in FIGS. 7 and 8, respectively. The notations used in the figures and in the mathematical formulations to follow are:

$Z^r_{m/c}$, $m = 1,2,..., M$; $c = 1,2,..., C$ = the ERP formed by averaging $r$ single-trial ERPs (referred to as r-EPs), where, $M$ is the number of channels and $C$ is the number of categories.

$z^r_{m/c}(k)$, $k = 1,2,..., K$ = the kth sample of $Z^r_{m/c}$ where $K$ is the number samples in each ERP.

$UC_m(k)$, $k = 1,2,..., K$ = the $k$th univariate classifier designed to classify sample $z^r_m(k)$.

$\alpha^r_m(k)$ = classification accuracy of $U^r_m(k)$

$R^r_m(k)$ = rank of element $z^r_m(k)$

$d_j$ = decision of classifier $UCj$
$D_L$ = decision fusion vector formed by combining the decisions of the channel-time elements with the $L$ highest classification accuracies.

In FIG. 7, $\{z^r_m(k)\}$ represents the entire C-class training set ensemble of the $k$th sample of channel $m$ that is used to determine the ranking. The EP samples of each channel are classified independently at each time instant and the classification accuracy is used to determine the ranking $R^r_m(k)$ of the element $z^r_m(k)$.

The steps involved in classifying an unknown multi-channel ERP signal are shown in FIG. 8. The entire set of $M$-channel test ERPs are represented by $\{Z^r_m\}$. Note that the first step of rank-based ordering is only a simple re-ordering of the samples of $\{Z^r_m\}$ according to the ranking as determined in the training stage. In the subsequent steps, only the elements with the highest $L$ classification accuracies are selected for classification and the decisions of the classifiers are fused into a single discrete random fusion vector $D_L$. The decisions are weighted using the *a priori* classification accuracy probabilities determined during the training stage and a separate discrete parametric classifier is designed to determine the ERP class from the decision fusion vector. The mathematical formulations of the strategy are described in detail next.

In generalized formulation, let the discriminant function for the $c$ class ERPs of channel $m$ at the sampling instant $k$ be

$$g^r_{k/m/c}[z^r_m(k)], \quad k = 1,2,...,K; m = 1,2,...,M; c = 1,2,...,C, \tag{1}$$

A test sample of channel $m$ at time $k$ is assigned to the discriminant function that yields the highest value. That is, the test sample $z^r_m(k)$ is assigned to the category $c^*_{k/m}$, $c^*_{k/m} \in \{c = 1,2,...,C\}$ given by

$$c^*_{k/m} = \arg \max_c \{g^r_{k/m/c}[z^r_m(k)]\} \tag{2}$$

The classification accuracy of the classifier for each channel *m* and time-instant *k* combination can be determined from the probability of classification error which is given by

$$P_m^r(k) = \sum_{c=1}^{C} P_m^r(k/c)P_c \qquad (3)$$

where $P_m^r(k/c)$ is the probability of misclassifying $z_m^r(k)$ when the true category of $z_m^r(k)$ is *c* and $P_c$ is the *a priori* probability of class *c*. The classification accuracy of the classifier, expressed as a percentage, for $z_m^r(k)$ is then given by

$$\alpha_m^r(k) = [1 - P_m^r(k)] \times 100\% . \qquad (4)$$

The total number of univariate classifiers are $M \times K$ and the $M \times K$ classifiers can be ranked according to their respective classification accuracies. If $R_m^r(k)$ is the rank of the classifier for $z_m^r(k)$ such that

$$R_m^r(k) < R_n^r(j) \;\; if \;\; \alpha_m^r(k) > \alpha_n^r(j); \quad all \; n, j; \; j \neq k \quad if \quad n = m \qquad (5)$$

$$R_m^r(k) = 1 \;\; if \;\; \alpha_m^r(k) > \alpha_n^r(j); \quad all \; n, j; \; j \neq k \; if \; n = m \qquad (6)$$

$$R_m^r(k) = M \times K \;\; if \;\; \alpha_m^r(k) < \alpha_n^r(j); \quad all \; n, j; \; j \neq k \; if \; n = m \qquad (7)$$

Let $Z^r$ be a new rank-ordered vector formed by re-arranging the samples $z_m^r(k)$, $k = 1,2,..., N; m = 1,2,..., M$ according to

$$z^r(j) = z_m^r(k) \;\; if \;\; R_m^r(k) = j \qquad (8)$$

where $z^r(j)$, $j = 1,2,...,M \times K$, is the jth element of $Z^r$. As a result of the ranking, $z^r(j)$ is ordered such that $z^r(1)$ is the channel-time ERP sample yielding the highest classification accuracy and $z^r(M \times K)$ is the channel-time sample yielding the lowest classification accuracy. Let $Z_L^r$ be the data vector formed by selecting the elements of $Z^r$ with the first L ranks and let the classification decision of element $z_L^r(j)$, $j = 1,2,...,L$, be $c_j^\bullet$, $c_j^\bullet \in \{c = 1,2,...,C\}$, where

$$c_j^\bullet = \arg \max_c \{g_{j/c}[z^r(j)]\} \qquad (9)$$

and

$$g_{j/c}[z^r(j)]; \quad j = 1,2,...,(M \times K), c = 1,2,...,C \qquad (10)$$

is the univariate discriminant function for $z^r(j)$ under class $c$. In order to fuse the decisions,

$$\text{let } d_j = c_j^\bullet \tag{11}$$

and let
$$D_L = \overset{L}{\underset{j=1}{\nabla}} d_j, \tag{12}$$

where $\nabla$ represents the concatenation operation. That is, $D_L = (d_1, d_2, ..., d_L)^T$ is the decision fusion vector formed by concatenating the independent decisions of the channel-time classifiers with the first $L$ ranks. The decision fusion vector $D_L$ is a discrete random vector in which each element can take one of $C$ values. Let the PDF of $D_L$ under category $c$ be $P(D_L / c)$. Then, the Bayes decision function for class $c$ is

$$g_c(D_L) = P_c P(D_L / c) \tag{13}$$

which can also be written as

$$g_c(D_L) = \ln P_c + \ln P(D_L / c) \tag{14}$$

The final decision $c_L^\bullet$ resulting from decision fusion is given by

$$c_L^\bullet = \arg \max_c [g_c(D_L)] \tag{15}$$

The discriminant function for the $C$-class case can be derived explicitly by letting

$$p_{j,a/c} = P(d_j = a / c), \, j = 1, 2, ..., L \tag{16}$$

That is, $p_{j,a/c}$ is the probability that $d_j = a$ when the true class is $c$. Then, the probability density function (PDF) of $D_L$ under the class $c$, $c=1,2, ... C$, can be written as

$$P(D_L / c) = \prod_{j=1}^{L} (p_{j,1/c})^{\delta(d_j - 1)} (p_{j,2/c})^{\delta(d_j - 2)} ... (p_{j,C/c})^{\delta(d_j - C)}, \tag{17}$$

where

$$\delta(x - a) = \begin{cases} 1 & \text{if} \quad x = a \\ 0 & \text{if} \quad x \neq a \end{cases} \tag{18}$$

By substituting the PDFs into Equation (27), it can be shown that the discriminant function for category $c$ can be written as

$$g_c(D_L) = \sum_{j=1}^{L} \left[ \delta(d_j - 1)\ln(p_{j,1/c}) + \delta(d_j - 2)\ln(p_{j,2/c}) + \ldots + \delta(d_j - C)\ln(p_{j,C/c}) \right] + \ln P_c \qquad (19)$$

The above formulation is general in the sense that the discriminant functions were not explicitly specified. If the univariate channel-time classifiers are the Euclidean distance nearest mean classifiers, then, the discriminant function in Equation (1) is given by

$$g^r_{k/m/c}[z^r_m(k)] = z^r_m(k)\bar{z}^r_{m/c}(k) - (1/2)[\bar{z}^r_{m/c}(k)]^2 \qquad (20)$$

where $\bar{z}^r_{m/c}(k)$ is the mean of $z^r_{m/c}(k)$ under category $c$. The discriminant function of Equation (10) is then given by

$$g_{j/c}[z^r(j)] = z^r(j)\bar{z}^r_c(j) - (1/2)[\bar{z}^r_c(j)]^2 \qquad (21)$$

where $\bar{z}^r_c(j)$ is the mean of $z^r(j)$ under category $c$.

If the univariate classifiers are Gaussian, then, the discriminant function in Equation (1) is given by

$$g^r_{k/m/c}[z^r_m(k)] = -\ln \sigma^r_{m/c}(k) - [z^r_m(k) - \mu_{m/c}(k)]^2 / 2[\sigma^r_{m/c}(k)]^2 + \ln P_c \qquad (21)$$

where $\mu_{m/c}(k)$ and $[\sigma^r_{m/c}(k)]^2$ are the mean and variance of $z^r_m(k)$ under class $c$, respectively, as computed using pairwise cross-correlation averaging. Note that $\mu^r_{m/c}(k) = \mu^1_{m/c}(k) = \mu_{m/c}(k)$. Similarly, the univariate Gaussian discriminant function of Equation (10) is given by

$$g_{j/c}[z^r(j)] = -\ln \sigma^r_c(j) - [z^r(j) - \mu_c(j)]^2 / 2[\sigma^r_c(j)]^2 + \ln P_c, \qquad (22)$$

where $\mu_c(j)$ and $[\sigma^r_c(j)]^2$ are the mean and variance of $z^r(j)$ under class $c$, respectively.

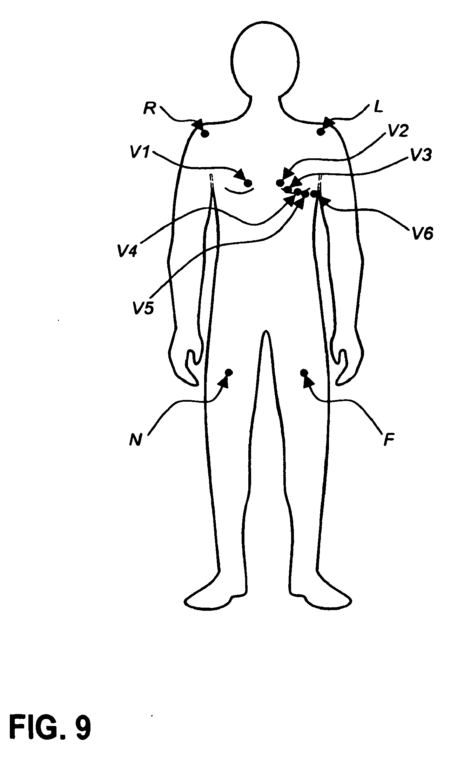

FIG. 9 shows a diagram of recommended electrode positions for continuous 12-lead ECG recording where the references R, L, V1, V2, V3, V4, V5, V6, N, and F represent standard electrode identifiers commonly know to those in the art.

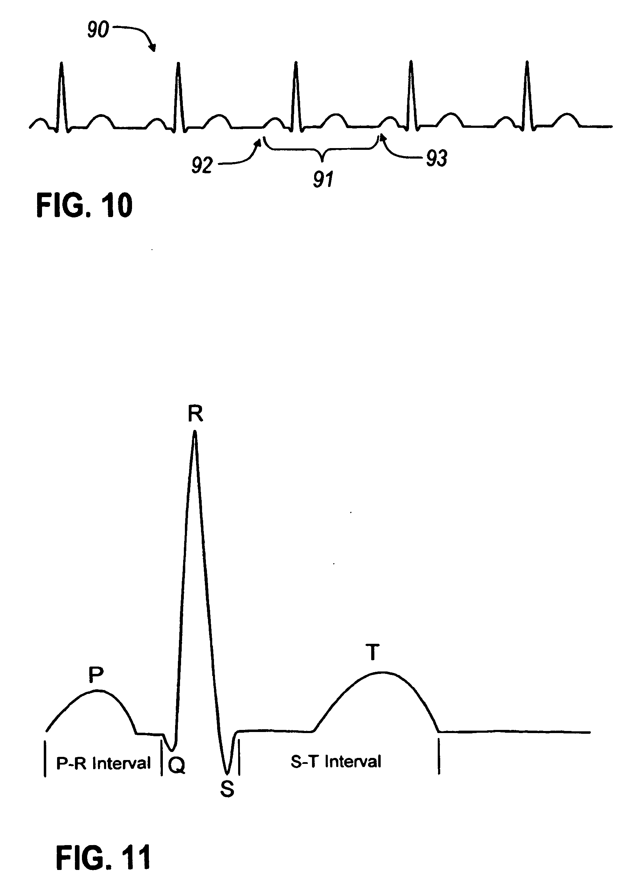

FIG. 10 shows a segment 90 of an ECG recorded from a normal subject. The repeating segment 91 which begins at point 92 and ends at point 93 represents a single heartbeat.

FIG. 11 shows an expanded view of segment 91 representing a single heartbeat. This segment includes common components, know to those in the art, such as the P-wave, P-R interval, Q-R-S complex, T-wave, and the S-T interval. This repeating segment 91, representing a single heartbeat, can be used in the same manner as an ERP waveform in the fusion classifier method described herein to identify abnormal or pathologic cardiac function.

PARAMETRIC CLASSIFICATION OF MULTICHANNEL AVERAGED EVENT-RELATED POTENTIALS.

This section focuses on the systematic development of a parametric approach for classifying averaged event-related potentials (ERPs) recorded from multiple channels. It is shown that the parameters of the averaged ERP ensemble can be estimated directly from the parameters of the single-trial ensemble, thus, making it possible to design a class of parametric classifiers without having to collect a prohibitively large number of single-trial ERPs. An approach based on random sampling without replacement is developed to generate a large number of averaged ERP ensembles in order to evaluate the performance of a classifier. A 2-class ERP classification problem is considered and the parameter estimation methods are applied to independently design a Gaussian likelihood ratio classifier for each channel. A fusion rule is formulated to classify an ERP using the classification results from all the channels. Experiments using real and simulated ERPs are designed to show that, through the approach developed, parametric classifiers can be designed and evaluated even when the number of averaged ERPs does not exceed the dimension of the ERP vector. Additionally, it is shown that the performance of a majority rule fusion classifier is consistently superior to the rule that selects a single best channel.

INTRODUCTION. The accurate classification of event-related potentials (ERPs) is crucial in numerous human cognition studies and in clinical applications. ERPs are brain responses time-locked to the onset of an external event. The external event may be a sensory stimulus such as a visual flash, an auditory sound or a small electrical impulse. The event may also be a cognitive or motor event. The measured single-trial response is modeled as a superposition of the ERP signal and the background electroencephalogram (EEG). The on-going EEG is regarded as background noise because it is activity not caused by the external stimulus. Because the stimulus-induced changes in the EEG are small, the ERP is estimated by averaging a set of responses obtained through a repetitive application of the external stimulus. Averaging reduces the effects of noise, thus, leading to an improvement in the signal-to-noise ratio (SNR) in averaged ERPs.

Because the SNR improves by averaging single-trial responses, it could be expected that the classification accuracy would increase by averaging single-trial responses. The responses are averaged over $r$-trials by partitioning the sequentially ordered single-trial ensemble into sets, each of size $r$, and computing the average of each set. For convenience, single-trial responses will be referred to as 1-ERPs and responses averaged over $r$ trials will be referred to as $r$-ERPs. In practice, only a finite number of single-trial responses can be collected, therefore, the size of the 1-ERP ensemble is finite. Therefore, when $r$ is increased in order to improve the classification accuracy, the number of averaged ERPs in the $r$-ERP ensemble decreases. As a result, the size of the $r$-ERP ensemble may not be sufficient to design and evaluate $r$-ERP classifiers. A simple solution to these problems would be to collect a very large number of single-trial responses to form a sufficiently large $r$-ERP ensemble. However, this is not a practical solution because individuals experience fatigue and lose concentration when the ERP experiment is repeated a large number of times. In this section, it is shown that the parameters of the smaller $r$-ERP ensemble can be estimated from those of the larger 1-ERP ensemble. It is thus possible to design and evaluate a class of parametric classifiers even when the number of $r$-ERPs is smaller than the dimension of the $r$-ERP vector.

ESTIMATING PARAMETERS OF AVERAGED ERPs. Parametric classifiers such as the Gaussian classifier, quadratic minimum distance classifier, and the Fisher linear discriminant classifier require the estimation of the mean and the covariance matrix of the feature vectors for each class. Additionally, the inverse of the covariance matrix must be computed. The mean and covariance are estimated from the design set selected from the $r$-ERP ensemble. Let $\Delta_r$ be the number of $r$-ERPs in the design set and $K$ be the dimension of the ERP vector. When $\Delta_r < K$, the estimate of the covariance matrix from the design set will be poor and problems occur in computing the inverse of the covariance matrix because it will be singular. These problems can be solved if the parameters of the smaller $r$-ERP set can be determined from those of the larger 1-ERP set.

In order to show how the parameters of the 1-ERP ensemble and the $r$-ERP ensemble are related, consider the discrete additive model that is most often assumed to describe a single-trial ERP resulting from the application of an external stimulus:

$$Z = S + N,$$

where, $Z$ is the measured single-trial response, $S$ is the ERP, and $N$ is the on-going EEG signal. It is assumed that $S$ and $N$ are independent, $S$ is deterministic, and $N$ is a zero mean correlated process. Let $Z^r$ denote the response obtained by averaging $r$ single-trial responses. Then,

$$Z^r = S + N^r,$$

where, $N^r$ is the average of the $r$ EEG vectors in $Z^r$. From the multivariate central limit theorem, it can be assumed that the averaged noise vector $N^r$ is Gaussian. Clearly,

$$E[N^r] = E[N] = 0$$

$$E[Z^r] = E[Z] = S,$$

where, E[.] is the expectation operator. If $C_n$, $C_n^r$, and $C_z^r$ are the covariance matrices of $N$, $N^r$, and $Z^r$, respectively, it can be shown that

$$C_z^r = C_n^r = (1/r)C_n .$$

It can also be shown that

$$[C_z^r]^{-1} = [C_n^r]^{-1} = r[C_n]^{-1}.$$

That is, the mean, covariance, and the inverse of the covariance matrix of the smaller $r$-ERP ensemble can be determined directly from those of the larger 1-ERP ensemble. For $C_n$ to be nonsingular, the number of single-trial ERPs in the design set must be greater than the dimension of the ERP vector. That is, $\Delta_1 > K$, where, $\Delta_1$ is the number of single-trial ERPs in the 1-ERP design set.

Dimensionality reduction through principal component analysis (PCA) is often employed in pattern recognition problems to improve classification by removing redundant information from the measured vectors. PCA requires the computation of the eigenvalues and eigenvectors of the covariance matrix. For the r-ERP case, problems in computing the eigenvalues and eigenvectors occur when the number of $r$-ERPs is less than the dimension of the response vectors. When $\Delta_r < K$, the largest number of meaningful eigenvectors that can be determined is ($\Delta_r$ - 1) because only ($\Delta_r$ - 1) eigenvalues will be non-zero.

In order to solve this problem, let $\lambda_k$, $k$ = 1,2,...,$K$ be the eigenvalues of $C_n$ and let $\Phi_n$ be the eigenvector matrix of the corresponding eigenvectors $\phi_k$. Then, the following equation must be satisfied:

$$C_n\phi_k = \lambda_k\phi_k. \tag{23}$$

Similarly, if $\lambda_k^r$, $k$ = 1,2,....,$K$, are the eigenvalues of $C_n^r$ and $\Phi_n^r$ is the eigenvector matrix of the corresponding eigenvectors $\phi_k^r$, then,

$$C_n^r\phi_k^r = \lambda_k^r\phi_k^r . \tag{24}$$

Substituting $C_n^r = (1/r)C_n$ in Equation (2) gives,

$$C_n\phi_k^r = r\lambda_k^r\phi_k^r . \tag{25}$$

By comparing Equations (23) and (25), it follows from the uniqueness of the eigenvalues of a matrix that,

$$\lambda_k^r = (1/r)\lambda_k . \tag{26}$$

The eigenvectors, however, are not unique. Therefore, $\phi_k^r$ can be replaced by $\phi_k$ in Equation (24). Therefore, Equation (24) can be written as

$$C_n^r\phi_k = [(1/r)\lambda_k]\phi_k. \tag{27}$$

Equation (27) shows that the eigenvectors of the covariance matrix of the 1-ERP ensemble are also the eigenvectors of the covariance matrix of the *r*-ERP ensemble. The eigenvalues of the covariance matrix of the *r*-ERP ensemble can be determined directly from those of the 1-ERP covariance matrix using Equation (26).

MULTICHANNEL ERP CLASSIFIER DEVELOPMENT. This section focuses on the development of a multichannel classification methodology to classify averaged ERPs into one of two classes. The formulation of the methodology is described in terms of classifying match and mismatch ERPs, however, it is important to note that it is applicable to general 2-class ERP classification problems. Numerous studies in human cognition relating to attention, memory, perception, and language comprehension employ ERPs to study the relationships that exist between cognitive processes and changes in the brain's electrical activity. In one such class of studies involving simple discrimination tasks, subjects are required to decide on the correspondence between two sequentially presented stimuli by partitioning the stimuli into two mutually exclusive classes: a "match" if the two stimuli are the same and a "mismatch" if the stimuli are different. In certain experiments designed to study the brain activity time-course due to infrequent stimuli, as in the "oddball paradigm," the second stimulus is presented such that mismatch condition is less likely to occur than the match condition. In other experiments involving explicit unbiased comparisons between two stimulus conditions, the second stimulus is presented so that the match and mismatch conditions are equally likely to occur. It has been shown, using visual analysis of averaged ERPs and ANOVAs, that mismatch or unexpected stimuli are characterized by increased negativity in the 200 to 600 ms range. There are also indications that mismatch and unexpected stimuli may actually be associated with an increased amplitude of late positive components that follow the N400 response. Additionally, there is evidence from match/mismatch experiments that ERPs of different channels reflect different aspects of cognitive comparisons. Therefore, in order to accurately classify an averaged response as a match or a mismatch ERP, the information from each channel can be captured by designing a classifier independently for each channel and then fusing the results obtained from each channel.

In the formulations to follow, the channels are represented by $\varepsilon_i$, $i = 1,2,...,I$ where $I$ is the number of channels. Let the two sequentially presented stimuli for the *j*th trial be denoted by $S_{j1}$ and $S_{j2}$, where, $S_{j1} = S_{j2}$ and $S_{j1} \neq S_{j2}$ with probabilities $P_0$ and $P_1$, respectively. If $Z_{ij}$ is the subject's response at channel $\varepsilon_i$, then,

$$Z_{i,j} = \begin{cases} Z_{i,j,x} & \text{if } S_{j1} = S_{j2} \\ Z_{i,j,y} & \text{if } S_{j1} \neq S_{j2} \end{cases}$$

where, $Z_{i,j,x}$ and $Z_{i,j,y}$ denote single-trial responses recorded as match and mismatch, respectively.

Because the averaged noise vector $N^r$ is assumed Gaussian, the classification of an averaged *r*-ERP $Z_i^r$ recorded at channel $\varepsilon_i$, $i = 1,2,...,I$, can be formulated as a problem of detecting two signals in Gaussian noise. It will be initially assumed that the two signals and the covariance of the noise are known. If $X_i$, $Y_i$, and $N_i^r$ are the match ERP, mismatch ERP, and the noise vector, respectively, then the two hypotheses for match/mismatch signal detection at channel $\varepsilon_i$ can be written as:

$$H_0: Z_i^r = X_i + N_i^r; \quad \text{a match condition occurred}$$

$$H_1: Z_i^r = Y_i + N_i^r; \quad \text{a mismatch condition occurred.}$$

From the ERP model assumptions and the Gaussian assumption, $N_i^r$ is Gaussian with zero mean and covariance $C_{i,n}^r$.

Because the components of the responses are correlated, redundant information can be removed by first decorrelating the components and then applying a criterion to select a subset of the decorrelated components. A modification of PCA for feature selection is formulated to decrease redundant information from the responses. Let $\lambda_{i,k}^r$, $k = 1,2,...,K$, be the eigenvalues of $C_{i,n}^r$ and let $\Phi_{i,n}^r$ be the eigenvector matrix of the corresponding eigenvectors $\phi_{i,k}^r$. Using $\Phi_{i,n}^r$ for the transformation matrix, the transformed responses $\tilde{Z}_i^r$ are given by

$$\tilde{Z}_i^r = (\Phi_{i,n}^r)Z_i^r.$$

The transformed match, mismatch, and noise vectors are given by

$$\tilde{X}_i^r = (\Phi_{i,n}^r)X_i$$

$$\tilde{Y}_i^r = (\Phi_{i,n}^r)Y_i,$$

and

$$\tilde{N}_i^r = (\Phi_{i,n}^r)N_i^r,$$

respectively. Under the transformation, $\tilde{N}_i^r$ remains Gaussian with zero-mean and covariance $\tilde{C}_{i,n}^r$. $\tilde{C}_{i,n}^r$ is a diagonal matrix whose elements along the main diagonal are the eigenvalues $\lambda_{i,k}^r$, $k = 1,2,...,K$ of $C_{i,n}^r$. That is, the components of $\tilde{N}_i^r$ are uncorrelated. Because $\tilde{N}_i^r$ is Gaussian, the components are also independent.

Although the principal components (components corresponding to the highest variance) are often selected as a means to decrease the dimension of a feature vector, the principal components may not necessarily be the best for separating pattern classes. Therefore, instead of selecting the components with the highest variance as in PCA, we select the components with the highest inter-class separation between the two ERP classes. If the transformed match and mismatch responses are denoted by $\tilde{Z}_{i,x}^r$ and $\tilde{Z}_{i,y}^r$, respectively, the inter-class separation between the corresponding component $k$, $k = 1,2,...,K$, in the match and mismatch responses is given by

$$\beta(k) = \frac{\left[\overline{\tilde{Z}}_{i,x}^r(k) - \overline{\tilde{Z}}_{i,y}^r(k)\right]^2}{\sum_{j=1}^{\Delta_{x,r}}\left[\tilde{Z}_{i,j,x}^r(k) - \overline{\tilde{Z}}_{i,x}^r(k)\right]^2 + \sum_{j=1}^{\Delta_{y,r}}\left[\tilde{Z}_{i,j,y}^r(k) - \overline{\tilde{Z}}_{i,y}^r(k)\right]^2},$$

where, $\tilde{Z}_{i,j,x}^r(k)$ is the kth component of the jth match response and $\overline{\tilde{Z}}_{i,x}^r$ is the mean of the kth component of $\tilde{Z}_{i,x}^r$. $\Delta_{x,r}$ $\Delta_{x,r}$ and $\Delta_{y,r}$ are the number of $r$-ERPs in the match and mismatch design sets, respectively. The unordered eigenvalues and the eigenvectors in the matrix $\Phi_{i,n}^r$ are arranged in decreasing order of separation. The transformed response of reduced dimension $\hat{K}_i$ is given by

$$\hat{Z}_i^r = (\hat{\Phi}_{i,n}^r)Z_i^r,$$

where, $\hat{\Phi}_{i,n}^r$ is the $(\hat{K}_i \times K)$ truncated matrix consisting of the first $\hat{K}_i$ eigenvectors selected from $\Phi_{i,n}^r$ which yield the highest inter-class separations between the components of the transformed match and mismatch responses. The truncated match, mismatch, and noise vectors of dimension $\hat{K}_i$ are given by

$$\hat{X}_i^r = (\hat{\Phi}_{i,n}^r)X_i$$

$$\hat{Y}_i^r = (\hat{\Phi}_{i,n}^r)Y_i,$$

and

$$\hat{N}_i^r = (\hat{\Phi}_{i,n}^r)N_i^r,$$

respectively. The two hypotheses can now be written as

$$H_0: \hat{Z}_i^r = \hat{X}_i + \hat{N}_i^r; \quad \text{a match condition occurred}$$

$$H_1: \hat{Z}_i^r = \hat{Y}_i + \hat{N}_i^r; \quad \text{a mismatch condition occurred.}$$

The truncated noise vector $\hat{N}_i^r$ is Gaussian with $\mathbf{E}[\hat{N}_i^r] = \mathbf{0}$. Let $\hat{C}_{i,n}^r$ be the $(\hat{K}_i \times \hat{K}_i)$ covariance matrix of $\hat{N}_i^r$. $\hat{C}_{i,n}^r$ is a diagonal matrix whose diagonal elements $\hat{\lambda}_{i,k}^r$, $k = 1,2,...,\hat{K}_i$, are the eigenvalues of $C_{i,n}^r$ corresponding to the selected eigenvectors of $C_{i,n}^r$. The conditional probability density functions of the truncated responses under the two hypotheses are:

$$p(\hat{Z}_i^r \mid H_0) = (2\pi)^{-\hat{K}/2}\left|\hat{C}_{i,n}^r\right|^{-1/2}\exp\left\{-\tfrac{1}{2}(\hat{Z}_i^r - \hat{X}_i^r)^{\mathsf{T}}(\hat{C}_{i,n}^r)^{-1}(\hat{Z}_i^r - \hat{X}_i^r)\right\} \tag{28}$$

$$p(\hat{Z}_i^r \mid H_1) = (2\pi)^{-\hat{K}/2}\left|\hat{C}_{i,n}^r\right|^{-1/2}\exp\left\{-\tfrac{1}{2}(\hat{Z}_i^r - \hat{Y}_i^r)^{\mathsf{T}}(\hat{C}_{i,n}^r)^{-1}(\hat{Z}_i^r - \hat{Y}_i^r)\right\} \tag{29}$$

The likelihood ratio decision rule for this case is:

$$\Lambda(\hat{Z}_i^r) = p(\hat{Z}_i^r \mid H_1)/p(\hat{Z}_i^r \mid H_0) \mathop{\gtrless}_{H_0}^{H_1} (P_0/P_1) = \eta. \tag{30}$$

Taking logarithms and rearranging terms after substituting Equations (28) and (29) in Equation (30) gives

$$(\hat{Z}_i^r)^{\mathsf{T}}(\hat{C}_{i,n}^r)^{-1}[\hat{Y}_i^r - \hat{X}_i^r] \mathop{\gtrless}_{H_0}^{H_1} \ln\eta + \tfrac{1}{2}\{(\hat{Y}_i^r)^{\mathsf{T}}(\hat{C}_{i,n}^r)^{-1}(\hat{Y}_i^r) - (\hat{X}_i^r)^{\mathsf{T}}(\hat{C}_{i,n}^r)^{-1}(\hat{X}_i^r)\} \tag{31}$$

The decision rule in Equation (31) was derived by assuming that the match ERP $X_i$, the mismatch ERP $y_i$, and noise covariance matrix $C_{i,n}^r$ are known. In practice, they are unknown but can be estimated from the design sets and substituted in Equation (31). The maximum likelihood estimates of $X_i$ and $Y_i$ are given by the sample means of the respective design sets, that is,

$$\overset{\bullet}{X}_i = 1/(\Delta_x)\sum_{j=1}^{\Delta_x} Z_{i,j,x} \tag{32a}$$

$$\overset{\bullet}{Y}_i = 1/(\Delta_y)\sum_{j=1}^{\Delta_y} Z_{i,j,y} \tag{32b}$$

where, $\Delta_x$ and $\Delta_y$ are the number of 1-ERPs in the match and mismatch design sets, respectively. Using the relationships developed in Section 2, $C_{i,n}^r$, $\lambda_{i,k}^r$ and $\Phi_{i,n}^r$ can be determined if $C_{i,n}$ is known. However, $C_{i,n}$ is also unknown but can be estimated from the responses. The maximum likelihood estimate of $C_{i,n}$ is the sample covariance, which can be determined from either the match or the mismatch design set. Alternatively, a better estimate of $C_{i,n}$ can be obtained from the larger set of size $\Delta = (\Delta_x + \Delta_y)$ consisting of the single-trial noise vectors of both the match and mismatch design sets whose estimates are given by

$$\overset{\bullet}{N}_{i,j,x} = Z_{i,j,x} - \overset{\bullet}{X}_i, \quad j = 1, 2,\ldots, \Delta_x$$

$$\overset{\bullet}{N}_{i,j,y} = Z_{i,j,y} - \overset{\bullet}{Y}_i, \quad j = 1, 2,\ldots, \Delta_y$$

respectively. If $\overset{\bullet}{N}_{i,j}$, $i = 1,2,\ldots, I$, are the single-trial noise vectors of the resulting mixture, then, $C_{i,n}$ can be estimated as

$$\overset{\bullet}{C}_{i,n} = (1/\Delta)\sum_{j=1}^{\Delta} (\overset{\bullet}{N}_{i,j})(\overset{\bullet}{N}_{i,j})^{\mathsf{T}}.$$

Note that for $\overset{\bullet}{C}_{i,n}$ to be nonsingular, $\Delta > K$, that is, the sum of the number of 1-ERPs in the match and mismatch design sets must exceed the dimension of the ERP vector.

FUSION RULE CLASSIFIER. A likelihood ratio classifier $L_i$, $i = 1, 2,\ldots, I$, can be designed independently for each of the $I$ channels. During a test, the response recorded at each channel is classified independently of the other channels. The classification results from all the channels can be fused, for example, by using the majority rule. That is, it is decided that a match (mismatch) condition occurred during a test if the majority of the classifiers decide a match (mismatch) condition occurred. Let $d_i$ be the decision made by classifier $L_i$ and let $d_i = 1$ if the classifier decides a match occurred and $d_i = 0$ if the classifier decides a mismatch occurred. Then, if $D = \sum_{i=1}^{I} d_i$, the decision rule for the majority rule fusion classifier is:

if $D > (I/2)$, a match condition occurred;
$D < (I/2)$, a mismatch condition occurred;
$D = (I/2)$, randomly decide between a match and mismatch condition.
Note that this rule is applicable for both even and odd values of $I$.

EMPIRICAL PERFORMANCE EVALUATION. The r-ERP ensemble is typically formed through sequential averaging. That is, by partitioning the single-trial responses, in the order they were collected, into sets of size $r$ and computing the average of each set. The cross-validation method and the leave-one-out method are the most often used methods to partition a given ensemble of each pattern class into a design set and a test set. The design set is used to estimate the parameters for classifier design and the test set is used to evaluate the performance of the classifier. A small number of $r$-ERPs in the test set will result in a poor evaluation of the classifier performance. In order to generate a larger set for performance evaluation, a resampling approach can be employed to form several $r$-ERP ensembles from a single ensemble consisting of $J$ single-trial ERPs. An ensemble consisting of ($J_r = J/r$) $r$-ERPs is formed by averaging *r* single-trial responses using sampling without replacement <u>prior</u> to dividing the *r*-ERP ensemble into the design and test sets. Instead of having just one *r*-ERP ensemble of size $J_r$ for classifier design and evaluation using sequential averaging, the random sampling method applied to the single-trial ERPs can generate up to $\{J!/[r!(J-r)!J]\}$ distinct *r*-ERP ensembles, each of size $J_r$. Distinct implies that no two r-ERPs in all possible ensembles are formed by averaging exactly the same *r* single-trial responses. Samples drawn from the same population whether sequentially or randomly must have the same statistics. Therefore, it directly follows that the first 2 moments of randomly sampled and sequentially sampled averaged ERPs must be equivalent in the first 2 moments for a given *r*. The cross-validation method or the leave-one-out method can be applied to design and evaluate the performance of the classifier using the $J_r$ *r*-ERPs of each ensemble. It is important to note that even though the number of *r*-ERPS in the design set may be small for large values of r, the classifier parameters can be estimated from the larger 1-ERP design set using the results developed in Section 2. A good estimate of the classifier performance is then possible by averaging the estimates of the classification accuracies obtained across a sufficiently large number of *r*-ERP ensembles. If $J_{x,r}$ and $J_{y,r}$ are the number of *r*-ERPs in the match and mismatch design sets of ensemble $\tau_r, \tau_r = 1,2,...,T_r$, respectively, the classification accuracy for the test *r*-ERPs of ensemble $\tau_r$ can be estimated as

$$\alpha(\tau_r) = \frac{\text{number of correctly classified ERPs in the test set of ensemble } \tau_r}{(J_{x,r} - \Delta_{x,r}) + (J_{y,r} - \Delta_{y,r})},$$

where, $T_r$ is the total number of *r*-ERP ensembles generated to evaluate the performance. The classification accuracy $\alpha_r\%$ of the classifier for a given *r* can then be estimated by averaging the classification accuracies over the $T_r$ ensembles. That is,

$$\alpha_r = [(1/T_r)\sum_{\tau_r=1}^{T_r}\alpha(\tau_r)]\times 100\%.$$

EXPERIMENTS AND EVALUATIONS. Data from two match/mismatch experiments involving explicit comparisons between 2 stimuli conditions were used to demonstrate the design and evaluation methods developed. The goal of these experiments was to show that ERPs can identify when a match occurs between what a subject thinks and sees. The two experiments differed in the representation used for the second stimulus. In both experiments, the match and mismatch conditions were equally likely, therefore, $\eta = 1$ in Equation (31).

In the first experiment, data for classifier design and evaluation was collected from a single subject, A, who was instructed to "think" about the first video stimulus (picture of an object) and then to respond whether the next video stimulus (picture of an object) matched or did not match the first stimulus. The period between the first and second stimulus varied randomly between 2 to 5 seconds. The response from each channel, time-locked to the second stimulus, was recorded as a match or mismatch depending on which of 2 keys was pressed by the subject. Responses due to incorrect key presses were discarded. Single trial ERPs were collected from 3 half-hour sessions. Each session was separated by a 10 minute break. ERP data were collected from I= 6 electrodes placed on the scalp over frontal (F3, F4), temporal (T3, T4), and parietal (P3, P4) regions over the left and right hemispheres. The 6 electrodes were referenced to linked earlobe leads. The electrooculogram (EOG) was also recorded with two electrodes placed lateral and below the left eye (bipolar montage). Single-trial ERPs were digitized over 1 sec using a 10 msec sampling period beginning 100 msec prior to stimulus onset. The 10 samples corresponding to the prestimulus period were removed, thus the dimension of the response vectors was decreased from 100 to $K = 90$. Trials in which the peak-to-peak amplitude exceeded 100 $\mu V$ in any one electrode channel or 50 $\mu V$ in the eye channel were regarded as artifacts and rejected. After rejecting artifacts, the ensembles consisted of $J_x = 280$ match and $J_y = 280$ mismatch single-trial responses. Given the nature of the experiment, ensemble sizes of 280 are relatively large.

The next set of experiments was similar to the first except that the second stimulus was a printed word instead of a picture. Data were collected from 4 subjects B1, B2, B3, and B4, who were instructed to decide whether a picture (first stimulus) and a printed word (second stimulus) matched in meaning. The response from each channel was recorded as a match or a mismatch if the subject decided that the sequentially presented stimuli matched or did not match in meaning, respectively. ERP data were collected from 14 channels (F7, F8, F3, F4, Fz, T3, T4, T5, T6, C3, C4, P3, P4, Pz) and processed exactly as in the first experiment. The data was collected over 2 sessions, each separated by a 10 minute break. An equal number of $J_x = 96$ match and $J_y = 96$ mismatch single-trial ERPs was selected from each subject's artifact-free ensemble to design and evaluate the classifiers for each subject.

For both experiments, the random sampling method described in Section 5 was used to generate enough $r$-ERP ensembles to test at least 200 r-ERPs. For each ensemble, the cross-validation technique was applied to design and test the classifiers using an equal number of r-ERPs in the design and test sets. That is, $\Delta_{x,r} = \Delta_{y,r}$ = Integer (140/$r$) in the first experiment and in the second experiment, $\Delta_{x,r} = \Delta_{y,r}$ = Integer (48/$r$).The dimension of the $r$-ERP vectors was reduced from $K$ = 90 to $\hat{K}_i$ =20, $i$ = 1,2,...,$I$, by selecting the 20 eigenvectors that accounted for the highest separation in the components of the transformed responses.

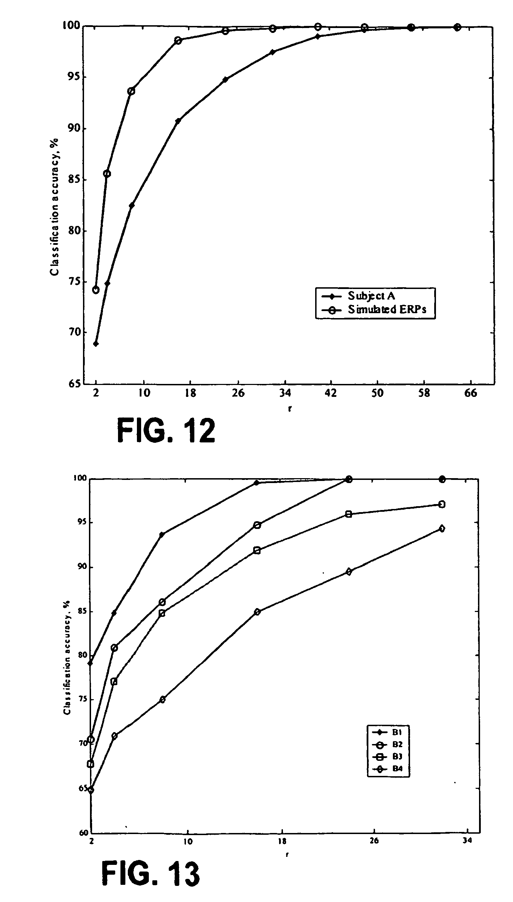

The first set of results presented is for Subject A who was involved in the first experiment. The classification accuracies for different values of $r$ obtained using the match and mismatch ERPs are shown in Table 1 for each channel as well as the fusion rule classifier. The classification accuracies, as a function of r, for the fusion rule classifiers are also shown graphically in Figure 12. These results were obtained by assuming that the real ERPs satisfied the single-trial ERP model described in Section 2. However, the assumptions that the ERP is deterministic and that the ERP and EEG are independent are questionable. It would, therefore, be interesting to compare these results with those that are obtained for ERPs simulated to satisfy the model's assumptions. Single-trial match and mismatch ERPs were simulated by adding the respective ensemble means of the real ERPs to a separate set of EEG signals collected from Subject A. By simulating ERPs in this manner, it can be assumed that the ERP signal is deterministic. Because the ERP is not estimated from the EEG ensemble, it can also be assumed that the ERP and the EEG are independent. In order for the comparisons to be meaningful, exactly the same number of match and mismatch responses that were used to obtain the results in Table 1 and Figure 12, were simulated. That is, an ensemble of 560 one-second EEG signals were collected from each of the 6 channels. The EEGs were digitized and processed exactly as the ERP data. A total of $J_x$ = 280 single-trial match and $J_x$ = 280 mismatch responses were simulated by adding the respective ensemble sample means to randomly selected EEGs from the EEG ensemble. The simulated match and mismatch ERPs were classified exactly as the real ERPs and the classification results are presented in Table 2 and the fusion results are superimposed in Figure 12.

Table 1. Classification accuracies for the real ERPs of Subject A

| r | Channel | | | | | | Fusion Rule |
|----|------|------|------|------|------|------|-------|
| | F3 | F4 | P3 | P4 | T3 | T4 | |
| 2 | 63.4 | 57.7 | 66.5 | 58.1 | 60.9 | 58.2 | 69.0 |
| 4 | 69.0 | 61.7 | 72.1 | 60.3 | 66.2 | 61.2 | 74.9 |
| 8 | 77.5 | 67.3 | 79.5 | 63.5 | 73.2 | 65.1 | 82.5 |
| 16 | 85.0 | 74.4 | 87.2 | 69.0 | 80.4 | 72.9 | 90.8 |
| 24 | 90.4 | 78.7 | 91.0 | 71.4 | 85.9 | 76.2 | 94.8 |
| 32 | 93.6 | 82.3 | 94.8 | 72.4 | 89.8 | 81.1 | 97.5 |
| 40 | 95.8 | 84.9 | 96.2 | 76.8 | 92.9 | 85.2 | 99.0 |
| 48 | 97.7 | 88.8 | 97.3 | 72.2 | 95.2 | 86.7 | 99.7 |
| 56 | 99.1 | 93.0 | 97.9 | 66.6 | 97.1 | 91.0 | 99.9 |
| 64 | 99.0 | 92.5 | 98.7 | 73.7 | 98.3 | 91.5 | 100.0 |

The next set of results presented is for the real ERP data collected from the 4 subjects B1 to B4. Table 3 shows the classification accuracies for each channel as well as the fusion rule classifiers. The classification accuracies of the fusion rule classifiers, as a function of r, are shown graphically in Figure 13. Note that each classification result and each fusion result presented in the 6 tables is estimated by testing 200 r-ERPs.

Table 2. Classification accuracies for simulated ERPs

| r | Channel | | | | | | Fusion Rule |
|----|------|------|------|------|------|------|-------|
| | F3 | F4 | P3 | P4 | T3 | T4 | |
| 2 | 68.9 | 61.4 | 67.5 | 60.7 | 61.8 | 58.7 | 74.3 |
| 4 | 80.7 | 73.6 | 80.4 | 62.9 | 67.9 | 65.4 | 85.7 |
| 8 | 91.8 | 75.0 | 92.5 | 63.9 | 86.1 | 67.5 | 93.6 |
| 16 | 97.1 | 76.9 | 93.7 | 70.0 | 88.2 | 80.0 | 98.6 |

(continued)

| r | Channel | | | | | | Fusion Rule |
|---|---|---|---|---|---|---|---|
| | F3 | F4 | P3 | P4 | T3 | T4 | |
| 24 | 98.9 | 81.1 | 94.6 | 88.3 | 95.0 | 81.4 | 99.6 |
| 32 | 99.0 | 89.6 | 97.9 | 94.3 | 96.8 | 84.3 | 99.8 |
| 40 | 100 | 94.6 | 98.9 | 94.7 | 98.6 | 87.5 | 100 |
| 48 | 100 | 95.0 | 99.6 | 95.6 | 98.9 | 91.8 | 100 |
| 56 | 100 | 97.5 | 99.6 | 98.2 | 98.9 | 92.1 | 100 |
| 64 | 100 | 98.9 | 100 | 98.7 | 99.6 | 95.4 | 100 |

Table 3(a) Classification accuracy; Subject B1

| r | Channel | | | | | | | | | | | | | | Fusion |
|---|---|---|---|---|---|---|---|---|---|---|---|---|---|---|---|
| | F7 | F8 | F3 | F4 | T3 | T4 | T5 | T6 | C3 | C4 | P3 | P4 | Fz | Pz | |
| 2 | 60.6 | 71.6 | 63.1 | 71.6 | 59.6 | 73.4 | 73.2 | 69.7 | 64.4 | 73.1 | 63.7 | 70.0 | 69.8 | 65.1 | 79.1 |
| 4 | 63.6 | 77.8 | 69.3 | 75.7 | 68.0 | 78.1 | 80.9 | 76.3 | 68.1 | 77.9 | 66.2 | 75.7 | 72.9 | 70.0 | 84.8 |
| 8 | 78.8 | 86.4 | 80.9 | 90.3 | 72.8 | 91.8 | 81.8 | 84.6 | 73.0 | 92.9 | 73.8 | 85.8 | 86.1 | 76.2 | 93.7 |
| 16 | 77.9 | 91.9 | 88.0 | 97.6 | 81.0 | 95.9 | 92.1 | 95.0 | 83.1 | 96.5 | 79.6 | 95.5 | 96.1 | 88.4 | 99.5 |
| 24 | 84.0 | 96.5 | 88.5 | 97.5 | 74.3 | 99.8 | 99.3 | 97.3 | 86.5 | 98.3 | 85.0 | 99.0 | 85.3 | 91.0 | 100 |
| 32 | 84.5 | 96.8 | 95.0 | 100 | 85.8 | 100 | 99.8 | 97.3 | 91.3 | 100 | 84.5 | 99.0 | 95.0 | 92.8 | 100 |

Table 3(b) Classification accuracy; Subject B2

| r | Channel | | | | | | | | | | | | | | Fusion |
|---|---|---|---|---|---|---|---|---|---|---|---|---|---|---|---|
| | F7 | F8 | F3 | F4 | T3 | T4 | T5 | T6 | C3 | C4 | P3 | P4 | Fz | Pz | |
| 2 | 53.7 | 57.3 | 58.5 | 60.3 | 63.0 | 60.9 | 58.2 | 63.1 | 61.4 | 67.4 | 61.0 | 63.9 | 64.1 | 67.1 | 70.6 |
| 4 | 67.3 | 65.6 | 66.2 | 70.1 | 73.3 | 70.3 | 70.3 | 62.6 | 74.4 | 73.6 | 66.1 | 66.7 | 72.1 | 65.3 | 80.9 |
| 8 | 73.8 | 67.4 | 81.3 | 77.1 | 83.9 | 69.4 | 72.9 | 73.6 | 82.0 | 73.5 | 75.1 | 69.4 | 76.7 | 71.2 | 86.1 |
| 16 | 74.5 | 80.4 | 79.4 | 87.8 | 92.9 | 87.8 | 82.4 | 80.6 | 93.1 | 90.8 | 77.3 | 79.9 | 84.1 | 74.3 | 94.8 |
| 24 | 87.0 | 84.8 | 78.5 | 84.8 | 96.5 | 93.3 | 93.0 | 89.0 | 98.0 | 86.3 | 88.8 | 96.8 | 76.0 | 88.0 | 100 |
| 32 | 92.5 | 88.3 | 99.5 | 96.5 | 91.5 | 87.8 | 86.8 | 92.8 | 99.5 | 98.5 | 95.0 | 85.0 | 95.0 | 72.5 | 100 |

Table 3(c) Classification accuracy; Subject B3

| r | Channel | | | | | | | | | | | | | | Fusion |
|---|---|---|---|---|---|---|---|---|---|---|---|---|---|---|---|
| | F7 | F8 | F3 | F4 | T3 | T4 | T5 | T6 | C3 | C4 | P3 | P4 | Fz | Pz | |
| 2 | 61.3 | 51.3 | 61.8 | 55.2 | 58.6 | 60.4 | 55.9 | 61.4 | 62.4 | 57.9 | 60.7 | 62.0 | 56.3 | 60.7 | 67.8 |
| 4 | 69.5 | 49.9 | 68.3 | 64.4 | 62.5 | 65.5 | 57.5 | 68.3 | 73.9 | 64.7 | 64.3 | 64.8 | 60.1 | 68.3 | 77.1 |
| 8 | 71.2 | 48.6 | 77.9 | 68.8 | 66.4 | 72.8 | 59.6 | 76.9 | 79.0 | 70.3 | 62.1 | 70.8 | 72.9 | 74.0 | 84.8 |
| 16 | 73.0 | 55.9 | 81.1 | 75.5 | 74.0 | 77.1 | 70.1 | 84.0 | 89.1 | 76.9 | 69.8 | 85.5 | 72.9 | 77.4 | 91.9 |
| 24 | 80.5 | 58.0 | 88.5 | 87.5 | 81.0 | 76.5 | 63.0 | 85.0 | 93.0 | 92.5 | 78.0 | 75.5 | 80.5 | 84.0 | 96.0 |
| 32 | 85.9 | 48.0 | 94.3 | 88.0 | 78.3 | 59.6 | 61.9 | 78.5 | 92.6 | 79.6 | 83.3 | 86.9 | 84.1 | 85.5 | 97.1 |

Table 3(d) Classification accuracy; Subject B4

| r | Channel | | | | | | | | | | | | | | Fusion |
|---|---|---|---|---|---|---|---|---|---|---|---|---|---|---|---|
| | F7 | F8 | F3 | F4 | T3 | T4 | T5 | T6 | C3 | C4 | P3 | P4 | Fz | Pz | |
| 2 | 54.3 | 55.9 | 58.2 | 60.4 | 60.8 | 58.1 | 55.4 | 56.1 | 59.4 | 58.4 | 56.1 | 58.0 | 55.9 | 59.1 | 64.9 |
| 4 | 56.2 | 57.4 | 61.0 | 63.7 | 66.3 | 66.2 | 61.2 | 60.8 | 64.3 | 65.9 | 62.0 | 63.2 | 61.4 | 66.5 | 70.9 |
| 8 | 57.9 | 62.4 | 67.5 | 72.0 | 73.5 | 65.8 | 61.1 | 65.1 | 66.4 | 67.9 | 64.1 | 61.2 | 62.9 | 68.6 | 75.0 |
| 16 | 64.9 | 66.9 | 70.8 | 75.3 | 79.6 | 76.6 | 75.5 | 68.1 | 84.9 | 76.6 | 74.8 | 75.9 | 76.6 | 81.3 | 85.0 |
| 24 | 68.5 | 67.8 | 85.8 | 80.8 | 85.8 | 83.0 | 66.8 | 67.8 | 84.0 | 79.5 | 62.8 | 73.3 | 69.8 | 79.8 | 89.5 |
| 32 | 70.3 | 78.5 | 80.0 | 84.8 | 92.3 | 81.0 | 85.3 | 81.0 | 86.3 | 85.3 | 77.0 | 77.3 | 79.5 | 86.5 | 94.3 |

By examining the results, it is important to note the following: (a) The trends in the classification results in Tables 1 and 2 and in Figure 12, for the real and simulated ERPs, are similar. The classification accuracies for each channel and the fusion rule classifiers increased when $r$ was increased. Higher classification accuracies, especially for smaller values of $r$, were obtained with the simulated ERPs. This result is not unexpected because the assumed ERP model is satisfied better for the simulated ERPs. Most importantly, it should be noted that for higher values of $r$ ($r > 32$), the fusion rule classification accuracies for the real and simulated ERPs are very close. These results are quite significant because, although the additive model that is implicitly assumed in ERP averaging is questionable, the improvement in classification accuracy shows that averaging does improve the SNR of real ERPs.

(b) The fusion rule classifier gives better results than the rule that selects a single best channel if it were known a priori which channel gives the best classification accuracy. An additional problem that could occur with the best channel selection rule is that a single channel may not be the best consistently. The fusion rule, however, does not require such a priori knowledge and is not affected by the variability in the best channels.

(c) Although the classification results for the 4 subjects in the second experiment show individual differences, which is not unexpected, the results have a similar trend. Additionally, it is interesting to note that even though the match/mismatch experiments were not the same for the 1-subject and the 4-subject cases, the trends in the results for both cases are quite similar.

(d) The results can also be used to roughly determine the number of real 1-ERPs that would be required to obtain a desired classification accuracy if the approach developed in this section is not used. For example, if an average classification accuracy of over 90% is desired for subject A, the smallest value of $r$ as determined from Table 1 is r=16. Therefore, the number of single-trial responses that should be collected for each match and mismatch ensemble, to obtain the desired classification accuracy, must satisfy $J/[(16)] > (K = 90)$, i.e., $J > 1440$. This number of single-trial ERPS is a prohibitively large to collect in practice. Alternatively, it would not have been possible to design and evaluate $r$-ERP classifiers for $r > 3$ given that the dimension of the ERPs is $K = 90$ and each ensemble contained 280 responses.

CONCLUSIONS. This section focused on problems encountered in designing and evaluating classifiers for averaged ERPs. Although parametric classifiers appear to be difficult to design and evaluate for $r$-ERPs, it is shown that parametric $r$-ERP classifiers, in fact, offer an attractive solution provided that the classifier parameters can be estimated from those of the 1-ERP ensemble. It is shown that the mean and covariance of the smaller r-ERP ensemble can be estimated from those of the larger 1-ERP ensemble. It is also shown that the eigenvalues and eigenvectors of the $r$-ERPs can be determined from the eigenvalues and eigenvectors of the 1-ERPs. These results were systematically applied to design a Gaussian likelihood ratio classifier for each channel for 2-class ERP classification problems. A majority rule was proposed to fuse the classification results from each channel. Additionally, an approach based on random sampling was developed to generate a large number of r-ERP ensembles to test the performance of $r$-ERP classifiers. A range of values of $r$ that did satisfy and did not satisfy the condition ($\Delta_r > K$) were considered in two match/mismatch classification problems. It was shown that the classification results had similar trends across the experiments, across the subjects, and across the real and simulated ERPs. The consistently superior performance of the fusion rule classifier shows the merit of using the classification information from all the channels rather than selecting a single best channel. In summary, the systematic approach developed makes it possible to design and evaluate a class of parametric ERP classifiers, even for responses averaged over a large number of single-trials, without having to collect an impracticably large number of single-trial responses.

MULTI-STIMULI MULTI-CHANNEL DATA AND DECISION FUSION STRATEGIES FOR DYSLEXIA PREDICTION USING NEONATAL ERPS.

Data fusion and decision fusion classification strategies are introduced to predict dyslexia from multi-channel event related potentials (ERPs) recorded, at birth, in response to multiple stimuli. Two data and 2 decision fusion strategies are developed in conjunction with nearest-mean classification rank selection to classify multi-stimuli multi-channel (MSMC) ERPs. The fusion vector in the data fusion strategy is formed by directly combining the rank-ordered MSMC ERP vectors or the rank-ordered elements of the MSMC ERPS. The resulting fusion vector is classified using a vector nearest-mean classifier. The nearest-mean classification decisions of the rank-ordered MSMC ERP vectors or the rank-ordered MSMC ERP elements are combined into a fusion vector in the decision fusion strategy. The resulting decision fusion vector is classified using a discrete Bayes classifier. The MSMC fusion classification strategies are tested on the averaged ERPs recorded at birth of 48 children: 17 identified as dyslexic readers, 7 as poor readers, and 24 identified as normal readers at 8 years of age. The ERPs were recorded at 6 electrode sites in response to 2 speech sounds and 2 non-speech sounds. It is shown that through the MSMC ERP element decision fusion strategy, dyslexic readers and poor readers can be predicted with almost 100% accuracy. Consequently, future reading problems can be detected early using neonatal responses making it possible to introduce more effective interventions earlier to children with reading problems emerging later in their lives. Furthermore, it is noted that because of the generalized formulations, the fusion strategies introduced can be applied, in general, to problems involving the classification of multi-category multi-sensor signals.

INTRODUCTION. The aim of this investigation is to show, through data and decision fusion classification strategies, that information from multi-stimuli event related potentials (ERPs) recorded at birth from multiple channels can predict dyslexia with very high accuracies. In a previous study on 48 children, it was shown that amplitude and latency measures of 3 auditory ERP components recorded at birth discriminated with 81.25% accuracy among 3 groups of children identified as normal, poor, or dyslexic readers based on reading and IQ scores obtained at 8 years of age. The significance of these results is that dyslexia can be detected early using neonatal ERPs. Consequently, early and more effective interventions can be provided to children before they enter school and thus improve their ability to learn. The ERPs were recorded from six scalp electrodes (FL, FR, T3,T4, PL, PR) to 4 stimuli consisting of 2 speech (/bi/ and /gi/) and 2 non-speech sounds (/nbi/ and /ngi/). Each child was represented by 24 (6 channels x 4 stimuli) averaged ERPs, that is, one averaged ERP for each stimulus-channel combination. Each ERP consisted of 200 data points, sampled at 5 ms intervals and collected sequentially over a 1 sec period beginning at stimulus onset. The first 13 samples and the last 47 samples were dropped and the remaining 140 point signal was down-sampled by a factor of 2 to facilitate discriminant analysis. Each ERP, therefore, consisted of 70 samples. The 6 ERP features selected for discriminant analysis classification consisted of (a) the first large negative peak (N1) latency to the speech syllable /gi/ recorded at FL, (b) the first large negative peak (N1) latency to the speech syllable /gi/ recorded at PL, (c) the first large negative peak (N1) latency to the speech syllable /gi/ recorded at T4, (d) the second large negative peak (N2) amplitude that differed between the 2 groups in response to the /gi/ speech syllable at FR, (e) the (N1) amplitude change recorded at T4, in response to /nbi/, and (f) the second large positive peak amplitude (P2) elicited in response to /bi/ at PR. Details regarding the subjects, stimuli, ERP procedures, and results of the study can be found in Reference 1.

Given the complexity of the problem, the classification results reported in the previous study are quite impressive. However, even higher classification accuracies with lower false positives are desired in practice. The specific goal in this paper, therefore, is to improve the classification performance using exactly the same averaged data used in the previous study. In this investigation, the 48 children were first divided into 2 categories consisting of 24 normal readers and 24 poor readers (dyslexics and poor readers combined) and next, into 3 categories (24 normal readers, 17 dyslexic readers, and 7 poor readers). Given the small number of ERPs with respect to the dimension of the ERP vector, it is clearly not possible to design parametric classifiers that depend on second-order statistics. This limits the choice of parametric classifiers to those that depend only on the first-order statistics. Given this limitation, the nearest-mean parametric classifier is selected because the mean representing each category can be estimated from the training sets using either the "leave-one-out method" or the "random equi-partition training and testing method. L. Gupta, J. Phegley, & D.L. Molfese, "Parametric classification of multichannel evoked potentials," IEEE Transactions on Biomedical Engineering, vol. 49, No. 8, 905-911, Aug. 2002 (vol. 49, No. 9, 1070, September 2002)." Two multi-stimuli multi-channel (MSMC) data fusion and 2 decision fusion strategies are introduced in conjunction with nearest-mean classifiers. In the both fusion strategies, the MSMC ERP vectors or the MSMC ERP elements are first ranked according to their individual Euclidean nearest mean classification accuracies. In the data fusion strategies, the vectors or elements are selected according to their rank and fused into a data fusion vector. The resulting data fusion vector is classified using a vector Euclidean nearest mean classifier. In the decision fusion strategies, the vectors or elements in the data fusion vector are classified independently using the Euclidean nearest mean classifier and the decisions are fused into a single decision fusion vector. The decision fusion vector is classified using a discrete Bayes classifier. The formulations of the strategies are quite general and are not limited by the number of ERP categories, channels, or stimuli. The strategies are tested on the same data used in Reference 1 and the results are presented in terms of the classification decision probabilities and the corresponding posterior probabilities. The performance is compared with the results reported in Reference and also with the nearest mean classification results of the single best stimulus and channel combination. In studies involving multiple channels and multiple stimuli, it is important to determine which channels, which stimuli, and which channel-stimuli give the best discriminatory information, therefore, a ranking strategy derived from the nearest-mean classification accuracies of the MSMC ERPs is first introduced. Ranking is also used to rank and select MSMC ERP vectors and MSMC ERP elements in the 4 fusion strategies.

CHANNEL-STIMULUS, STIMULUS , AND CHANNEL RANKING. In the generalized multi-category formulations to follow, $Z_{s/m}$ represents the $K$-dimensional averaged ERP elicited by external stimulus $s$, $s$=1,2,...,$S$, at channel $m$, $m$ = 1,2,..$M$, where S and M are the number of stimuli and channels, respectively. An averaged ERP elicited by external stimulus s at channel m given the category is $c$, $c$=1,2,...,$C$, is represented by $Z_{s/m/c}$ where C is the total number of categories. The number of different types of MSMC ERPs in each category is, therefore, $(S \times M)$.

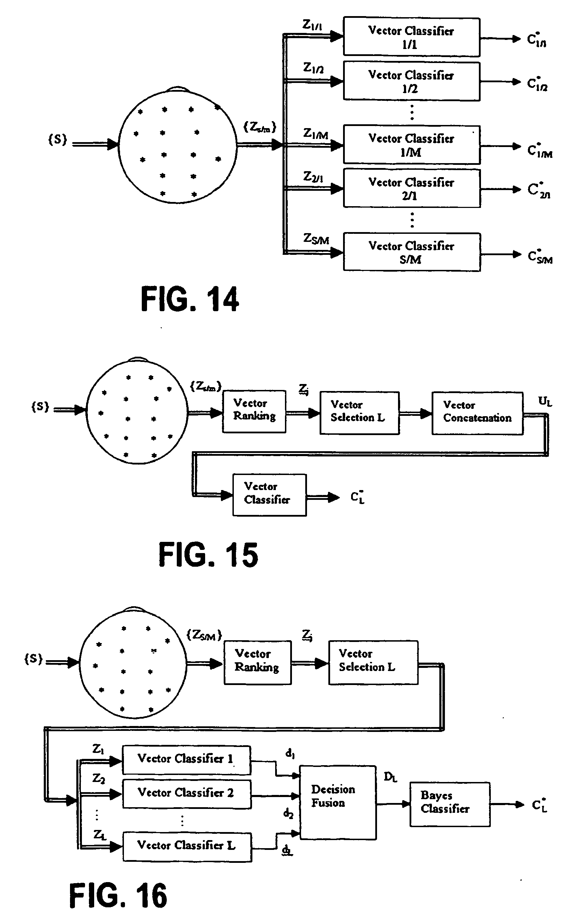

The nearest-mean classifier is developed for each ERP type as shown in Figure 14. The notation { } in the figure is used to represent the entire collection. Therefore, {s} is the entire set of S stimuli and {$Z_{s/m}$} is the entire set of $(S \times M)$ MSMC ERPs. The Euclidean norm nearest-mean vector discriminant function for category c, is given by

$$g_{c;s/m}(Z_{s/m}) = (Z_{s/m}^T)(\bar{Z}_{s/m/c}) - (1/2)(\bar{Z}_{s/m/c}^T)(\bar{Z}_{s/m/c}), \tag{33}$$

where $\overline{Z}_{s/m/c}$ is the mean of the $c$-category ERPs of channel $m$ in response to stimulus $s$. A test ERP $Z_{s/m}$ is assigned to the discriminant function that yields the highest value.

That is, the test MSMC ERP $Z_{s/m}$ is assigned to the category $\overset{\bullet}{c}_{s/m}$, $\overset{\bullet}{c}_{s/m} \in \{c\}$, given by

$$c^{\bullet}_{s/m} = \arg \max_{c} [g_{c;s/m}(Z_{s/m})]. \tag{34}$$

The classification accuracy of the classifier for each ERP type can be determined from the probability of classification error which is given by

$$P_{s/m}(\varepsilon) = \sum_{c=1}^{C} P_{s/m}(\varepsilon/c)P_c \tag{35}$$

where $P_{s/m}(\varepsilon/c)$ is the probability of misclassifying $Z_{s/m}$ when the true category of $Z_{s/m}$ is c and $P_c$ is the *a priori* probability of category *c*. The classification accuracy of the classifier for $Z_{s/m}$, expressed as a percentage, is given by

$$\alpha_{s/m} = [1 - P_{s/m}(\varepsilon)] \times 100\% . \tag{36}$$

The ($S \times M$) MSMC ERP classifiers can be ranked according to their respective classification accuracies. Furthermore, the stimuli and channels can be ranked individually using the ranks of the marginal rank-sums. The marginal rank-sums $\tilde{R}_s$ and $\tilde{Q}_m$ of the stimuli and of the channels are given, respectively, by

$$\tilde{R}_s = \sum_{m=1}^{M} R_{s/m} \tag{37}$$

$$\tilde{Q}_m = \sum_{s=1}^{S} R_{s/m} \tag{38}$$

where $R_{s/m}$ is the rank (1= highest classification accuracy, $S \times M$= lowest classification accuracy) of the classifier for ERP $Z_{s/m}$. The ranks $R_s$ and $R_m$ of the stimuli and channel are given by the ranks of the rank-sums, $\tilde{R}_s$ and $\tilde{Q}_m$, respectively.

MSMC VECTOR DATA FUSION. It is of interest to determine the classification accuracies that can be expected through different channel-stimuli combinations. Therefore, the goal in this section is to determine the classification performance as a function of the number of MSMC ERPs. The ($S \times M$) ERPs can clearly be combined in numerous ways. To simplify the selection of the combinations, the MSMC ERPs can be systematically combined using the rankings established in the previous section. That is, let $R_{s/m}$ be the rank of $Z_{s/m}$ and let

$$Z_j = Z_{s/m} \quad if \quad R_{s/m} = j . \tag{39}$$

As a result of the ranking, $Z_j$ is ordered such that $Z_1$ is the MSMC ERP vector yielding the highest classification accuracy and $Z_{(M \times S)}$ is the MSMC ERP yielding the lowest classification accuracy. Let $U_L$ be the data fusion vector formed according to

$$U_L = \overset{L}{\underset{j=1}{\nabla}} Z_j \tag{40}$$

where $\nabla$ represents the concatenation operation. That is, the fusion vector $U_L = (Z_1, Z_2, ..., Z_L)^T$ is formed by concatenating the ERP vectors with the first $L$ ranks to form a vector of dimension $(L \times K)$. Using $U_L$, the Euclidean-norm nearest mean vector discriminant function for category c is given by

$$g_{c;L}(U_L) = (U_L^T)(\overline{U}_{L/c}) - (1/2)(\overline{U}_{L/c}^T)(\overline{U}_{L/c}) \tag{41}$$

where $\overline{U}_{L/c}$ is the mean of the fusion vectors of category $c$. A test fusion vector $U_L$ is assigned to the category $c_L^{\bullet}$, $c_L^{\bullet} \in \{c\}$, given by

$$c_L^{\bullet} = \arg\max_c [g_{c;L}(U_L)]. \tag{42}$$

The MSMC vector data fusion strategy is summarized in Figure 15.

MSMC VECTOR DECISION FUSION. An alternative to data fusion is decision fusion in which the decisions of individual classifiers are combined to decide the category of a test pattern. As in the previous section, let

$$Z_j = Z_{s/m} \quad if \quad R_{s/m} = j \tag{43}$$

and let

$$c_j^{\bullet} = \arg\max_c [g_{c;j}(Z_j)] \tag{44}$$

where

$$g_{c;j}(Z_j) = (Z_L^T)(\overline{Z}_j) - (1/2)(\overline{Z}_j^T)(\overline{Z}_j) \tag{45}$$

is the nearest-mean vector discriminant function for category $c$ and $\overline{Z}_j$ is the mean of $Z_j$ belonging to category $c$. In order to fuse the decisions, let

$$d_j = c_j^{\bullet} \tag{46}$$

and let

$$D_L = \overset{L}{\underset{j=1}{\nabla}} d_j. \tag{47}$$

That is, $D_L = (d_1, d_2, ..., d_L)^T$ is the fusion vector formed by concatenating the decisions of the MSMC ERP vector classifiers with the first $L$ ranks. The decision fusion vector $D_L$ is a discrete random vector in which each element can take one of $C$ values. Let the PDF of $D_L$ under category c be $P(D_L/c)$ and $P_c$ be the a priori probability of category $c$, then, the Bayes decision function for category $c$ is

$$g_c(D_L) = P_c P(D_L / c) \tag{48}$$

which can also be written as

$$g_c(D_L) = \ln P_c + \ln P(D_L / c) \tag{49}$$

The final decision $c_L^\bullet$, $c_L^\bullet \in \{c\}$, resulting from decision fusion is given by

$$c_L^\bullet = \arg \max_c [g_c(D_L)] \tag{50}$$

The discriminant function for the *C*-category case can be derived explicitly by letting

$$p_{j,a/c} = P(d_j = a / c), \; j = 1,2,...,L \tag{51}$$

That is, $P_{j,a/c}$ is the probability that $d_j = a$, $a \in \{c\}$, when the true category is *c*. The probability density function (PDF) of $d_j$ under category c can be written as

$$P(d_j / c) = (p_{j,1/c})^{\delta(d_j-1)} (p_{j,2/c})^{\delta(d_j-2)} ... (p_{j,C/c})^{\delta(dj-C)} \tag{52}$$

Because the classifiers are developed independently for each MSMC ERP type, we assume that the decisions of the MSMC ERP classifiers are independent, therefore, the PDF of $D_L$ under the category *c* can be written as

$$P(D_L / c) = \prod_{j=1}^{L} (p_{j,1/c})^{\delta(d_j-1)} (p_{j,2/c})^{\delta(d_j-2)} ... (p_{j,C/c})^{\delta(dj-C)}, \tag{53}$$

where

$$\delta(x - a) = \begin{cases} 1 & if \quad x = a \\ 0 & if \quad x \neq a \end{cases} \tag{54}$$

By substituting the PDFs into Equation (49), it can be shown that the discriminant function for category c can be written as

$$g_c(D_L) = \sum_{j=1}^{L} \left[ \delta(d_j - 1) \ln(p_{j,1/c}) + \delta(d_j - 2) \ln(p_{j,2/c}) + ... + \delta(d_j - C) \ln(p_{j,C/c}) \right] + \ln P_c \tag{55}$$

MSMC ELEMENT DATA FUSION. In this data fusion strategy, the fusion vector is formed by combining selected elements of MSMC ERPs. The fusion vector *Z* is formed by first concatenating all MSMC ERPs into a vector of dimension $(S \times M \times K)$. That is

$$Z = \overset{S}{\underset{s=1}{\nabla}} \overset{M}{\underset{m=1}{\nabla}} Z_{s/m} \,. \tag{56}$$

Let $z(j)$, $j = 1,2,...,(S \times M \times K)$, be the jth element of Z and $d_j = c_j^{\bullet}$, $c_j^{\bullet} \in \{c\}$, where,

$$c_j^{\bullet} = \arg \max_{c} [g_c(z(j)] \tag{57}$$

and

$$g_c(z(j)) = z(j)\bar{z}_c(j) - (1/2)[\bar{z}_c(j)]^2 \tag{58}$$

is the nearest mean discriminant function for category $c$ and $\bar{z}_c(j)$ is the mean of $z(j)$ under category $c$. Let $\alpha_j$ be the classification accuracy of the nearest mean scalar classifier for element $z(j)$ and let $R_j$ be the rank of $z(j)$ according to the classification accuracies. Let

$$u(j) = z(k) \quad if \quad R_k = j \,. \tag{59}$$

As a result of the ranking, $u(1)$ is the element yielding the highest classification accuracy and $u(M \times S \times K)$ is the element yielding the lowest classification accuracy. Let $U_L$ be the data fusion vector formed according to

$$U_L = \overset{L}{\underset{j=1}{\nabla}} u(j) \,. \tag{60}$$

That is, the fusion vector $U_L = [u(1),u(2),...,u(L)]^T$ is formed by concatenating the elements with the first $L$ ranks to form a vector of dimension $L$. Note that the elements of $U_L$ are samples from various MSMC EPs. This corresponds to selecting different time-instants from different MSMC ERPs. Using $U_L$, the Euclidean-norm nearest mean discriminant function for category c is given by

$$g_{c;L}(U_L) = (U_L^T)(\bar{U}_{L/c}) - (1/2)(\bar{U}_{L/c}^T)(\bar{U}_{L/c}) \,, \tag{61}$$

where $\bar{U}_{L/c}$ is the mean of the fusion vector of category c. A test fusion vector $U_L$ is assigned to the category $c_L^{\bullet}$, $c_L^{\bullet} \in \{c\}$, given by

$$c_L^{\bullet} = \arg \max_{c} [g_{c;L}(U_L)] \,. \tag{62}$$

The MSMC element data fusion strategy is summarized in Figure 4.
MSMC ELEMENT DECISION FUSION. The fusion vector for this case is formed by combining the independent decisions of selected MSMC ERP element scalar classifiers. Let $d_j = c_j^{\bullet}$ where $c_j^{\bullet}$, $c_j^{\bullet} \in \{c\}$, is given by

$$c_j^\bullet = \arg\max_c \left[ g_c u(j) \right] \tag{63}$$

where $u(j)$ is defined in Equation (59) and

$$g_c(u(j)) = u(j)\bar{u}_c(j) - (1/2)[\bar{u}_c(j)]^2 \tag{64}$$

is the nearest mean discriminant function for category c and $\bar{u}_c(j)$ is the mean of $u(j)$ belonging to category $c$. The decision fusion vector for this case is given by

$$D_L = \overset{L}{\underset{j=1}{\nabla}} d_j. \tag{65}$$

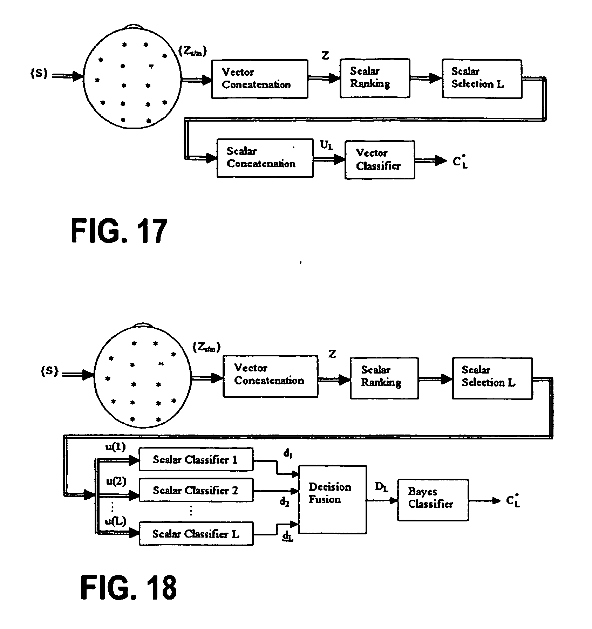

That is, $D_L = (d_1, d_2, ..., d_L)^T$ is the fusion vector formed by concatenating the decisions of the nearest mean element scalar classifiers with the first $L$ ranks. Given $D_L$, the sequence of steps to derive the decision rule is identical to those described in Section 4. The discriminant function is given by Equation (55). The probability $p_{j,a/c}$ in the discriminant function for this case is the probability that the scalar classifier for $u(j)$ decides category $a$ when the true category is $c$. The MSMC element decision fusion strategy is summarized in Figure 18.

PERFORMANCE MEASURES. The performance of the 4 fusion classification strategies can be specified in terms of the classification decision probability or the classification accuracy which are computed from the classification rates. The classification rate for category $c$ is given by

$$\alpha_{c,L} = [1 - P_{c,L}(\varepsilon)] \times 100\%. \tag{66}$$

where

$P_{c,L}(\varepsilon)$ = is the probability of misclassifying the data fusion vector $U_L$ of Section 3 when the true category of $U_L$ is $c$.
$P_{c,L}(\varepsilon)$ = is the probability of misclassifying the decision fusion vector $D_L$, of Section 4 when the true category of $D_L$ is $c$.
$P_{c,L}(\varepsilon)$ = is the probability of misclassifying the data fusion vector $U_L$ of Section 5 when the true category of $U_L$ is $c$.
$P_{c,L}(\varepsilon)$ = is the probability of misclassifying the decision fusion vector $D_L$ of Section 6 when the true category of $D_L$ is $c$.

The corresponding classification decision probability can be determined as

$$\alpha_L = [1 - P_L(\varepsilon)], \tag{67}$$

where

$$P_L(\varepsilon) = \overset{C}{\underset{c=1}{\Sigma}} P_{c,L}(\varepsilon) P_c. \tag{68}$$

The classification accuracy, expressed as a percentage, is given by $(\alpha_L \times 100)\%$.

EXPERIMENTS AND RESULTS. The data used to design and evaluate the classifiers were exactly the same as that used in the study described in Reference 1 except that the signals were not down-sampled. Each ERP, therefore, consisted of 140 samples (140 elements in the ERP vector). Each child was represented by (6X4)=24 MSMC averaged ERPs. The means and the probabilities of each discriminant function were estimated from the respective training sets of each category.

Control/Dyslexia (2-Category Classification). The 48 children were grouped into 2 categories: control ($C=1$)

and dyslexia ($C$=2). The dyslexic and poor readers were grouped into the dyslexic group. The number of children in each category was 24. Because dyslexia occurs in approximately 10% of the population, the prior probabilities $P_1$ (control) and $P_2$ (dyslexia) were selected, to be 0.9 and 0.1, respectively. The MSMC ERPs of each category were partitioned randomly into 2 mutually exclusive equi-sized sets to form the training set and the test set. The channels $m$=1, 2, 3, 4, 5, and 6 are FL, FR, T3, T4, PL, and PR and the stimuli $s$=1, 2, 3, and 4 are /bi/, /gi/, /nbi/, and /ngi/, respectively. Table 4 shows the classification accuracies of each MSMC ERP type and the rankings of the channel-stimuli, stimuli, and channels obtained from the rankings of the classification accuracies. The entries in Table 4(a) show the classification accuracies $a_{s/m}$ of each ERP type, where, as described in Section 2, an ERP type is the ERP elicited at a given channel in response to a given stimulus. The number of ERP types in this study is 24 (6 channels x 4 stimuli). The first entry in Table 4(a) is, therefore, the classification accuracy of the ERPs elicited at channel 1 (FL) in response to stimulus 1 (/bi/). The first entry in Table 4(b) shows the rank $R_{s/m} = R_{1/1}$ of the ERP at channel 1 (FL) in response to the stimulus s=1(/bi/). $R_{4/5}$ has rank 1 because the ERPs of channel 5 in response to stimulus 4, give the highest classification accuracy and $R_{1/6}$ has rank 24 because the ERPs of channel 6 in response to stimulus 1 yield the lowest classification accuracies. The last row gives the individual rank of each channel computed from the marginal rank sum of Equation (38) and the last column gives the rank of each stimulus computed from the marginal rank sum of Equation (37). For example, $R_m = R_5$ =1 because the sum of the $R_{s/m}$ values in column 5 has the smallest value. Similarly, $R_s = R_3$ =1 because the sum of the $R_{s/m}$ values in row 3 has the smallest value.

Tables 5-8 show, for the four fusion strategies, the decision probabilities and the posterior probabilities for $L$ giving the maximum classification accuracy. The best value of $L$ was selected by evaluating the performances for all possible values of $L$ (1 to 24 for vector data and vector decision fusion; 1 to 3360 (6x4x140 elements) for element data and element decision fusion). All classification results were estimated by averaging the decision probabilities over 100x 12=1,200 tests for each category. The classifier decision is represented by $Y$, where, $Y$=1 when the classifier decision is the control category and $Y$=2 if the classifiers decision is the dyslexic category. In order to interpret the results in Tables 5-8, consider Tables 5(a) and 5(b) which show the classifier's decision probabilities and the corresponding posterior probabilities, respectively. The first entry in Table 5(a) is the conditional probability P($Y$=1/$C$=1), that is, the probability that the classifier correctly classifies control subjects. The first entry in Table 5(b) is the conditional probability P($C$=1/$Y$=1) which gives the probability that the test subject is from the control group given that the classifier has decided that the test subject is from the control group (true-negative probability). The posterior probabilities are computed using Bayes rule. Table 9 shows the interpretation of the posterior probabilities in terms of the probabilities of true negatives, false negatives, false positives, and true positives.

For comparison, the results of discriminant analysis from the previous study, for the 2-category case, using the prior probabilities of 0.9 and 0.1 are shown in Table 10. In studies such as the one reported in this paper, the posterior probabilities are quite important because the performance in terms of the classification accuracy by itself can be quite misleading. Consider, for example, the decision probability of 0.8125 which was reported in Reference 1. Although the classifier's decision probability is quite high, the probabilities of a false positive and a true positive are quite high (0.6735) and quite low (0.3264), respectively, as seen in in Table 10(b).

Control/Dyslexia/Poor Readers (3-Category Classification). The 48 children were grouped into 3 categories: control ($C$=1), dyslexia ($C$=2), and poor readers ($C$=3). The corresponding classifier decisions are represented by $Y$=1, $Y$=2, and $Y$=3. The number of children in the categories were 24, 17, and 7, respectively. The prior probabilities $P_1$ (control), $P_2$ (dyslexia), and $P_3$ (poor readers) were selected, to be 0.9, (17/24 $\times$ 0.1)=0.07, and (7/24 $\times$ 0.1)=0.03, respectively. Because of the small number of MSMC ERPs in the poor reader category, the MSMC ERPs of each category were partitioned using the "leave-one-out" method to form the training set and the test set. Table 11(a) shows the estimated classification accuracies $\alpha_{s/m}$ of each MSMC ERP type as described in Section 2. The channel-stimulus, stimulus, and channel rankings are presented in Table 11 (b). All results were estimated by averaging the classification accuracies using the "leave-one-out method." Tables 12-15 show, for the four fusion strategies, the results for $L$ giving the maximum decision probabilities and the corresponding posterior probabilities. For comparison, the results of discriminant analysis from the previous study, for the 3-category case, using the prior probabilities of 0.9, 0.07, and 0.03 are shown in Table 16.

CONCLUSIONS. The goal in this paper was to obtain very high accuracies for predicting dyslexia in children from their MSMC ERPs recorded at birth. A ranking strategy was developed to rank the channel-stimuli combinations, channels, stimuli, and ERP elements in terms of their effectiveness in classifying dyslexia. The prediction problem was formulated as a classification problem and the rankings were used to develop 2 MSMC data-fusion and 2 decision-fusion classification strategies. The data fusion methods systematically combined the rank-ordered MSMC ERP vectors or the rank-ordered MSMC ERP elements. For each case, the resulting data fusion vector was classified using a single vector nearest-mean classifier. In the decision fusion strategy, the independent classification decisions of rank-ordered MSMC ERP vectors or the independent classification decisions of rank-ordered MSMC ERP elements were combined into a decision fusion vector. The resulting decision fusion vector for each case was classified using a Bayes discrete vector classifier.

The fusion classification strategies were tested using exactly the same data in the study reported in D.L. Molfese, "Predicting dyslexia at 8 years of age using neonatal brain responses," Brain and Language, 72, 238-245, 2000 ("Reference 1"). The results presented facilitate performance comparisons not only between the 4 fusion classification strategies but also between ERP vector fusion versus ERP element fusion as well as data fusion versus decision fusion. The best results were obtained using MSMC element decision fusion. It was shown that a classification accuracy of 99.88% with zero false positives are possible for the control/dyslexia prediction problem using $L$=2822 ERP elements out of the total of 3360 elements. For the control/dyslexia/poor reader prediction problem, a classification accuracy of 100% was possible using "leave-one-out" evaluations using $L$=497 ERP elements. Thus it can be concluded that future reading difficulties can be predicted with almost 100% accuracy. The significance of the decision fusion results can be appreciated by noting that, individually, the best ($L$=1) MSMC ERP vector and MSMC ERP element give classification accuracies of only 57.06% and 74.43% for the 2-category case, respectively, and 50.95% and 69.95% for the 3-category case, respectively. Additionally, the significant reduction in the false positive probabilities must be noted by comparing the results with those reported in Table 10(b). In terms of complexity, the data fusion strategies require only a single vector classifier whereas the decision fusion classifiers require multiple element (scalar) classifiers and an additional Bayes discrete vector classifier. The scalar classifiers are, however, computationally relatively simple.

The results presented in this paper further support the findings reported in reference 1. That is, auditory ERPs recorded within 36 hours of birth can successfully predict reading performance in children 8 years later. Therefore, potential problems in language or cognitive development can be identified at birth and, consequently, planned interventions can be introduced earlier to the child and be more successful in the remediation of the child's later emerging language problems. This will have a tremendously positive impact by avoiding the psychological damage resulting from being labeled "slow", enabling the child to take full advantage of his/her schooling and thus make it possible to reach his/her full intellectual potential.

In summary, it is shown that through the MSMC element decision fusion classification strategy, dyslexia and poor reading can be predicted with almost 100% accuracy from the ERPs of infants. Furthermore, because the formulations of the MSMC fusion classification strategies are quite general, they can also be applied to other multi-category prediction/classification problems involving ERPs and, in general, to multi-category multi-sensor signal classification problems.

Table 4. Two-category ranking

| (a) classification accuracies of each MSMC ERP Type | | | | | | | | | | | | | | |
| (b) The channel stimulus, stimulus and channel rankings | | | | | | | | | | | | | | |
| (a) s | M | | | | | | (b) s | m | | | | | | $R_s$ |
| | 1 | 2 | 3 | 4 | 5 | 6 | | 1 | 2 | 3 | 4 | 5 | 6 | |
| 1 | 48.14 | 48.32 | 45.43 | 46.18 | 53.99 | 39.39 | 1 | 17 | 15 | 19 | 18 | 3 | 24 | 4 |
| 2 | 48.77 | 52.98 | 51.46 | 49.93 | 40.03 | 52.38 | 2 | 14 | 6 | 10 | 13 | 23 | 7 | 2 |
| 3 | 45.34 | 50.58 | 53.52 | 55.4 | 53.67 | 51.47 | 3 | 20 | 12 | 5 | 2 | 4 | 9 | 1 |
| 4 | 52 | 51.2 | 41.85 | 48.16 | 57.06 | 43.68 | 4 | 8 | 11 | 22 | 16 | 1 | 21 | 3 |
| | | | | | | | $R_m$ | 5 | 2 | 4 | 3 | 1 | 6 | |

Table 5. MSMC vector data fusion results

| (a) Classification decision probabilities for the maximum classification accuracy ($\alpha_2$=58.29%) | | | | (b) Posterior probabilities for the maximum classification accuracy ($\alpha_2$=58.29%) | | |
| (a) True Class | Classifier Decision | | | (b) Classifier Decision | True Class | |
| | $Y$=1 | $Y$=2 | | | $C$=1 | $C$=2 |
| $C$=1 | 0.58583 | 0.41417 | | $Y$=1 | 0.92244 | 0.077562 |
| $C$=2 | 0.44333 | 0.55667 | | $Y$=2 | 0.87006 | 0.12994 |

Table 6. MSMC vector decision fusion results

| (a) Classification decision probabilities for the maximum classification accuracy ($\alpha_{24}$=90.29%) | | | | (b) Posterior probabilities for the maximum classification accuracy ($\alpha_{24}$=90.29%) | | | |
|---|---|---|---|---|---|---|---|
| (a) True | Classifier Decision | | | (b) Classifier | True Class | | |
| Class | $Y$=1 | $Y$=2 | | Decision | $C$=1 | $C$=2 | |
| $C$=1 | 0.99917 | 0.00083 | | $Y$=1 | 0.90324 | 0.09676 | |
| $C$=2 | 0.96333 | 0.036667 | | $Y$=2 | 0.16925 | 0.83075 | |

Table 7. MSMC element data fusion results

| (a) Classification decision probabilities for the maximum classification accuracy ($\alpha_{336}$=87.97%) | | | | (b) Posterior probabilities for the maximum classification accuracy ($\alpha_{336}$=87.97%) | | | |
|---|---|---|---|---|---|---|---|
| True | Classifier Decision | | | Classifier | True Class | | |
| Class | $Y$=1 | $Y$=2 | | Decision | $C$=1 | $C$=2 | |
| $C$=1 | 0.88667 | 0.15417 | | $Y$=1 | 0.97754 | 0.022458 | |
| $C$=2 | 0.18333 | 0.88583 | | $Y$=2 | 0.55535 | 0.44465 | |

Table 8. MSMC element decision fusion results

| (a) Classification decision probabilities for the maximum classification accuracy ($\alpha_{2822}$=99.88%) | | | | (b) Posterior probabilities for the maximum classification accuracy ($\alpha_{2822}$=99.88%) | | | |
|---|---|---|---|---|---|---|---|
| True | Classifier Decision | | | Classifier | True Class | | |
| Class | $Y$=1 | $Y$=2 | | Decision | $C$=1 | $C$=2 | |
| $C$=1 | 1 | 0 | | $Y$=1 | 0.99871 | 0.001295 | |
| $C$=2 | 0.01167 | 0.98833 | | $Y$=2 | 0 | 1 | |

Table 9.

| Positions of true negatives, false negatives, false positives, and true positives in Tables 5(b) - 8(b) | | |
|---|---|---|
| Classifier | True Class | |
| Decision | $C$=1 | $C$=2 |
| $Y$=1 | True negative | False negative |
| $C$=2 | False positive | True positive |

Table 10. Two-category discriminant analysis results from previous study

| (a) Classification decision probabilities | | | | (b) Posterior probabilities | | | |
|---|---|---|---|---|---|---|---|
| (a) True | Classifier Decision | | | (b) Classifier | True Class | | |
| Class | $Y$=1 | $Y$=2 | | Decision | $C$=1 | $C$=2 | |
| $C$=1 | 0.79 | 0.21 | | $Y$=1 | 0.9884 | 0.01153 | |
| $C$=2 | 0.083 | 0.916 | | $Y$=2 | 0.6735 | 0.3264 | |

Table 11. Three-category rankings

| (a) Classification accuracies of each MSMC ERP type. | | | | | | | | | | | | | | |
|---|---|---|---|---|---|---|---|---|---|---|---|---|---|---|
| (b) The channel-stimulus, stimulus, and channel rankings. | | | | | | | | | | | | | | |
| (a) | M | | | | | | (b) | M | | | | | | |
| s | 1 | 2 | 3 | 4 | 5 | 6 | s | 1 | 2 | 3 | 4 | 5 | 6 | R |
| 1 | 28.12 | 31.84 | 28.42 | 34.60 | 44.50 | 12.59 | 1 | 16 | 14 | 15 | 12 | 6 | 24 | 3 |
| 2 | 23.81 | 50.95 | 45.58 | 38.79 | 22.70 | 43.78 | 2 | 20 | 1 | 5 | 11 | 22 | 8 | 2 |
| 3 | 25.10 | 41.96 | 40.42 | 45.74 | 44.43 | 46.37 | 3 | 19 | 9 | 10 | 4 | 7 | 3 | 1 |
| 4 | 33.53 | 27.42 | 20.43 | 27.39 | 48.54 | 22.85 | 4 | 13 | 17 | 23 | 18 | 2 | 21 | 4 |
| | | | | | | | $R_m$ | 6 | 2 | 4 | 3 | 1 | 6 | |

Table 12. MSMC vector data fusion results (3-category)

| (g) Classification decision probabilities for the maximum classification accuracy ($\alpha_{11}$=60.78%) | | | | | | | |
|---|---|---|---|---|---|---|---|
| (h) Posterior probabilities for the maximum classification accuracy ($\alpha_{11}$=60.78%) | | | | | | | |
| True | Classifier Decision | | | | Classifier | True Class | | |
| Class | $Y$=1 | $Y$=2 | $Y$=3 | | Decision | $C$=1 | $C$=2 | $C$=3 |
| $C$=1 | 0.64671 | 0.32913 | 0.02416 | | $Y$=1 | 0.92806 | 0.063392 | 0.0085489 |
| $C$=2 | 0.56127 | 0.30462 | 0.1341 | | $Y$=2 | 0.87786 | 0.063945 | 0.058199 |
| $C$=3 | 0.18382 | 0.67332 | 0.14286 | | $Y$=3 | 0.61407 | 0.26826 | 0.11767 |

Table 13. MSMC vector decision fusion results (3-category)

| (a) Classification decision probabilities for the maximum classification accuracy ($\alpha_{24}$=95.65%) | | | | | | | |
|---|---|---|---|---|---|---|---|
| (b) Posterior probabilities for the maximum classification accuracy ($\alpha_{24}$=95.65%) | | | | | | | |
| True | Classifier Decision | | | | Classifier | True Class | | |
| Class | $Y$=1 | $Y$=2 | $Y$=3 | | Decision | $C$=1 | $C$=2 | $C$=3 |
| $C$=1 | 0.99125 | 0.007703 | 0.001050 | | $Y$=1 | 0.96585 | 0.02930 | 0.004854 |
| $C$=2 | 0.382 | 0.59139 | 0.026611 | | $Y$=2 | 0.13594 | 0.8214 | 0.042654 |
| $C$=3 | 0.15371 | 0.07458 | 0.77171 | | $Y$=3 | 0.03731 | 0.07439 | 0.8883 |

Table 14. MSMC element data fusion results (3-category)

| (a) Classification decision probabilities for the maximum classification accuracy ($\alpha_{260}$=77.14%) | | | | | | | |
|---|---|---|---|---|---|---|---|
| (b) Posterior probabilities for the maximum classification accuracy ($\alpha_{260}$=77.14%) | | | | | | | |
| True | Classifier Decision | | | | Classifier | True Class | | |
| Class | $Y$=1 | $Y$=2 | $Y$=3 | | Decision | $C$=1 | $C$=2 | $C$=3 |
| $C$=1 | 0.83333 | 0.15406 | 0.012601 | | $Y$=1 | 0.9637 | 0.036298 | 0 |
| $C$=2 | 0.39881 | 0.30182 | 0.29937 | | $Y$=2 | 0.73285 | 0.113 | 0.15416 |
| $C$=3 | 0 | 1 | 0 | | $Y$=3 | 0.34853 | 0.65147 | 0 |

Table 15. MSMC element decision fusion results (3-category)

| (a) Classification decision probabilities for the maximum classification accuracy ($\alpha_{497}$=100%) | | | | (b) Posterior probabilities for the maximum classification accuracy ($\alpha_{497}$=100%) | | | |
|---|---|---|---|---|---|---|---|
| True Class | Classifier Decision | | | Classifier Decision | True Class | | |
| | $Y$ =1 | $Y$ =2 | $Y$ =3 | | $C$ =1 | $C$ =2 | $C$ =3 |
| $C$ =1 | 1 | 0 | 0 | $Y$ =1 | 1 | 0 | 0 |
| $C$ =2 | 0 | 1 | 0 | $Y$ =2 | 0 | 1 | 0 |
| $C$ =3 | 0 | 0 | 1 | $Y$ =3 | 0 | 0 | 1 |

Table 16. Three-cateaorv discriminant analysis results from previous study

| (a) Classification decision probabilities | | | | (b) Posterior probabilities | | | |
|---|---|---|---|---|---|---|---|
| True Class | Classifier Decision | | | Classifier Decision | True Class | | |
| | $Y$ =1 | $Y$ =2 | $Y$ =3 | | $C$ =1 | $C$ =2 | $C$ =3 |
| $C$ =1 | 0.79 | 0.08 | 0.13 | $Y$ =1 | 0.9882 | 0.0118 | 0 |
| $C$ =2 | 0.12 | 0.76 | 0.12 | $Y$ =2 | 0.5752 | 0.4278 | 0 |
| $C$ =3 | 0 | 0 | 1 | $Y$ =3 | 0.7665 | 0.0550 | 0.1886 |

MULTI-CHANNEL FUSION MODELS FOR THE PARAMETRIC CLASSIFICATION OF DIFFERENTIAL BRAIN ACTIVITY.

This section introduces parametric multi-channel fusion models to exploit the different but complementary brain activity information recorded from multiple channels in order to accurately classify differential brain activity into their respective categories. A parametric weighted decision fusion model and 2 parametric weighted data fusion models are introduced for the classification of averaged multi-channel evoked potentials (EPs). The decision fusion model combines the independent decisions of each channel classifier into a decision fusion vector and a parametric classifier is designed to determine the EP class from the discrete decision fusion vector. The data fusion models include the weighted EP-sum model in which the fusion vector is a linear combination of the multi-channel EPs and the EP-concatenation model in which the fusion vector is a vector-concatenation of the multi-channel EPs. The discrete Karhunen-Loeve transform (DKLT) is used to select features for each channel classifier and from each data fusion vector. The difficulty in estimating the probability density function (PDF) parameters from a small number of averaged EPs is identified and the class conditional PDFs of the feature vectors of averaged EPs are, therefore, derived in terms of the PDFs of the single-trial EPs.

Multivariate parametric classifiers are developed for each fusion strategy and the performances of the different strategies are compared by classifying 14-channel EPs collected from 5 subjects involved in making explicit match/ mismatch comparisons between sequentially presented stimuli. It is shown that the performance improves by incorporating weights in the fusion rules and that the best performance is obtained using multi-channel EP concatenation. It is also noted that the fusion strategies introduced are also applicable to other problems involving the classification of multi-category multivariate signals generated from multiple sources.

INTRODUCTION. Numerous human brain function studies employ brain waveforms to determine relationships between cognitive processes and changes in the brain's electrical activity. Brain waveforms are also used extensively in clinical investigations to study normal and abnormal brain functions. The goal of this section is to introduce and evaluate multi-channel fusion strategies that will automatically classify differential brain activity into their respective categories. The categories may include brain activity correlated with different stimuli or with different stimuli presentation methods that are typical in human cognition studies. The categories in clinical investigations may be the hypothesized dysfunctional states or different stages of medical conditions. The section focuses on the classification of multi-channel evoked potentials (EPs), however, the strategies developed are also applicable to the classification of multi-channel electroencephalograms (EEGs) and in general, to the classification of multi-sensor signals. Because of the poor signal-to-noise-ratio (SNR), analysis and classification is typically conducted on EPs averaged over a large number of trials. However, repeating an experiment a large number of times in order to collect sufficient single-trial data to form averages is not practical and may even not be possible in some studies and investigations (Reference 1). Consequently, improving the classification accuracies for small-average EPs will be a major breakthrough for the more flexible application of EPs in differential brain waveform studies and clinical investigations.

Analyses of multi-channel EPs show that different channels reflect different aspects of the brain activity from the presentation of an external stimulus. For example, consider the ensemble averaged EPs of 6 different channels shown in Figure 6. Each figure shows EPs belonging to 2 different categories (brain activity classes). The EPs within the same category (responses to the same external input) are clearly different across the 6 channels. Methods that focus on classifying the EPs of each channel independently do not fully exploit this different but complementary information buried in the multi-channel recordings of brain activity. This section, therefore, focuses on ways by which the information from multiple-channels can be combined in order to improve the classification accuracies of averaged EPs. Classifying multi-channel EPs can be formulated as that of classifying multi-sensor data. Several fusion methodologies have been proposed to combine data, features, or decisions of multiple sensors in order improve the performance over that of single sensor systems. Our previous efforts to improve the classification accuracies led to the development of a decision fusion strategy in which multi-channel EPs were classified by fusing the classification results of all channels (Reference 1). A 2-category classifier was developed independently for each channel and the classification results were fused using a majority decision rule. It was shown that the classification accuracy of the majority decision fusion rule classifier was consistently superior when compared with the rule that selected the classification accuracy of a single best channel.

This section focuses on comparing the performances of several parametric models uniquely formulated for both multi-channel decision fusion and multi-channel data fusion. Although most studies and investigations typically involve 2 categories, multi-category formulations are presented to generalize the fusion models and classification strategies. A parametric weighted decision fusion model and two data fusion models are introduced. In the weighted decision fusion model, a classifier is designed independently for each channel and the decisions of the channel classifiers are fused into a single discrete random fusion vector. The channel decisions are weighted using a priori classification accuracy information and a separate parametric classifier is designed to determine the EP class from the weighted decision fusion vector. The two data fusion models introduced are referred to as multi-channel EP-sum and multi-channel EP-concatenation. In the multi-channel EP-sum model, the weighted EPs are summed across all channels to form a single fusion vector which has the same dimension as the individual channel EPs. Without weighting, this is similar to the "grand averaging" operation that is often used in EP analysis. The EP elements are weighted according to their multi-class separations. The EPs of all channels are stacked into a fusion vector in the multi-channel EP-concatenation model. The dimension of the fusion vector increases by a factor equal to the number of channels. A 2-step dimensionality reduction algorithm which takes into account the inter-class separation and the correlation of the components in the fusion vector is introduced to facilitate classifier development. In order to compare the performances of the different fusion strategies, multivariate parametric classifiers are developed for each fusion strategy and the performances are compared by classifying 14-channel EPs collected from 5 subjects involved in picture-word matching tasks.

SINGLE-TRIAL EP AND AVERAGED-EP MODELS. In the fusion formulations to follow, $Z_m$ represents the $K$-dimensional response vector to an external input at channel $m = 1,2,...M$ , where $M$ is the number of channels. Single-trial responses are referred to as 1-EPs and responses averaged over r single-trials are referred to as r-EPs. Single-trial and averaged responses elicited by external stimulus $c$, $c = 1.2...,C$ at channel $m$ are represented by $Z_{m/c}$ and $Z_{m/c}^r$, respectively, where C is the total number of external stimuli. It is assumed that each stimulus elicits a different class of brain activity, therefore, the number of EP classes is also C.

Single-trial EP Model. The single-trial response vector at channel $m$ in response to an external stimulus c is often modeled as

$$Z_{m/c} = S_{m/c} + N_m, \tag{69}$$

where $S_{m/c}$ is the EP elicited by stimulus c and $N_m$ is the ever-present EEG which is regarded as background noise.

r-Average EP Model. In order to show the improvement in signal-to-noise ratio through single-trial averaging, it is assumed in the single-trial model that $S_{m/c}$ and $N_m$ are independent, $S_{m/c}$ is deterministic, and $N_m$ is a zero-mean correlated process. The model assumed for single-trial EPs averaged over r-trials is Reference 1.

$$Z_{m/c}^r = S_{m/c} + N_m^r, \tag{70}$$

where $N_m^r$ is the noise averaged over r trials. We have shown in Reference 1 that from the multivariate central-limit-theorem, $N_m^r$ can be assumed to be a zero-mean Gaussian random vector. That is, $p(N_m^r) \sim G(0, \Psi_m^r)$, where $p(N_m^r) = p_{N_m^r}(.)$ is the probability density function (PDF) of the random vector $N_m^r$, $\Psi_m^r$ is the covariance of $N_m^r$, and $G(\mu, \Psi)$ represents the multivariate Gaussian PDF with mean $\mu$ and covariance $\Psi$. Furthermore, we have shown that the parameters of the smaller r-EP design set can be determined from the parameters of the larger 1-EP design set. Specifically, it was shown that: (1) If $\mu_{m/c}$ and $\mu_{m/c}^r$ are the ($K \times 1$) mean vectors of $Z_{m/c}$ and $Z_{m/c}^r$, respectively, then,

$$\mu_{m/c}^r = \mu_{m/c} = S_{m/c} . \tag{71}$$

(2) If $\Psi_m = E[(N_m)(N_m)^T]$ and $\Psi_m^r = E[(N_m^r)(N_m^r)^T]$ are the ($K \times K$) covariance matrices of $Z_{m/c}$ and $Z_{m/c}^r$, respectively, then,

$$\Psi_m^r = (1/r)\Psi_m . \tag{72}$$

Therefore,

$$p(Z_{m/c}^r) = p_{N_m^r}(Z_{m/c}^r - S_{m/c}) \sim G(S_{m/c}, [(1/r)\Psi_m]) , \tag{73}$$

where $p(Z_{m/c}^r)$ is the PDF of the r-EPs of channel m belonging to class c. Note that because the noise in each channel is assumed statistically equivalent under the different categories, the covariance matrices of the EPs of each channel are independent of c.

(3) If $\lambda_m^r$ and $\lambda_m$ are the ($K \times 1$) eigenvalue vectors and $E_m^r$ and $E_m$ are the ($K \times K$) eigenvector matrices of the covariance matrices of the r-EP and 1-EP ensembles, respectively, then,

$$\lambda_m^r = (1/r)\lambda_m , \tag{74}$$

$$E_m^r = E_m . \tag{75}$$

It must be emphasized that these results are quite important for the design of practical parametric r-EP classifiers because a lower bound is placed on the larger number of 1-EPs in the design set and not on the smaller number of r-EPs in the design set for covariance estimation. Consequently, an impractically large number of single-trial EPs does not have to be collected to generate enough r-EPs for parameter estimation. These results will be used to determine the PDF parameters of the r-EP fusion vectors of each fusion model, in terms of the PDF parameters of the 1-EPs of the multiple channels, in order to determine the discriminant functions for the brain activity categories. The specific goal in the next 3 sections is to develop fusion-based classification strategies to determine, from a set of multi-channel r-EPs, the class of the stimulus that elicited the r-EPs.

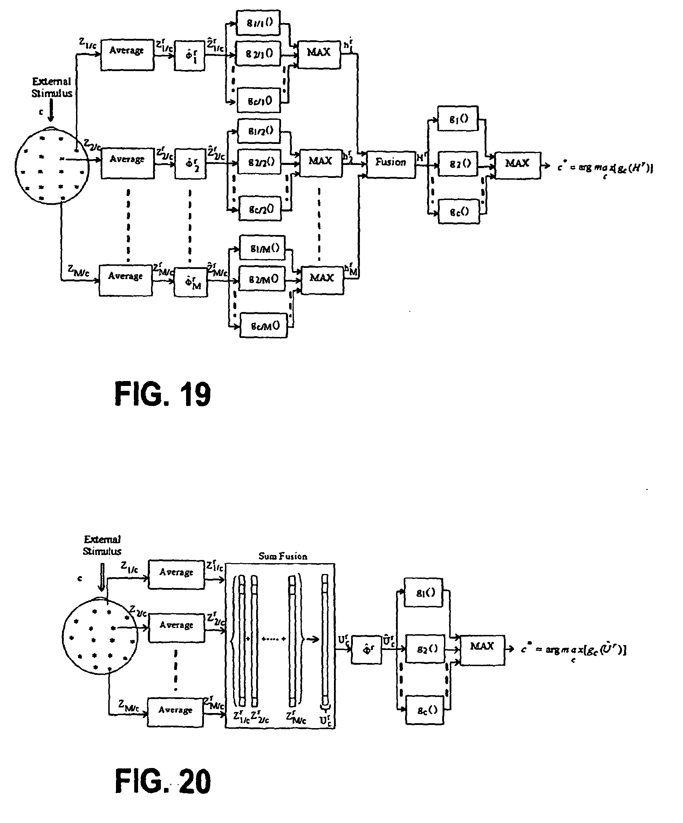

MULTI-CHANNEL EP DECISION FUSION MODEL. In the multi-channel EP decision fusion model, the outputs of the M channel classifiers are fused so as to implement a single decision rule as shown in Figure 19. Each channel classifier is designed to independently classify the brain activity elicited by the external stimulus at its corresponding location on the scalp. A general weighted fusion rule is now introduced for $C \geq 2$ categories and it is shown that the simple majority rule is a special case of the weighted fusion rule. In order be explicit, the formulations are described in terms of the averaging parameter r, the channel parameter m, and the class parameter c. The C-class classifier for the r-EPs of channel m is developed as follows:

DKLT Feature Selection. The features for classification are selected from a set of linear combinations of the samples of $Z_{m/c}^r$. The linear combinations are given by

$$\tilde{Z}^r_{m/c} = \Phi^r_m Z^r_{m/c} , \tag{76}$$

where $\Phi^r_m$ is the ($K \times K$) DKLT of the r-EPs of channel m. That is, $\Phi^r_m$ is the eigenvector matrix of the r-EP covariance matrix of channel m with the eigenvectors arranged in an order corresponding to decreasing eigenvalues. Let $\tilde{\Phi}^r_m$ be the eigenvector matrix formed by arranging the rows of $\Phi^r_m$ in a descending order of separation so that the first row yields the linear combination with the highest C-class separation and the last row yields the lowest C-class separation where the C-class separation of each element in $\tilde{Z}^r_m$ is found by summing the pairwise interclass separation. That is, if $\tilde{z}^r_{m/a;i}(k)$ and $\tilde{z}^r_{m/b;i}(k)$ are the kth elements of the ith vectors of $\tilde{Z}^r_{m/a}$ and $\tilde{Z}^r_{m/b}$, respectively, then the class-a vs class-b pairwise scalar separation of element k of $\tilde{Z}^r_m$ is given by

$$\rho^r_{m;ab}(k) = \frac{[\bar{\tilde{z}}^r_{m/a}(k) - \bar{\tilde{z}}^r_{m/b}(k)]^2}{\sum\limits_{i=1}^{I}[\tilde{z}^r_{m/a;i}(k) - \bar{\tilde{z}}^r_{m/a}(k)]^2 + \sum\limits_{i=1}^{I}[\tilde{z}^r_{m/b;i}(k) - \bar{\tilde{z}}^r_{m/b}(k)]^2} , \qquad k = 1,2,...,K , \tag{77}$$

where, $\bar{\tilde{z}}^r_{m/a}(k)$ and $\bar{\tilde{z}}^r_{m/b}(k)$ are the means of the kth elements of $\tilde{Z}^r_{m/a}$ and $\tilde{Z}^r_{m/b}$, respectively, and I is the number of vectors in each class (assumed equal for convenience). Then, the C-class separation of the kth element of $\tilde{Z}^r_m$ is given by

$$\rho^r_m(k) = \sum_{a=1}^{C-1} \sum_{b=a+1}^{C} \rho^r_{m;ab}(k) \tag{78}$$

Typically, only a small number of linear combinations (eigenvectors) yield high C-class separations. We, therefore, select the eigenvectors to form the feature vectors based on their corresponding C-class separations. That is, we arrange the eigenvectors in a descending order of C-class separation and choose the first $\hat{K}_m$ eigenvectors that satisfy

$$\frac{\rho^r_m(k)}{MAX[\rho^r_m(k)]} \ge \delta; \quad k = 1,2,...,K , \tag{79}$$

where δ is a specified threshold, to form the truncated DKLT feature selection matrix $\hat{\Phi}^r_m$ of dimension ($\hat{K}_m \times K$). For the dimension of the feature vectors for all the channels to be the same, the dimension $\hat{K}$ is selected as

$$\hat{K} = (1/M) \sum_{m=1}^{M} \hat{K}_m \tag{80}$$

The $\hat{K}$ dimensional feature vector of channel m for class c is given by

$$\hat{Z}^r_{m/c} = \hat{\Phi}^r_m Z^r_{m/c} \tag{81}$$

The mean vectors and covariance matrices of $\hat{Z}^r_{m/c}$ are given, respectively, by

$$E[\dot{Z}^r_{m/c}] = \hat{\Phi}^r_m E[Z^r_{m/c}] = \hat{\Phi}^r_m S_{m/c} \tag{82}$$

$$\begin{aligned}\hat{\Psi}^r_m &= E[(\dot{Z}^r_{m/c} - \hat{\Phi}^r_m S_{m/c})(\dot{Z}^r_{m/c} - \hat{\Phi}^r_m S_{m/c})^T] \\ &= \hat{\Phi}^r_m \Psi^r_m (\hat{\Phi}^r_m)^T \end{aligned} \tag{83}$$

Under the linear transformation, $\dot{Z}^r_{m/c}$ remains Gaussian. The class-conditional density of the feature vector of channel m under class c is, therefore,

$$p(\dot{Z}^r_m / c) \sim G(\hat{\Phi}^r_m S_{m/c}, \hat{\Phi}^r_m \Psi^r_m (\hat{\Phi}^r_m)^T). \tag{84}$$

From Equation (75), $\hat{\Phi}^r_m = \hat{\Phi}^1_m = \hat{\Phi}_m$. Therefore, the class-conditional density of the feature vector in terms of the single-trial EP parameters is

$$p(\dot{Z}^r_m / c) \sim G(\hat{\Phi}_m S_{m/c}, \hat{\Phi}_m[(1/r)\Psi_m]\hat{\Phi}^T_m). \tag{85}$$

The Bayes minimum-error classification rule (0-1 loss function) is to assign a test rEP $\dot{Z}^r_m$ to class c if

$$P_c p(\dot{Z}^r_m / c) > P_j p(\dot{Z}^r_m / j) \ all \ j \neq c, \tag{86}$$

where $P_c$ is the a priori probability of the r-EPs belonging to class c. That is, $P_c$ is the probability of occurrence of external stimulus c. The discriminant function for the r-EPs of channel m under class c is given by

$$g_{c/m}(\dot{Z}^r_m) = P_c p(\dot{Z}^r_m / c). \tag{87}$$

Substituting the PDF in Equation (84) into Equation (87) and taking the natural logarithm, the discriminant function for the c-class EPs of channel m can be written as

$$\begin{aligned}g_{c/m}(\dot{Z}^r_m) = \ln(P_c) - (1/2)(\dot{Z}^r_m - \hat{\Phi}^r_m S_{m/c})^T \\ \times [\hat{\Phi}^r_m \Psi^r_m (\hat{\Phi}^r_m)^T]^{-1}(\dot{Z}^r_m - \hat{\Phi}^r_m S_{m/c})\end{aligned} \tag{88}$$

The discriminant function in terms of the single-trial parameters can be written as

$$\begin{aligned}g_{c/m}(\dot{Z}^r_m) = \ln(P_c) - (1/2)(\dot{Z}^r_m - \hat{\Phi}_m S_{m/c})^T \\ \times \{\hat{\Phi}_m[(1/r)\Psi_m]\Phi^T_m\}^{-1}(\dot{Z}^r_m - \hat{\Phi}_m S_{m/c})\end{aligned} \tag{89}$$

and the class detected by channel m is, therefore, given by

$$h^r_m = \arg\max_c [g_{c/m}(\dot{Z}^r_m)]. \tag{90}$$

Weighted Decision fusion Rule. For each channel m, $h_m^r$ takes one of C values, therefore, $h_m^r$ is a discrete random variable. The outputs of the M channel classifiers can be fused into a single M-dimensional decision fusion discrete random vector

$$H^r = (h_1^r, h_2^r, ..., h_M^r)^T .$$ 

(91)

The channel decisions can be weighted by taking the probabilities of $h_m^r$ into account. Let $p_{m,a/c}^r$, $a,c = 1,2,...,$ C be the probability that the decision $h_m^r$ is class a when the true class is c and let the PDF of $H^r$ under class c be $P(H^r/c)$, then, the Bayes decision function for class c is

$$g_c(H^r) = P_c P(H^r / c)$$

(92)

which can also be written as

$$g_c(H^r) = \ln P_c + \ln P(H^r / c)$$

(93)

The final decision resulting from decision fusion is given by

$$c^\bullet = \arg \max_c [g_c(H^r)]$$

(94)

For this general case, the discriminant function $g_c(H^r)$, $c = 1,2,...,C$, can be derived explicitly by noting that the PDF of $h_m^r$ under class c can be written as

$$P(h_m^r / c) = (p_{m,1/c}^r)^{\delta(h_m^r - 1)} (p_{m,2/c}^r)^{\delta(h_m^r - 2)} ... (p_{m,C/c}^r)^{\delta(h_m^r - C)} ,$$

(95)

where

$$\delta(x - a) = \begin{cases} 1 & if & x = a \\ 0 & if & x \neq a \end{cases}$$

(96)

Because the classifiers are developed independently for each channel, we assume that the decisions of the M channel classifiers are independent, therefore, the PDF of $H^r$ under the class c can be written as

$$P(H^r / c) = \prod_{m=1}^{M} \frac{(p_{m,1/c}^r)^{\delta(h_m^r - 1)}}{(p_{m,2/c}^r)^{\delta(h_m^r - 2)} ... (p_{m,C/c}^r)^{\delta(h_m^r - C)}}$$

(97)

By substituting the PDF into Equation (93), it can be shown that the discriminant function for class c can be written as

$$g_c(H^r) = \sum_{m=1}^{M} [\delta(h_m^r - 1)\ln(p_{m,1/c}^r) + \delta(h_m^r - 2)\ln(p_{m,2/c}^r)$$
$$+ \dots + \delta(h_m^r - C)\ln(p_{m,C/c}^r)] + \ln P_c \qquad (98)$$

Majority Decision Fusion Rule. In order to show that the majority decision fusion rule is a special case of the above weighted decision fusion rule, assume that the number of EP classes is C=2, the number of channels is odd, and the classes are equi-probable. Also let

$$p_{m,1/1}^r = p^r, \quad m = 1,2,\dots,M \qquad (99)$$

$$p_{m,1/2}^r = (1 - p^r), \quad m = 1,2,\dots,M \quad . \qquad (100)$$

Through substitution of the probabilities into the discriminant function in Equation (98), the Bayes rule: decide class c=1 if $g_1(H^r) > g_2(H^r)$; otherwise decide class c=2, reduces to: decide class c=1 if

$$\sum_{m=1}^{M} \delta(h_m^r - 1) > \sum_{m=1}^{M} \delta(h_m^r - 2) ; \qquad (101)$$

otherwise decide class c=2, which is the majority rule.

Estimation of probabilities. Prior knowledge of the classification results can be used to assign the probabilities $p_{m;a/b}^r$. Alternatively, the probabilities can be estimated from a validation set of channel m as

$$\hat{p}_{m;a/b}^r = \frac{number\ of\ r - EPs\ of\ class\ b\ classified\ as\ class\ a}{total\ number\ of\ r - EPs\ belonging\ to\ the\ class\ b\ validation\ set},$$
$$a, b = 1,2,\dots,C$$

THE MULTI-CHANNEL EP-SUM FUSION MODEL. In the EP-sum fusion model, each element $u_c^r(j)$, $j = 1,2,\dots K$, of the K-dimensional fusion vector $U_c^r$ is given by a linear combination of the jth elements of the M-channel EPs as shown in Figure 20. That is, $u_c^r(j)$ can be written as

$$u_c^r(j) = a_1^r(j,j)z_{1/c}^r(j) + a_2^r(j,j)z_{2/c}^r(j) + \cdots$$
$$+ a_M^r(j,j)z_{M/c}^r(j) \qquad (102)$$

and the fusion vector for this case can, therefore, be written as the linear combination

$$U_c^r = \sum_{m=1}^{M} A_m^r Z_{m/c}^r , \qquad (103)$$

where $A_m^r$ is a $(K \times K)$ diagonal matrix of weights. The dimension of the fusion vector is the same as the dimension of the EPs of each channel. Each component in the sum term of Equation (103) is a Gaussian random vector with PDF

$$p(A_m^r Z_{m/c}^r) \sim G[A_m^r S_{m/c}, A_m^r \Psi_m^r (A_m^r)^T], \quad m = 1,2,...,M \tag{104}$$

so that

$$p(U^r / c) \sim G(\sum_{m=1}^{M} A_m^r S_{m/c}, \sum_{j=1}^{M} \sum_{k=1}^{M} A_j^r (\Psi_{jk}^r)(A_k^r)^T), \tag{105}$$

where

$$\Psi_{jk}^r = E[(N_j^r)(N_k^r)^T]. \tag{106}$$

Classifier Development. Using the DKLT approach outlined in Section III-A, the features for class c are selected from

$$\hat{U}_c^r = \hat{\Phi}^r U_c^r \tag{107}$$

where $\hat{\Phi}^r$ is the truncated matrix formed by selecting the $\hat{K}$ rows that satisfy Equation (80), of the DKLT of the fusion vector. The PDF of $\hat{U}_c^r$ is, therefore,

$$p(\hat{U}^r / c) \sim G(\hat{\Phi}^r \sum_{m=1}^{M} A_m^r S_{m/c}, \hat{\Phi}^r [\sum_{j=1}^{M} \sum_{k=1}^{M} A_j^r (\Psi_{jk}^r)(A_k^r)^T](\hat{\Phi}^r)^T) . \tag{108}$$

The Bayes discriminant function for class c is given by

$$g_c(\hat{U}^r) = \ln(P_c) - (1/2)\{(\hat{U}^r - \hat{\Phi}^r \sum_{m=1}^{M} A_m^r S_{m/c})^T \times$$
$$\{\hat{\Phi}^r [\sum_{j=1}^{M} \sum_{k=1}^{M} A_j^r (\Psi_{jk}^r)(A_k^r)^T][\hat{\Phi}^r]^T\}^{-1}(\hat{U}^r - \hat{\Phi}^r \sum_{m=1}^{M} A_m^r S_{m/c}), \tag{109}$$

and a test fusion vector $\hat{U}^r$ is assigned to the class $c^*$ given by

$$c^* = \arg \max_{c} [g_c(\hat{U}^r)] . \tag{110}$$

Determining the weighting matrix $A_m^r$. The weight assigned to element $z_{m/c}^r(j)$ in the linear combination that forms $u_c^r(j)$ is $a_m^r(j,j)$. The weights can be selected according to the normalized C-class separations of the jth element of each channel. The highest weight is assigned to the channel element with the highest C-class separation across the M channels which is found through summing the pairwise interclass separations in a manner similar to that described in Section III. Let the C-class separation of the jth element of channel m be $\rho_m^r(j)$. Then, the weight for the jth component of channel m is given by

$$a_m^r(j,j) = \frac{\rho_m^r(j)}{\sum\limits_{i=1}^{M} \rho_i^r(j)}, \quad j = 1,2,...,M . \tag{111}$$

Because $a_m^r(j,j) = a_m^1(j,j) = a_m(j,j)$,

$$A_m^r = A_m . \tag{112}$$

Furthermore, $\hat{\Phi}^r = \hat{\Phi}$ and $\Psi_{jk}^r = (1/r)\Psi_{jk}$, therefore, the discriminant function for class c can be written as

$$g_c(\hat{U}^r) = \ln(P_c) - (1/2)\{(\hat{U}^r - \hat{\Phi}\sum\limits_{m=1}^{M} A_m S_{m/c})^T \times$$
$$\{\hat{\Phi}[\sum\limits_{j=1}^{M}\sum\limits_{k=1}^{M} A_j(\Psi_{jk}/r)(A_k)^T]\hat{\Phi}^T\}^{-1}(\hat{U}^r - \hat{\Phi}\sum\limits_{m=1}^{M} A_m S_{m/c}) \tag{113}$$

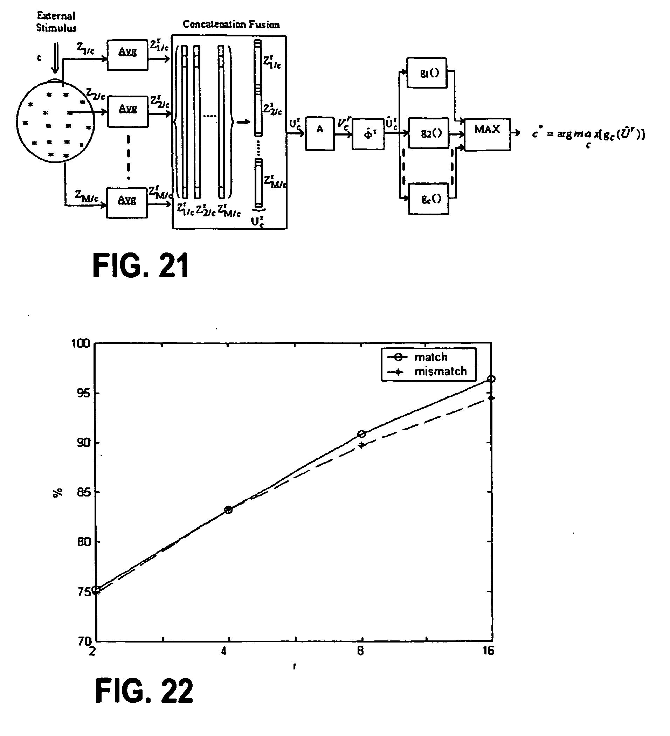

MULTI-CHANNEL EP-CONCATENATION FUSION MODEL. In this fusion model, the M-channel fusion vector for class c is formed by concatenating the EPs of the M channels into a single multi-channel EP of dimension ($K_u \times 1$), where, $K_u = (M \times K)$, as shown in Figure 21. The resulting M-channel fusion vector is given by

$$U_c^r = Z_{1/c}^r \nabla Z_{2/c}^r \nabla,...,\nabla Z_{M/c}^r \tag{114}$$

where we use $\nabla$ to represent the concatenation operation. That is,

$$U_c^r = \overset{M}{\underset{m=1}{\nabla}} Z_{m/c}^r . \tag{115}$$

The elements of $U_c^r$ are related to the elements of the M-channel vectors according to

$$u_c^r[(m-1)K + j] = z_{m/c}^r(j) \tag{116}$$

If $\mu_{u/c}^r$ and $\Psi_u^r$ are the ($K_u \times 1$) mean and ($K_u \times K_u$) covariance of $U_c^r$, respectively, then,

$$\mu_{u/c}^r = \overset{M}{\underset{m=1}{\nabla}} \mu_{m/c}^r = \overset{M}{\underset{m=1}{\nabla}} S_{m/c} \tag{117}$$

$$\Psi_u^r = E[(U_c^r - \mu_{u/c}^r)(U_c^r - \mu_{u/c}^r)^T] = \overset{M}{\underset{k=1}{\nabla}} \overset{M}{\underset{j=1}{\nabla}} \Psi_{jk}^r . \tag{118}$$

That is, the covariance matrix of the fusion vector is formed by concatenating the channel covariance matrices and the inter-channel cross-covariance matrices according to:

$$\begin{bmatrix} \Psi_{11}^r & \Psi_{12}^r & \cdot & \cdot & \cdot & \cdot & \Psi_{1M}^r \\ \Psi_{21}^r & \Psi_{22}^r & & & & & \Psi_{2M}^r \\ & & & & & & \\ & & & & & & \\ & & & & & & \\ \Psi_{M1}^r & \Psi_{M2}^r & & & & & \Psi_{MM}^r \end{bmatrix} \qquad (119)$$

The PDF of each component of the fusion vector is Gaussian and it will be assumed that the PDF of the fusion vector is also Gaussian. That is

$$p(U^r / c) \sim G(\mu_{u/c}^r, \Psi_u^r). \qquad (120)$$

The advantage of concatenation fusion is that the information in the raw EP data from all channels is contained in the multi-channel EP fusion vector. However, the drawback is that the dimensionality of the EP vector is increased by a factor $M$. This increase exacerbates even further the dimensionality problem identified in Reference 1. Note that the DKLT approach cannot be used to decrease the dimension unless, at least $K_u$ single-trial EPs are collected for the training set to ensure that the covariance matrix of the fusion vector is non-singular. In practice, collecting such a large number of single-trial EPs is quite prohibitive even when the number of channels is small. In order to facilitate the design of practical parametric classifiers for the EP concatenation fusion strategy, the dimension of the concatenated fusion vector must be decreased to satisfy the condition that the dimension is less than the number of single-trial EPs in the training set. The challenge, therefore, is to decrease the dimension of the fusion vector without losing useful discriminatory information. A 2-step procedure consisting of multi-class separation-based dimensionality reduction and correlation-based dimensionality reduction is introduced to decrease the dimension to the desired value $K_v$, with $K_v < K_u$. The operations in the 2 steps are combined into a single dimensionality reduction matrix $A^r$. The multi-channel fusion vector of reduced dimension is, then, given by

$$V_c^r = A^r U_c^r \qquad (121)$$

where, $A^r$ is a $(K_v \times K_u)$ matrix. The PDF of $V_c^r$ is, therefore,

$$p(V^r / c) \sim G(A^r \overset{M}{\underset{m=1}{\nabla}} S_{m/c}, A^r [\overset{M}{\underset{k=1}{\nabla}} \overset{M}{\underset{j=1}{\nabla}} \Psi_{jk}^r][A^r]^T). \qquad (122)$$

The 2-step procedure to determine the dimensionality reduction matrix $A^r$ is described next.

Separation-Based Dimensionality Reduction. In the first step of the 2-step procedure, the C-class separation of each element of $U_c^r$ is used to select only those elements that have high C-class separations and discard those with poor separations. This is achieved by multiplying $U_c^r$ with a diagonal matrix $B^r$ whose element $b^r(k,k)$ is given by

$$b^r(k,k) = \begin{cases} 1 \text{ if } w^r(k) \geq \rho_o^r \\ 0, \text{ otherwise} \end{cases} \qquad (123)$$

where $\rho_o^r$ is a C-class separation threshold and $w^r(k)$ is the normalized C-class separation of element k in $U_c^r$. That is, if $p^r(k)$ is the C-class separation of element k, found by summing the pairwise interclass separations as in Section III, $w^r(k)$ is given by

$$w^r(k) = \frac{\rho^r(k)}{\sum_{i=1}^{K_u} \rho^r(i)}, \quad k = 1,2,...,K_u.$$  (124)

The separation matrix $\hat{B}^r$ of dimension $\tilde{K} \times K_u$ is obtained by retaining the $\tilde{K}$ non-zero rows of $B^r$. Because $w^r(k) = w^l(k)$, r can be dropped from $\hat{B}^r$. Then, the separation-based fusion vector of reduced dimension $\tilde{K} \times 1$ is given by

$$\hat{Q}_c^r = \hat{B}U_c^r$$  (125)

Correlation-Based Dimensionality Reduction. In the second step, the correlation is exploited to systematically decrease the dimension by combining the most correlated elements of $\hat{Q}_c^r$. Let

$$\hat{Q}^r = \bigcup_{c=1}^{C} \hat{Q}_c^r$$  (126)

be the C-class mixture of $\hat{Q}_c^r$, $c$ = 1,2,...,C. The dimension is decreased iteratively by combining the most correlated pair of elements in $\hat{Q}^r$ and replacing the correlated pair with the combination. As a result, the dimension is decreased by 1 in each iteration. The combining of the most correlated pairs of elements is repeated until the dimension of the vector is $K_v$. If $\hat{Q}^r[j]$ is the vector at the jth iteration, the procedure for correlation based dimensionality reduction can be summarized as

$$\hat{Q}^r \rightarrow \hat{Q}^r[1] \rightarrow \hat{Q}^r[2] \rightarrow ....\hat{Q}^r[j]... \rightarrow \hat{Q}^r[T]$$  (127)

with dimensions

$$\tilde{K} \times 1 \rightarrow (\tilde{K} - 1) \times 1 \rightarrow (\tilde{K} - 2) \times 1 \rightarrow ...(\tilde{K} - j) \times 1... \rightarrow (\tilde{K}_v \times 1)$$  (128)

In iteration L+1, if $\hat{q}^r(m;L)$ and $\hat{q}^r(n;L)$ were the most correlated pair in $\hat{Q}^r[L]$ and their C-class normalized separations were $\beta_L(m)$ and $\beta_L(n)$, respectively, then, element $\hat{q}^r(m;L+1)$ of $\hat{Q}^r[L+1]$ is given by

$$\hat{q}^r(m; L+1) = \left[\frac{\beta_L(m)}{\beta_L(m) + \beta_L(n)}\right]\hat{q}^r(m; L) + \left[\frac{\beta_L(n)}{\beta_L(m) + \beta_L(n)}\right]\hat{q}^r(n; L)$$  (129)

and

$$\hat{q}^r(n; L+1) = 0.$$  (130)

The element $\hat{q}^r(n; L+1)$ = 0 is removed from $\hat{Q}^r[L+1]$, therefore, the dimension of $\hat{Q}^r[L+1]$ will be $[(\tilde{K} - (L+1)]$. The combining of the most correlated pairs is repeated until $\hat{Q}^r[T]$ has $(\tilde{K} - T) = K_v$ non-zero elements. At the end of each iteration, the elements and the weights of the pairwise linear combinations are noted. Upon termination, the weights assigned to the elements of $U_c^r$ that form the linear combination $v_c^r(j)$ are stored in row j, in the corresponding column, to form the dimensionality reduction matrix $A^r$. The concatenated fusion vector of reduced dimension is given by Equation (121).

Classifier Development. Let

$$\dot{U}_c^r = \hat{\Phi}^r V_c^r \qquad (131)$$

where $\hat{\Phi}^r$ is a $\hat{K} \times K_v$ matrix formed by selecting the $\hat{K}$ rows of the $(K_v \times K_v)$ DKLT matrix of the concatenated fusion vector that satisfy Equation (80). Then, the PDF of $\dot{U}_c^r$ is

$$p(\hat{U}^r / c) \sim G[\hat{\Phi}^r A^r \overset{M}{\underset{m=1}{\triangledown}} S_{m/c}, \hat{\Phi}^r A^r [\overset{M}{\underset{k=1}{\triangledown}} \overset{M}{\underset{j=1}{\triangledown}} \Psi_{jk}^r](A^r)^T(\hat{\Phi}^r)^T] \qquad (132)$$

Because r is not a factor in determining $\hat{B}$ and is also not a factor in the correlation based dimensionality reduction procedure, $A^r$ can be written as A. Therefore, the discriminant function for class c, in terms of the single-trial EP parameters, is given by

$$g_c(\hat{U}^r) = \ln(P_c) - (1/2)(\dot{U}^r - \hat{\Phi}A \overset{M}{\underset{m=1}{\triangledown}} S_{m/c})^T$$
$$\times [\hat{\Phi}A[\overset{M}{\underset{k=1}{\triangledown}} \overset{M}{\underset{j=1}{\triangledown}} (1/r)\Psi_{jk}]A^T\hat{\Phi}^T]^{-1}(\hat{U}^r - \hat{\Phi}A \overset{M}{\underset{m=1}{\triangledown}} S_{m/c}), \qquad (133)$$

and a test vector $\hat{U}^r$ is assigned to the class c* given by

$$c^* = \arg\max_c [g_c(\hat{U}^r)] \qquad (134)$$

PERFORMANCE EVALUATIONS. The goal in this section is to compare the performances of the 6 (3 unweighted and 3 weighted) fusion strategies using a real EP data set. The EPs in the data set were collected from individuals engaged in making explicit match/mismatch comparisons between C=2 sequentially presented stimuli. The match/mismatch effect along with the related mismatch negativity is one of the most studied ERP effects for over 30 years. The data set consisted of 14-channel EPs of 2 female and 3 male subjects. The goal of these experiments was to show that EPs can identify when a match occurs between what a subject thinks and sees. The subjects were instructed to "think" about the first video stimulus (picture of an object) and then to respond whether the next video stimulus (printed word of an object) matched or did not match the first stimulus in "meaning." The period between the first and second stimulus varied randomly between 2 to 5 seconds. The response from each channel, time-locked to the onset of the second stimulus, was recorded as a match category or mismatch category depending on which of 2 keys was pressed by the subject. Responses due to incorrect key presses were discarded. EP data were collected from F7, F8, F3, F4, Fz, T3, T4, T5, T6, C3, C4, P3, P4, and Pz. The 14 electrodes were referenced to linked earlobe leads. The electrooculogram (EOG) was also recorded with two electrodes placed lateral and below the left eye (bipolar montage). Single-trial EPs were digitized over 1 sec using a 10 msec sampling period beginning 100 msec prior to stimulus onset. The 10 samples corresponding to the prestimulus period were removed, thus the dimension of the single-trial EPs was K=90. Trials in which the peak-to-peak amplitude exceeded 100 $\mu$V in any one electrode channel or 50 $\mu$V in the eye channel were regarded as artifacts and rejected. In order to accommodate the different amplitudes across the channels, each single-trial EP was scale normalized by dividing the samples of the EP by the standard deviation of the EP samples. Additionally, each single-trial EP was de-trended to remove slope variations in the EPs within and across the channels. From the ensembles collected, an equal number of artifact-free single-trial match and mismatch EPs were selected for each subject, however, the number varied across the subjects. The total number of single-trial EPs collected for each category was 71, 71, 82, 71, and 63 for the 5 subjects.

Classifier Design. The single-trial covariance matrix for each channel was estimated, using the maximum likelihood estimate, by pooling the match and mismatch single-trial EPs because it is assumed that the noise under the match and mismatch conditions are statistically equivalent. The channel match and mismatch mean vectors were estimated directly from their respective single-trial training sets. Because the number of single-trials in the design set mixture of the EPs should exceed the dimension of the EP vector for covariance estimation, the EPs were downsampled from 90 to have dimensions 70, 70, 80, 70, and 60, respectively. The EPs were downsampled by assuming a piecewise linear fit to the EP samples and uniformly resampling the resulting piecewise linear curve to obtain the desired dimension. The channel weights for decision fusion were selected based on the individual channel classification accuracies of the channels. The unweighted results for the sum fusion corresponded to selecting $A^r = A = I$. The weights $\beta_{(.)}(.)$ introduced in the correlation step of dimensionality reduction for concatenation fusion were selected to be (1/2) for the unweighted case. The dimension of the concatenation fusion vector was decreased to satisfy the condition that the number of 1-EPs should be greater than or equal to the dimension of the EP vector. For example, for the subjects who had 71 single-trial EPs in each category, the dimension was decreased from $70 \times 14 = 980$ to 70. The dimensionality reduction matrix $A$ was determined by first selecting the threshold $\rho_o^r$ in Equation (123) to discard 50% (490) elements with the smallest inter-class separations. It must be noted that the 490 elements discarded accounted for only 7.6% of the total separation (sum of the 980 separations) averaged across the subjects. The correlation algorithm was applied to decrease the dimension from 490 to 70. Note that the dimension was decreased in the correlation algorithm, not by discarding any of the remaining 50% of the elements, but by systematically combining the most correlated elements in a pairwise fashion until the desired dimension was obtained. The features selected in all 3 fusion strategies were the DKLT linear combinations of the EP samples that accounted for the highest separation amongst the C classes. In the two data fusion strategies, the features were computed after the fusion vector was formed. For all cases, the threshold $\delta$ in Equation (79) for selecting the number of eigenvectors, thus the number of features, was 0.1. Because the second stimulus matched or did not match the first stimulus with equal probability, the a priori probabilities in the discriminant functions were set to $P_c = 0.5$, $c = 1,2$.

Classification Results. For each stimulus, the single-trial EP data of each channel was randomly partitioned into 2 equi-sized sets: the training set and the test set. In each set, the single-trial responses were randomly selected and averaged to form the r-EP training and test sets. A re-sampling approach was employed to generate $J^r$ design and test set pairs. Each pair is referred to as a partition and for each partition $j, j = 1,2,...,J^r$, the probability of classification error was estimated as

$$P_j^r(\varepsilon) = \sum_{c=1}^{C} P_j^r(\varepsilon/c)P_c \qquad (135)$$

where, $P_j^r(\varepsilon/c)$ is the estimated probability of misclassifying the r-EPs of class c in partition j. The classification rate $\alpha_c^r$ for class c and the classification accuracy $a^r$, estimated over $J^r$ partitions, are given by

$$\alpha_c^r = [1 - (1/J^r)\sum_{j=1}^{J^r} P_j^r(\varepsilon/c)] \times 100\% \qquad (136)$$

and

$$\alpha^r = [1 - (1/J^r)\sum_{j=1}^{J^r} P_j^r(\varepsilon)] \times 100\% , \qquad (137)$$

respectively. The classification accuracy was used to compare the performances of the different fusion strategies. For each subject, the classification accuracy was averaged over 200 stimulus presentations for r taking values 2, 4, 8, and 16. The classification accuracies, as a function of r, averaged across the 5 subjects are summarized in Table 17. Also included in the table is a ranking, enclosed in parentheses, of the 6 classifiers for each r. The classifier ranked 1 yielded the highest classification accuracy and the classifier ranked 6 yielded the lowest classification accuracy. The two columns under decision fusion compare the majority decision rule (unweighted) and weighted decision fusion rules. The best result for a given r is presented in boldface.

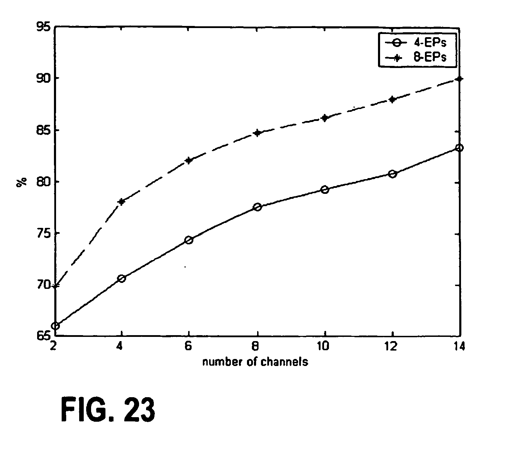

Figure 22 shows the match and mismatch classification rates corresponding to the classification accuracies of the EP-concatenation strategy shown in Table 17. In order to investigate the improvement in performance by increasing the number of channels, the concatenation fusion strategy was implemented by selecting M=2, 4, 6, 8, 10, 12, and 14 channels. The classification accuracies, averaged across the 5 subjects, for r=4 and 8 are shown in Figure 23.

The following conclusions can be drawn from the results in Table 17 and the results in Figures 22 and 23:

(1) The classification accuracies increased when r was increased for all fusion methods. This confirms an expected result.

(2) Also as expected, the match and mismatch classification rates increased when r was increased. The rates were not significantly different for the smaller values of r (2 and 4) and there was a small increase in the match rate for the higher values (8 and 16).

(3) The performance improved by incorporating weights into the fusion rules. In some decision and sum-fusion cases, the improvement was quite dramatic.

(4) The EP-concatenation fusion strategy yielded the highest classification accuracies consistently. If the majority rule (unweighted decision fusion) is used as a benchmark, the improvement in classification accuracy resulting from weighted concatenation fusion is quite significant.

(5) The rankings of the fusion strategies are consistent across all the values of r.

(6) The performance improved when the number of channels concatenated was increased. This is in spite of the fact the dimensionality reduction ratio, defined as $(K_u / K_v)$, increased proportionately when the number of channels concatenated was increased. The individual classification accuracies of the 5 subjects and an alternate method to rank the 6 classifiers are presented in Table 18.

CONCLUSIONS. The goal of this section was to develop fusion strategies to improve the classification accuracies of averaged EPs by exploiting the brain-activity information from multiple channels. A multi-channel decision fusion model and two multi-channel data fusion models were introduced and parametric classifiers were developed for each fusion model. The discriminant functions of the resulting fusion classifiers were derived in terms of the parameters of the single-trial EPs thus making it possible to develop and test r-EP classifiers even when the number of r-EPs was smaller than the EP dimension.

Results from the classification experiments showed that the performance improved by incorporating weights in the fusion rules. The channels were weighted according to their respective classification accuracies and the EP elements were weighted according to the multi-class separations in the decision fusion and data fusion strategies, respectively. The best results were obtained through weighted EP-concatenation fusion. It could be argued that this is an expected result because the information from all the channels is initially contained in the M-channel EP fusion vector. However, the dimension of the fusion vector increases by a factor of M thus exacerbating the problems even further in estimating the classifier parameters. The challenge, therefore, was to decrease the dimension of the fusion vector without losing useful discriminatory information. A 2-step dimensionality reduction algorithm which took into account the inter-class separation and the correlation of the components in the fusion vector was developed. Furthermore, it was shown that the performance improved when the number of channels was increased even though the dimensionality reduction ratio increased proportionately. It could, therefore, be expected that the performance would increase even further if a larger number of channels were used provided that the most useful discriminatory information is selected without loosing too much information during dimensionality reduction.

The dimension of the data fusion vector for EP-sum fusion was the same as that of the channel EPs. However, summing the EPs of different channels involves convolving the statistical information from the individual channels. As a result, the performance of the EP-sum data fusion strategy was not as impressive as that of concatenation fusion. An alternative approach to fuse multi-channel EPs without increasing the dimension is to pool the multi-channel EPs of each class to form a multi-channel EP mixture for each class. We refer to this strategy as "mixture-fusion." The mixture components can be weighted according to their respective classification accuracies. The discriminant function for each class can be derived by noting that the PDF of the mixture is given by the sum of the weighted component PDFs. Our preliminary studies showed that the mixture-fusion results were worse than the sum-fusion results due to the increase of the scatter within each class (intra-class scatter) resulting from the variations of the EPs across the multiple channels. Because this approach was not promising, it was not pursued any further.

The concatenation fusion strategy may also be used to directly fuse features computed from the EPs of each channel. Examples of features derived from EPs, among many others, include autoregressive model parameters, peak and latency measurements and wavelets. Fusing a small set of discriminatory features from each channel will help decrease the dimensionality problem in the concatenation fusion model. However, due to the loss of information during feature generation, feature-fusion systems yield lower classification accuracies when compared with data fusion systems.

In terms of implementation, the data fusion strategies are simpler than decision fusion strategies because only a single classifier is required. All the parameters in the discriminant functions are pre-computed during the design stage, therefore, testing is quite straightforward. The additional dimensionality reduction stage in the concatenation strategy involves only a simple matrix multiplication during testing. In summary, the concatenation fusion strategy yields the best performance and is also simpler to implement than decision fusion. Furthermore, the proposed data fusion classification strategies are quite general in their formulations and can, therefore, be applied to other problems involving the classification of multi-category-multivariate signals collected from multiple sensors.

The individual classification accuracies of the 5 subjects are shown in Table 18. The classifier rankings for each r are enclosed in parentheses. In order to determine the ranks of the 6 classifiers across the subjects and across r simultaneously, the ranks of each classifier were first summed along each column to give the rank-sum as shown in the row labeled "Rank-Sum". Next, the classifiers were ranked by ranking the rank-sums as shown in the row labeled "Rank of Rank-Sum." It is interesting to observe that although there were variations in the classifier rankings, the final rankings are identical to the ranking that would be obtained by ranking the rank-sums of the classifiers using the averaged (across subjects) classification accuracies shown in Table 17.

| Subject | r | Decision Fusion | | Sum fusion | | Concatenation fusion | |
|---|---|---|---|---|---|---|---|
| | | Unweighted | Weighted | Unweighted | Weighted | Unweighted | Weighted |
| S1 | 2 | 81.10 (4) | 82.06 (3) | 75.21 (6) | 80.31 (5) | 82.50 (2) | **82.51 (1)** |
| | 4 | 89.50 (4) | 90.44 (3) | 82.09 (6) | 88.69 (5) | 91.00 (2) | **91.33 (1)** |
| | 8 | 96.13 (4) | 96.73 (2) | 91.44 (6) | 95.25 (5) | 96.25 (3) | **97.28 (1)** |
| | 16 | 99.25 (4) | 99.55 (3) | 96.00 (6) | 99.25 (4) | 99.56 (2) | **99.69 (1)** |
| S2 | 2 | 57.54 (5) | 65.27 (4) | 51.47 (6) | 65.99 (3) | 68.94 (2) | **72.09 (1)** |
| | 4 | 60.41 (5) | 69.47 (4) | 53.78 (6) | 70.78 (3) | 75.34 (2) | **81.19 (1)** |
| | 8 | 63.75 (5) | 77.00 (4) | 55.31 (6) | 77.94 (3) | 83.19 (2) | **88.25 (1)** |
| | 16 | 69.25 (5) | 84.38 (4) | 54.00(6) | 86.50 (3) | 90.75 (2) | **92.50 (1)** |
| S3 | 2 | 65.90 (5) | 70.84 (3) | 55.65 (6) | 73.82 (2) | 70.18 (4) | **78.63 (1)** |
| | 4 | 71.53 (5) | 78.38 (4) | 55.66(6) | 82.41 (2) | 79.16(3) | **84.78 (1)** |
| | 8 | 78.69 (5) | 85.75 (4) | 59.00 (6) | 88.75 (2) | 86.63 (3) | **92.31 (1)** |
| | 16 | 84.63 (5) | 93.50 (3) | 65.88 (6) | 96.25 (2) | 93.50 (3) | **98.00 (1)** |
| S4 | 2 | 65.38 (5) | 69.65 (4) | 57.91 (6) | 73.35 (3) | 75.55 (2) | **75.88(1)** |
| | 4 | 70.78 (5) | 76.17 (4) | 59.6 (6) | 82.60 (3) | 83.18 (2) | **83.90(1)** |
| | 8 | 77.95 (5) | 84.00(4) | 64.55(6) | 89.40(3) | 91.10(2) | **91.70(1)** |
| | 16 | 87.75 (5) | 93.00 (4) | 77.00 (6) | 97.13 (3) | 98.50 (1) | **98.50 (1)** |
| S5 | 2 | 59.42 (5) | 62.73 (3) | 57.42 (6) | 62.39 (4) | 65.81 (2) | **68.03 (1)** |
| | 4 | 62.84 (5) | 67.78 (3) | 59.19 (6) | 66.84 (4) | 70.31 (2) | **74.81 (1)** |
| | 8 | 66.89(5) | 73.19 (4) | 61.75(6) | 73.56(3) | 77.13 (2) | **81.88 (1)** |
| | 16 | 71.50 (5) | 79.88 (3) | 68.63 (6) | 79.13 (4) | 84.63 (2) | **88.25 (1)** |
| Rank-Sum | | 96 | 70 | 120 | 66 | 45 | **20** |
| Rank of Rank-Sum | | 5 | 4 | 6 | 3 | 2 | **1** |

Table 18 The individual classification accuracies of the 5 subjects and the classifier rankings

| r | Decision Fusion | | Sum fusion | | Concatenation fusion | |
|---|---|---|---|---|---|---|
| | Unweighted | Weighted | Unweighted | Weighted | Unweighted | Weighted |
| 2 | 65.87 (5) | 70.05 (4) | 59.53 (6) | 71.17 (3) | 72.60 (2) | **75.03 (1)** |

(continued)

| | r | Decision Fusion | | Sum fusion | | Concatenation fusion | |
|---|---|---|---|---|---|---|---|
| | | Unweighted | Weighted | Unweighted | Weighted | Unweighted | Weighted |
| | 4 | 71.01 (5) | 76.45 (4) | 62.06 (6) | 78.26 (3) | 79.80 (2) | **83.20 (1)** |
| | 8 | 76.68 (5) | 83.33 (4) | 66.41 (6) | 84.98 (3) | 86.86 (2) | **90.28 (1)** |
| | 16 | 82.38 (5) | 90.06 (4) | 72.30 (6) | 91.65 (3) | 93.39 (2) | **95.39 (1)** |

**Claims**

1. An apparatus for classifying an unknown multi-channel biopotential signal, comprising:

   a memory operatively configured to access a biopotential dataset produced by detecting a respective biopotential, signal from a plurality of biopotential channels received from the patient and containing a program; and
   a controller in communication with the memory and operatively configured to execute the program comprising:

   determining - in a training stage - a classification accuracy ranking for each biopotential channel at each time instant, wherein the classification accuracy ranking is determined by using a dynamic classification strategy wherein different biopotential channels are selected at different time instants and the biopotential channels are ranked, at different time instants, according to their classification accuracies,
   creating a subset of the biopotential channels for a selected time instant by selecting the biopotential channels for the subset having the highest classification accuracy,
   classifying independently the selected subset of biopotential channels for the selected time instant,
   fusing the resulting independent classifications into a single decision fusion vector for the selected time instant, and
   classifying the single decision fusion vector using a discrete Bayes classifier.

2. The apparatus of claim 1, wherein the controller is further operatively configured to execute a program that selects the subset of the biopotential channels by reordering the biopotential dataset according to the classification accuracy ranking and selecting a subset based on a predetermined number of top channels.

3. The apparatus of claim 1, wherein the controller is further operatively configured to execute a program further comprising determining a time instant in reference to stimulus imparted to the patient.

4. The apparatus of claim 1, wherein the controller is further operatively configured to execute a program further comprising determining a time instant by pattern matching against a characteristic heart beat waveform.

**Patentansprüche**

1. Vorrichtung zum Klassifizieren eines unbekannten Mehrkanal-Biopotential-Signals, umfassend:

   einen Speicher, der betriebsfähig konfiguriert ist, auf einen Biopotential-Datensatz zuzugreifen, der erzeugt wird, indem ein entsprechendes Biopotential-Signal von einer Mehrzahl von von dem Patienten erhaltenen Biopotential-Kanälen detektiert wird, und der ein Programm enthält;
   eine Steuerung in Kommunikation mit den Speicher und betriebsfähig konfiguriert, das Programm auszuführen, umfassend:

   Ermitteln in einer Trainingsstufe einer Klassifikationsgenauigkeitsrangordnung für jeden Biopotential-Kanal zu jedem Zeitpunkt, wobei die Klassifikationsgenauigkeitsrangordnung unter Verwendung einer dynamischen Klassifikationsstrategie ermittelt wird, wobei unterschiedliche Biopotential-Kanäle zu unterschiedlichen Zeitpunkten gewählt werden und die Biopotential-Kanäle zu unterschiedlichen Zeitpunkten gemäß ihren Klassifikationsgenauigkeiten geordnet werden,
   Erzeugen einer Untergruppe der Biopotential-Kanäle für einen gewählten Zeitpunkt, indem für die Untergruppe die Biopotential-Kanäle gewählt werden, die die höchste Klassifikationsgenauigkeit haben,

unabhängiges Klassifizieren der gewählten Untergruppe von Biopotential-Kanälen für den gewählten Zeitpunkt,
Fusionieren der resultierenden unabhängigen Klassifikationen in einen einzelnen Entscheidungsfusionsvektor für den gewählten Zeitpunkt, und
Klassifizieren des einzelnen Entscheidungsfusionsvektors unter Verwendung eines diskreten Bayes-Klassifizierers.

**2.** Vorrichtung nach Anspruch 1, wobei die Steuerung ferner betriebsfähig konfiguriert ist, ein Programm auszuführen, das die Untergruppe der Biopotential-Kanäle wählt, indem der Biopotential-Datensatz gemäß der Klassifikationsgenauigkeitsrangordnung neu geordnet wird und eine Gruppe auf der Grundlage einer vorbestimmten Zahl bester Kanäle gewählt wird.

**3.** Vorrichtung nach Anspruch 1, wobei die Steuerung ferner betriebsfähig konfiguriert ist, ein Programm auszuführen, das ferner umfasst, einen Zeitpunkt bezüglich eines auf den Patienten übertragenen Stimulus zu ermitteln.

**4.** Vorrichtung nach Anspruch 1, wobei die Steuerung ferner betriebsfähig konfiguriert ist, ein Programm auszuführen, das ferner umfasst, einen Zeitpunkt mittels Mustervergleichs hinsichtlich einer charakteristischen Herzschlagwellenform zu ermitteln.

**Revendications**

**1.** Appareil pour classifier un signal de biopotentiel multicanaux inconnu, comprenant :

♦ une mémoire configurée opérationnellement pour accéder à un ensemble de données de biopotentiel produit en détectant un signal de biopotentiel respectif parmi une pluralité de canaux de potentiel reçus du patient et contenant un programme ; et

♦ un contrôleur en communication avec la mémoire et configuré opérationnellement pour exécuter le programme comprenant :

♦ la détermination - dans une phase d'apprentissage - d'un classement de précision de classification pour chaque canal de biopotentiel à chaque instant dans le temps, où le classement de précision de classification est déterminé en utilisant une stratégie de classification dynamique où des canaux de biopotentiel différents sont sélectionnés à différents instants dans le temps et les canaux de biopotentiel sont classés, à différents instants dans le temps, selon leurs précisions de classification,

♦ la création d'un sous-ensemble des canaux de biopotentiel pour un instant dans le temps sélectionné en sélectionnant les canaux de biopotentiel pour le sous-ensemble ayant la précision de classification la plus élevée,

♦ la classification de façon indépendante du sous-ensemble de canaux de biopotentiel sélectionné pour l'instant dans le temps sélectionné,

♦ la fusion des classifications indépendantes résultantes en un vecteur de fusion à décision unique pour l'instant dans le temps sélectionné, et

♦ la classification du vecteur de fusion à décision unique en utilisant un classificateur de Bayes discret.

**2.** Appareil selon la revendication 1, dans lequel le contrôleur est en outre configuré opérationnellement pour exécuter un programme qui sélectionne le sous-ensemble des canaux de biopotentiel en réordonnant l'ensemble de données de biopotentiel selon le classement de précision de classification et en sélectionnant un sous-ensemble sur la base d'un nombre prédéterminé de canaux supérieurs.

**3.** Appareil selon la revendication 1, dans lequel le contrôleur est en outre configuré opérationnellement pour exécuter un programme comprenant en outre la détermination d'un instant dans le temps en référence à un stimulus communiqué au patient.

**4.** Appareil selon la revendication 1, dans lequel le contrôleur est en outre configuré opérationnellement pour exécuter un programme comprenant en outre la détermination d'un instant dans le temps par un appariement de formes par rapport à une forme d'onde de battement du coeur caractéristique.

**FIG. 1**

FIG. 2

**FIG. 3**

**FIG. 4**

50

```
        ┌──────────────┐
        │      51      │
        │   New ERP    │
        │     Test     │
        │   Results    │
        └──────────────┘
               │
               ▼
┌────────────┐ ┌────────────┐ ┌────────────┐
│     53     │ │     52     │ │     61     │
│  Subject   │─│ Subject Test│─│Select Subject│
│  Medical/  │ │  Results   │ │ Phenotypes │
│ Behavioral │ │            │ │            │
│    Data    │ │            │ │            │
└────────────┘ └────────────┘ └────────────┘
                     │              │
                     ▼              ▼
              ◇──────────────◇  ⬡────────────⬡
              │     54       │  │     56     │   ┌────────────┐
              │ Phenotype yes│─▶│Calculate Pattern│─▶│     58     │
              │  Exist?      │  │ Recognition│   │  Pattern   │
              │     no       │  │ Classifier │   │Recognition │
              ◇──────────────◇  ⬡────────────⬡   │Classifiers │
                     │                           └────────────┘
                     ▼
              ⬡────────────⬡
              │     57     │
              │ Classifier │◀╌╌╌╌╌
              │ Compairson │
              ⬡────────────⬡
                     │
                     ▼
              ┌────────────┐
              │     59     │
              │  "At Risk" │
              │Classification│
              │   Report   │
              └────────────┘
                     ╎
                     ▼
              ┌────────────┐
              │     60     │
              │    New     │
              │  Medical/  │
              │ Behavioral │
              │    Data    │
              └────────────┘
```

# FIG. 5

FIG. 6

FIG. 7

EP 1 789 907 B1

FIG. 8

FIG. 9

**FIG. 10**

**FIG. 11**

FIG. 12

FIG. 13

**FIG. 14**

**FIG. 15**

**FIG. 16**

**FIG. 17**

**FIG. 18**

FIG. 19

FIG. 20

**FIG. 21**

**FIG. 22**

FIG. 23

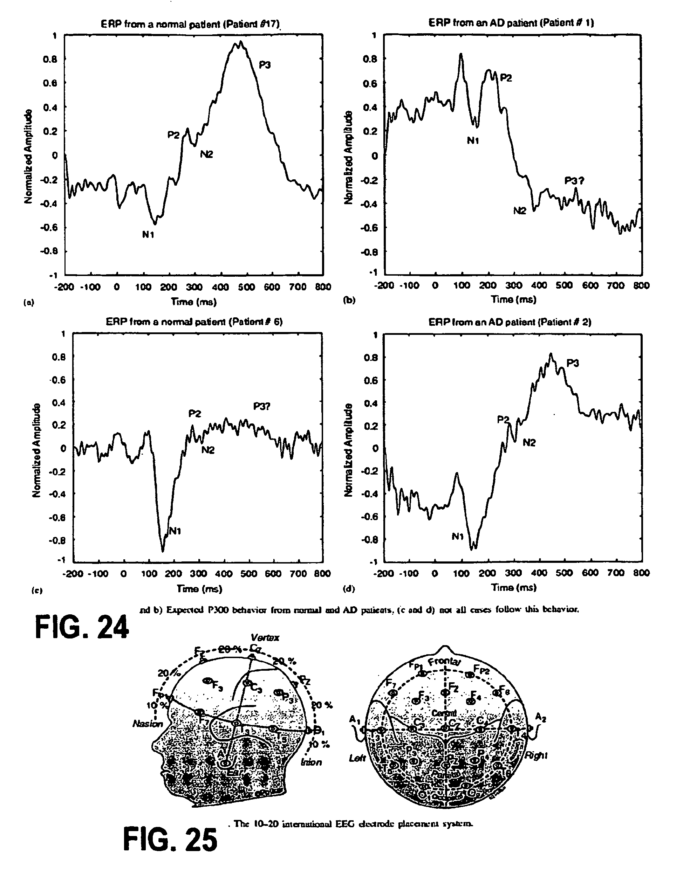

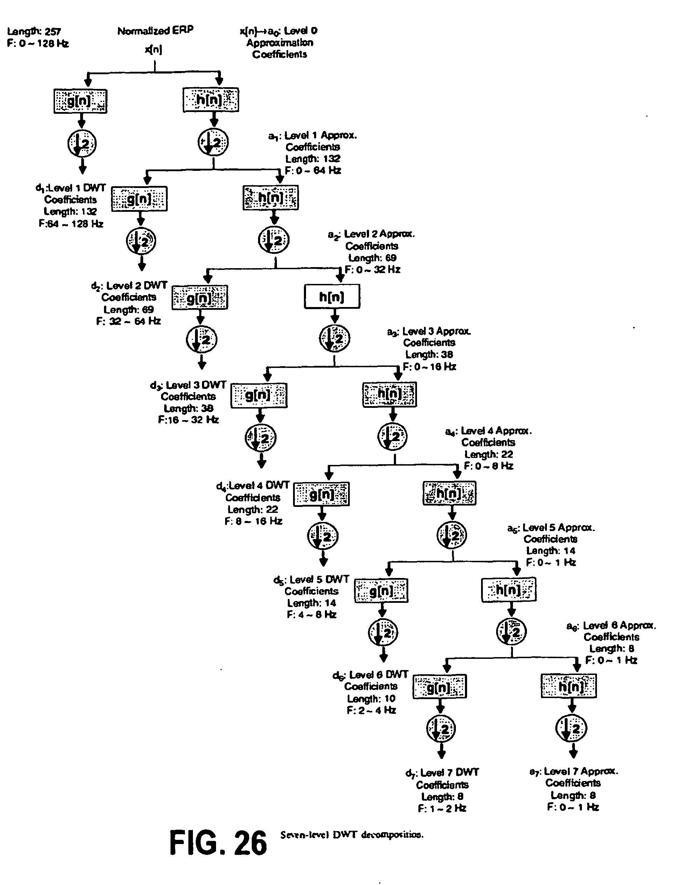

FIG. 24

and b) Expected P300 behavior from normal and AD patients, (c and d) not all cases follow this behavior.

FIG. 25

. The 10-20 international EEG electrode placement system.

**FIG. 26** Seven-level DWT decomposition.

70

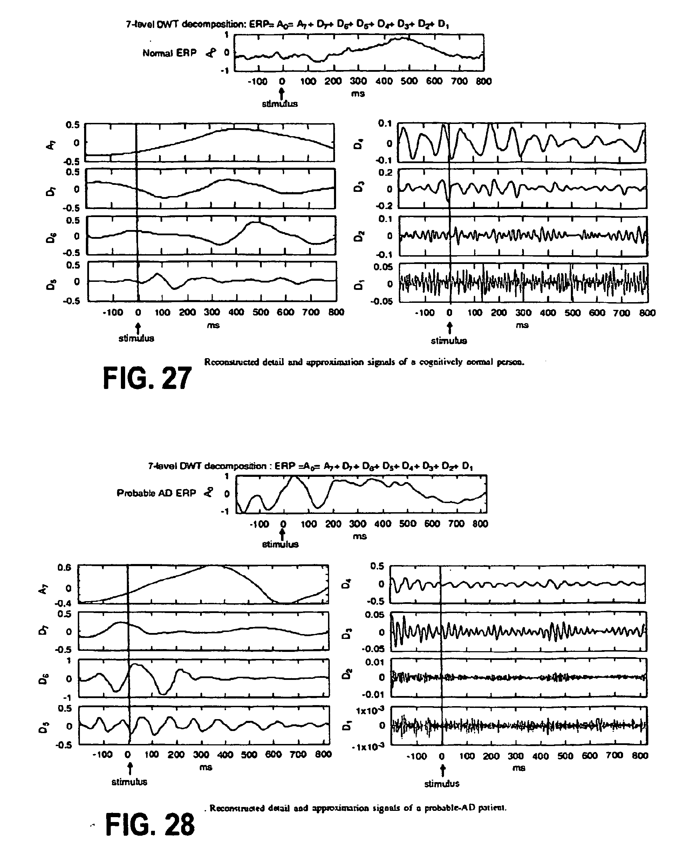

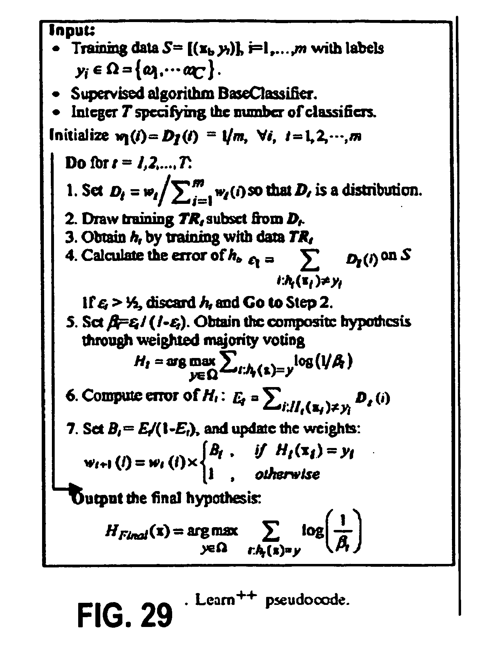

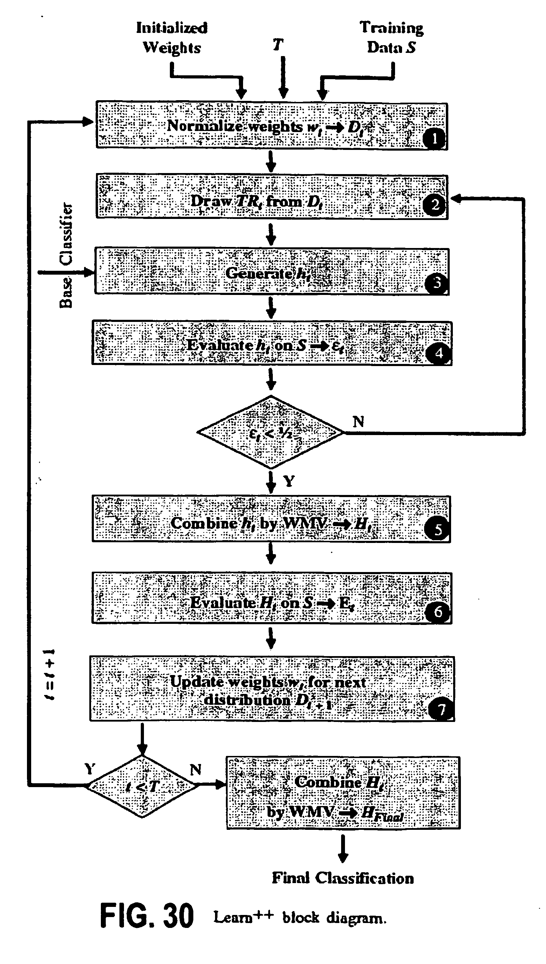

FIG. 27

Reconstructed detail and approximation signals of a cognitively normal person.

FIG. 28

Reconstructed detail and approximation signals of a probable-AD patient.

**Input:**
- Training data $S = [(x_i, y_i)]$, $i = 1, \ldots, m$ with labels $y_i \in \Omega = \{\omega_1, \cdots \omega_C\}$.
- Supervised algorithm BaseClassifier.
- Integer $T$ specifying the number of classifiers.

Initialize $w_1(i) = D_1(i) = 1/m$, $\forall i$, $i = 1, 2, \cdots, m$

Do for $t = 1, 2, \ldots, T$:

1. Set $D_t = w_t \big/ \sum_{i=1}^{m} w_t(i)$ so that $D_t$ is a distribution.

2. Draw training $TR_t$ subset from $D_t$.

3. Obtain $h_t$ by training with data $TR_t$

4. Calculate the error of $h_t$, $\varepsilon_t = \sum_{i:h_t(x_i) \neq y_i} D_t(i)$ on $S$

   If $\varepsilon_t > \frac{1}{2}$, discard $h_t$ and Go to Step 2.

5. Set $\beta_t = \varepsilon_t / (1 - \varepsilon_t)$. Obtain the composite hypothesis through weighted majority voting
   $$H_t = \arg\max_{y \in \Omega} \sum_{t:h_t(x) = y} \log(1/\beta_t)$$

6. Compute error of $H_t$: $E_t = \sum_{i:H_t(x_i) \neq y_i} D_t(i)$

7. Set $B_t = E_t / (1 - E_t)$, and update the weights:
   $$w_{t+1}(i) = w_t(i) \times \begin{cases} B_t, & \text{if } H_t(x_i) = y_i \\ 1, & \text{otherwise} \end{cases}$$

Output the final hypothesis:
$$H_{Final}(x) = \arg\max_{y \in \Omega} \sum_{t:h_t(x) = y} \log\left(\frac{1}{\beta_t}\right)$$

**FIG. 29** . Learn$^{++}$ pseudocode.

72

**FIG. 30** Learn++ block diagram.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 60605630 B
- WO 2004US19418 PCT
- WO 200411260 A2
- US 60479684 B
- WO 2005US010515 A
- US 09239505 A
- US 55723004 P
- WO 2005US21272 A

- US 58085304 P
- WO 2004US194018 A
- US 5467777 A
- US 5406956 A
- US 5363858 A
- US 4941477 A
- US 5661666 A
- WO 2004US19418 A

**Non-patent literature cited in the description**

- **L. Gupta ; J. Phegley ; D.L. Molfese.** Parameter estimation and multichannel fusion for classifying averaged ERPs. *The Second Joint Meeting of the IEEE Engineering in Medicine and Biology Society and the Biomedical Engineering Society,* 26 October 2002
- **L. Gupta ; B. Chung ; J. Phegley ; D.L. Molfese.** A multi-channel EP fusion classification strategy for brain-computer interface development. *The 7 World Multiconference on Systemics, Cybernetics and Informatics,* 27 July 2003

- **L. Gupta ; B. Chung ; J. Phegley ; D.L. Molfese.** Multi-channel fusion models for the parametric classification of multi-category differential brain activity. *26th Annual International Conference of the IEEE Engineering in Medicine and Biology Society,* 01 September 2004
- **L. Gupta ; J. Phegley ; D.L. Molfese.** Parametric classification of multichannel evoked potentials. *IEEE Transactions on Biomedical Engineering,* August 2002, vol. 49 (8), 905-911
- *IEEE TRANSACTIONS ON BIOMEDICAL ENGINEERING,* September 2002, vol. 49 (9), 1070
- **D.L. Molfese.** Predicting dyslexia at 8 years of age using neonatal brain responses. *Brain and Language,* 2000, vol. 72, 238-245